(19) Europäisches Patentamt
European Patent Office
Office européen des brevets

(11) EP 0 832 063 B1

(12) EUROPÄISCHE PATENTSCHRIFT

(45) Veröffentlichungstag und Bekanntmachung des Hinweises auf die Patenterteilung:
**23.02.2000 Patentblatt 2000/08**

(21) Anmeldenummer: **96915001.0**

(22) Anmeldetag: **30.04.1996**

(51) Int Cl.[7]: **C07C 401/00**, A61K 31/59

(86) Internationale Anmeldenummer:
**PCT/EP96/01788**

(87) Internationale Veröffentlichungsnummer:
**WO 97/00242 (03.01.1997 Gazette 1997/02)**

# (54) VITAMIN D-DERIVATE MIT SUBSTITUENTEN AN C-25, VERFAHREN ZU IHRER HERSTELLUNG, ZWISCHENPRODUKTE UND DIE VERWENDUNG ZUR HERSTELLUNG VON ARZNEIMITTELN

VITAMIN D DERIVATIVES WITH C-25 SUBSTITUENTS, PROCESS FOR THEIR PREPARATION, INTERMEDIATE PRODUCTS AND THEIR USE IN PREPARING MEDICAMENTS

DERIVES DE VITAMINE D COMPORTANT DES SUBSTITUANTS EN C-25, LEURS PROCEDES DE PREPARATION, PRODUITS INTERMEDIAIRES ET LEUR UTILISATION POUR PRODUIRE DES MEDICAMENTS

(84) Benannte Vertragsstaaten:
**AT BE CH DE DK ES FI FR GB GR IE IT LI LU MC NL PT SE**

(30) Priorität: **14.06.1995 DE 19522797**

(43) Veröffentlichungstag der Anmeldung:
**01.04.1998 Patentblatt 1998/14**

(73) Patentinhaber: **SCHERING AKTIENGESELLSCHAFT**
**13353 Berlin (DE)**

(72) Erfinder:
- **KIRSCH, Gerald**
  **D-14199 Berlin (DE)**
- **STEINMEYER, Andreas**
  **D-13581 Berlin (DE)**
- **NEEF, Günter**
  **D-10711 Berlin (DE)**
- **SCHWARZ, Katica**
  **D-10585 Berlin (DE)**
- **THIEROFF-EKERDT, Ruth**
  **D-13469 Berlin (DE)**
- **WIESINGER, Herbert**
  **D-10781 Berlin (DE)**
- **MENRAD, Andreas**
  **D-13505 Berlin (DE)**
- **HABEREY, Martin**
  **D-12247 Berlin (DE)**

(56) Entgegenhaltungen:
**WO-A-94/07853**

- **PROCEEDINGS OF THE NATIONAL ACADEMY OF SCIENCES OF USA, Bd. 84, Nr. 9, 1987, WASHINGTON US, Seiten 2610-2614, XP002009787 V. OSTREM ET AL: "24- and 26-Homo-1,25-dihydroxyvitamin D3: Preferential Activity in Inducing Differentiation of Human Leukemia Cells HL-60 "In Vitro""**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Die vorliegende Erfindung betrifft Vitamin D-Derivate mit Substituenten an C-25 der allgemeinen Formel **I**,

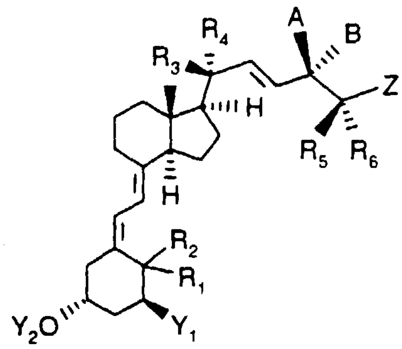

I

worin

$Y_1$ ein Wasserstoffatom, eine Hydroxylgruppe, eine Alkanoyloxygruppe mit 1 bis 12 C-Atomen oder eine Aroyloxygruppe,
$Y_2$ ein Wasserstoffatom oder eine Alkanoylgruppe mit 1 bis 12 C-Atomen oder eine Aroylgruppe,
$R_1$ und $R_2$ je ein Wasserstoffatom oder gemeinsam eine exocyclische Methylengruppe.
$R_3$ und $R_4$ unabhängig voneinander ein Wasserstoffatom, ein Chlor- oder Fluoratom, eine Alkylgruppe mit 1 bis 4 Kohlenstoffatomen, gemeinsam eine Methylengruppe oder gemeinsam mit dem quartären Kohlenstoffatom 20 einen 3-7-gliedrigen, gesättigten oder ungesättigten carbocyclischen Ring,
A und B gemeinsam eine Ketogruppe oder A eine Gruppe OR' und B ein Wasserstoffatom oder B eine Gruppe OR' und A ein Wasserstoffatom, wobei R' ein Wasserstoffatom oder eine gerad- oder verzweigtkettige, gesättigte Alkanoylgruppe mit bis zu 9 Kohlenstoffatomen oder eine Aroylgruppe ist,
$R_5$ und $R_6$ gleichzeitig je ein Wasserstoffatom, ein Chlor- oder Fluoratom, eine Trifluormethylgruppe, einen gerad- oder verzweigtkettigen, gesättigten oder ungesättigten Kohlenwasserstoffrest mit bis zu 4 Kohlenstoffatomen oder $R_5$ und $R_6$ gemeinsam mit dem Kohlenstoffatom 25 einen 3-7-gliedrigen, gesättigten oder ungesättigten carbocyclischen Ring und
Z einen gerad- oder verzweigtkettigen, gesättigten oder ungesättigten Kohlenwasserstoffrest mit bis zu 12 Kohlenstoffatomen der auch carbo- oder heterocyclische Partialstruktur haben kann und an beliebigen Positionen Ketogruppen, Hydroxygruppen (α- oder β-ständig), die ihrerseits verethert oder verestert sein können, Aminogruppen, Halogenatome oder Carbonsäureester oder -amideinheiten aufweisen kann und durch eine Carbonylgruppe, eine Hydroxymethylengruppe oder eine Ethendiyl-Einheit (-CH=CH-, *E* oder *Z*-Geometrie) mit dem Kohlenstoffatom 25 verknüpft ist,

bedeuten,
sowie Verfahren zu ihrer Herstellung, Zwischenprodukte für diese Verfahren, pharmazeutische Präparate, die diese Verbindungen enthalten sowie deren Verwendung zur Herstellung von Arzneimitteln.

Die für die Reste $Y_1$ und $Y_2$ möglichen Alkanoyl- bzw. Alkanoyloxygruppen mit 1 bis 12 C-Atomen leiten sich insbesondere von gesättigten Carbonsäuren ab. Diese Reste können cyclisch, acyclisch, carbocyclisch oder heterocyclisch sein. Die bevorzugten Reste leiten sich von $C_1$- bis $C_9$-, insbesondere $C_2$- bis $C_5$-Alkancarbonsäuren wie beispielsweise Acetyl(oxy)-, Propionyl(oxy)-, Butyryl(oxy)- ab.
Als Aroyl(oxy)gruppen sind die Benzoyl(oxy)- und substituierte Benzoyl(oxy)gruppen bevorzugt.

Für $R_3$ und $R_4$ gelten die folgenden bevorzugten Kombinationen: $R_3$= H, $R_4$= Methyl oder $R_3$= Methyl, $R_4$=H; $R_3$= F, $R_4$= Methyl oder $R_3$= Methyl, $R_4$=F; $R_3$, $R_4$= Methyl; $R_3$ und $R_4$ bilden zusammen eine Methylengruppe oder gemeinsam mit dem tertiären Kohlenstoffatom 20 einen Cyclopropylring.

Für A und B gelten folgende bevorzugte Kombinationen:
A=OH, B=H oder A=H, B=OH sowie A und B bilden eine Carbonylgruppe.
Für $R_5$ und $R_6$ gelten folgende bevorzugte Kombinationen:
$R_5$, $R_6$= Methyl oder Ethyl; $R_5$ und $R_6$ bilden gemeinsam mit dem Kohlenstoffatom 25 einen Cyclopropyl-, Cyclobutyl-, Cyclopentyl- oder Cyclohexylring.
Besonders bevorzugt sind die Fälle $R_5$, $R_6$= Methyl sowie $R_5$ und $R_6$ bilden zusammen mit dem Kohlenstoffatom 25

einen Cyclopropylring.

Für Z gelten folgende Bevorzugungen:

Z= -C(O)-$R_9$ oder Z= -CH(OH)-$R_9$ (α- oder β-Hydroxy), wobei $R_9$ ein gerad- oder verzweigtkettiger, gesättigter oder ungesättigter Kohlenwasserstoffrest mit bis zu 12 Kohlenstoffatomen ist oder auch carbo- oder heterocyclisch sein kann oder derartige Partialstrukturen aufweisen kann und auch perfluoriert sein kann.

Für $R_9$ gelten folgende besondere Bevorzugungen:

$R_9$= Methyl, Ethyl, Propyl, Butyl, Pentyl, Hexyl, Heptyl, Octyl, i-Propyl, i-Butyl, t-Butyl, 1-Butenyl, 1-Pentenyl, 1-Butinyl, 1-Pentinyl, Phenyl, Furanyl, Pyridinyl, Trifluormethyl, Perfluorethyl, Perfluorpropyl, Perfluorbutyl, Perfluorpentyl oder Perfluorhexyl.

Weiterhin gilt für Z die folgende Bevorzugung:

Z= 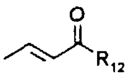 ,

mit $R_{12}$= $C_1$-$C_{10}$-Alkyl- oder Alkoxy (geradkettig, verzweigt, gesättigt, ungesättigt, cyclisch).

Besonders bevorzugt gemäß vorliegender Erfindung sind die folgenden Verbindungen:

(5*Z*,7*E*,22*E*)-(1*S*,3*R*,24*R*)-25-(1-Oxopentyl)-26,27-cyclo-9,10-secocholesta-5,7,10(19),22-tetraen-1,3,24-triol
(5*Z*,7*E*,22*E*)-(1*S*,3*R*,24*S*)-25-(1-Oxopentyl)-26, 27-cyclo-9,10-secocholesta-5,7,10(19),22-tetraen-1,3,24-triol
(5*Z*,7*E*,22*E*)-(1*S*,3*R*,24*R*)-25-Acetyl-26, 27-cyclo-9,10-secocholesta-5,7,10(19), 22-tetraen-1,3,24-triol
(5*Z*,7*E*,22*E*)-(1*S*,3*R*,24*S*)-25-Acetyl-26, 27-cyclo-9,10-secocholesta-5,7,10(19), 22-tetraen-1,3,24-triol
(5*Z*,7*E*,22*E*)-(1*S*,3*R*,24*R*)-25-(1-Oxopropyl)-26,27-cyclo-9,10-secocholesta-5,7,10(19),22-tetraen-1,3,24-triol
(5*Z*,7*E*,22*E*)-(1*S*,3*R*,24*S*)-25-(1-Oxopropyl)-26,27-cyclo-9,10-secocholesta-5,7,10(19),22-tetraen-1,3,24-triol
(5*Z*,7*E*, 22*E*)-(1*S*,3*R*,24*R*)-25-(1-Oxobutyl)-26,27-cyclo-9,10-secocholesta-5,7,10(19), 22-tetraen-1,3,24-triol
(5*Z*,7*E*,22*E*)-(1*S*,3*R*,24*S*)-25-(1-Oxobutyl)-26,27-cyclo-9,10-secocholesta-5,7,10(19), 22-tetraen-1,3,24-triol
(5*Z*,7*E*,22*E*)-(1*S*,3*R*,24*S*)-25-(1-Oxohexyl)-26,27-cyclo-9,10-secocholesta-5,7,10(19),22-tetraen-1,3,24-triol
(5*Z*,7*E*,22*E*)-(1*S*,3*R*,24*R*)-25-(1-Oxoheptyl)-26,27-cyclo-9,10-secocholesta-5,7,10(19),22-tetraen-1,3,24-triol
(5*Z*,7*E*,22*E*)-(1*S*,3*R*,24*S*)-25-(1-Oxoheptyl)-26,27-cyclo-9,10-secocholesta-5,7,10(19),22-tetraen-1,3,24-triol
(5*Z*,7*E*,22*E*)-(1*S*,3*R*,24*R*)-25-(1-Oxooctyl)-26,27-cyclo-9,10-secocholesta-5,7,10(19),22-tetraen-1,3,24-triol
(5*Z*,7*E*,22*E*)-(1*S*,3*R*,24*S*)-25-(1-Oxooctyl)-26,27-cyclo-9,10-secocholesta-5,7,10(19),22-tetraen-1,3,24-triol
(5*Z*,7*E*,22*E*)-(1*S*,3*R*,24*R*)-25-(1-Oxononyl)-26,27-cyclo-9,10-secocholesta-5,7,10(19),22-tetraen-1,3,24-triol
(5*Z*,7*E*,22*E*)-(1*S*,3*R*,24*S*)-25-(1-Oxononyl)-26,27-cyclo-9,10-secocholesta-5,7,10(19),22-tetraen-1,3,24-triol
(5*Z*,7*E*,22*E*)-(1*S*,3*R*,24*R*)-25-Benzoyl-26,27-cyclo-9,10-secocholesta-5,7,10(19),22-tetraen-1,3,24-triol
(5*Z*,7*E*,22*E*)-(1*S*,3*R*,24*S*)-25-Benzoyl-26,27-cyclo-9,10-secocholesta-5,7,10(19),22-tetraen-1,3,24-triol
(5*Z*,7*E*,22*E*)-(1*S*,3*R*,24*R*)-25-(2-Furanylcarbonyl)-26,27-cyclo-9,10-secocholesta-5,7,10(19),22-tetraen-1,3,24-triol
(5*Z*,7*E*,22*E*)-(1*S*,3*R*,24*S*)-25-(2-Furanylcarbonyl)-26,27-cyclo-9,10-secocholesta-5,7,10(19),22-tetraen-1,3,24-triol
(5*Z*,7*E*,22*E*)-(1*S*,3*R*,24*R*)-25-(2.2-Dimethyl-1-oxopropyl )-26.27-cyclo-9,10-secocholesta-5,7,10(19),22-tetraen-1,3,24-triol
(5*Z*,7*E*,22*E*)-(1*S*,3*R*,24*S*)-25-(2,2-Dimethyl-1-oxopropyl)-26,27-cyclo-9,10-secocholesta-5,7,10(19),22-tetraen-1,3,24-triol
(5*Z*,7*E*,22*E*)-(1*S*,3*R*,24*R*)-25-(2-Pyridinylcarbonyl)-26,27-cyclo-9,10-secocholesta-5,7,10(19),22-tetraen-1,3,24-triol
(5*Z*,7*E*,22*E*)-(1*S*,3*R*,24*S*)-25-(2-Pyridinylcarbonyl)-26.27-cyclo-9,10-secocholesta-5,7,10(19),22-tetraen-1,3,24-triol
[5*Z*,7*E*,22*E*,25(*E*)]-(1*S*,3*R*,24*R*)-25-(1-Oxo-2-hexenyl )-26,27-cyclo-9,10-secocholesta-5,7,10(19),22-tetraen-1,3,24-triol
[5*Z*,7*E*,22*E*,25(*E*)]-(1*S*,3*R*,24*S*)-25-(1-Oxo-2-hexenyl)-26,27-cyclo-9,10-secocholesta-5,7,10(19),22-tetraen-1,3,24-triol
(5*Z*,7*E*,22*E*)-(1*S*,3*R*,24*R*)-25-(1-Oxo-2-hexinyl)-26,27-cyclo-9,10-secocholesta-5,7,10(19),22-tetraen-1,3,24-triol
(5*Z*,7*E*,22*E*)-(1*S*,3*R*,24*S*)-25-(1-Oxo-2-hexinyl )-26,27-cyclo-9,10-secocholesta-5,7,10(19),22-tetraen-1,3,24-triol
(5*Z*,7*E*,22*E*)-(1*S*,3*R*,24*R*)-25-(Cyclopropylcarbonyl)-26,27-cyclo-9,10-secocholesta-5,7,10(19),22-tetraen-1,3,24-triol

(5*Z*,7*E*,22*E*)-(1*S*,3*R*,24*S*)-25-(Cyclopropylcarbonyl)-26,27-cyclo-9,10-secocholesta-5,7,10(19),22-tetraen-1,3,24-triol
(5*Z*,7*E*,22*E*,25(*E*)]-(1*S*,3*R*,24*R*)-25-(3-Ethoxy-3-oxo-1-propenyl )-26,27-cyclo-9,10-secocholesta-5,7,10(19),22-tetraen-1,3,24-triol
[5*Z*,7*E*,22*E*,25(*E*)]-(1*S*,3*R*,24*S*)-25-(3-Ethoxy-3-oxo-1-propenyl )-26,27-cyclo-9,10-secocholesta-5,7,10(19),22-tetraen-1,3,24-triol
[5*Z*,7*E*,22*E*,25(*E*)]-(1*S*,3*R*,24*R*)-25-[3-(1,1-Dimethylethoxy)-3-oxo-1-propenyl]-26,27-cyclo-9,10-secocholesta-5,7,10(19),22-tetraen-1,3,24-triol
[5*Z*,7*E*,22*E*,25(*E*)]-(1*S*,3*R*,24*S*)-25-[3-(1,1-Dimethylethoxy)-3-oxo-1-propenyl]-26,27-cyclo-9,10-secocholesta-5,7,10(19),22-tetraen-1,3,24-triol
[5*Z*,7*E*,22*E*,25(*E*)]-(1*S*,3*R*,24*R*)-25-(3-Propoxy-3-oxo-1-propenyl)-26,27-cyclo-9,10-secocholesta-5,7,10(19),22-tetraen-1,3,24-triol
[5*Z*,7*E*,22*E*,25(*E*)]-(1*S*,3*R*,24*S*)-25-(3-Propoxy-3-oxo-1-propenyl )-26,27-cyclo-9,10-secocholesta-5,7,10(19),22-tetraen-1,3,24-triol
[5*Z*,7*E*,22*E*,25(*E*)]-(1*S*,3*R*,24*R*)-25-(3-Butoxy-3-oxo-1-propenyl)-26,27-cyclo-9,10-secocholesta-5,7,10(19),22-tetraen-1,3,24-triol
[5*Z*,7*E*,22*E*,25(*E*)]-(1*S*,3*R*,24*S*)-25-(3-Butoxy-3-oxo-1-propenyl)-26,27-cyclo-9,10-secocholesta-5,7,10(19),22-tetraen-1,3,24-triol
(5*Z*,7*E*,22*E*)-[1*S*,3*R*,24*S*,25(*S*)]-25-(1-Hydroxy-2,2,3,3,4,4,5,5,5-nonafluorpentyl)-26,27-cyclo-9,10-secocholesta-5,7,10(19),22-tetraen-1,3,24-triol
(5*Z*,7*E*,22*E*)-(1*S*,3*R*,24*S*,25(*R*)]-25-(1-Hydroxy-2,2,3,3,4,4,5,5,5-nonafluorpentyl)-26,27-cyclo-9,10-secocholesta-5,7,10(19),22-tetraen-1,3,24-triol
(5*Z*,7*E*,22*E*)-[1*S*,3*R*,24*R*,25(*S*)]-25-(1-Hydroxy-2,2,3,3,4,4,5,5,5-nonafluorpentyl )-26,27-cyclo-9,10-secocholesta-5,7,10(19),22-tetraen-1,3,24-triol
(5*Z*,7*E*,22*E*)-[1*S*,3*R*,24*R*,25(*R*)]-25-(1-Hydroxy-2,2,3,3,4,4,5.5,5-nonafluorpentyl)-26.27-cyclo-9,10-secocholesta-5,7,10(19).22-tetraen-1,3,24-triol
(5*Z*,7*E*,22*E*)-[1*S*,3*R*,24*S*,25(*S*)]-25-(1-Hydroxy-2,2,3,3,4,4,5,5,6,6,7,7,7-tridecafluorheptyl)-26,27-cyclo-9,10-secocholesta-5,7,10(19),22-tetraen-1,3,24-triol
(5*Z*,7*E*,22*E*)-[1*S*,3*R*,24*S*,25(*R*)]-25-(1-Hydroxy-2,2,3,3,4,4,5,5,6,6,7,7,7-tridecafluorheptyl)-26,27-cyclo-9,10-secocholesta-5,7,10(19),22-tetraen-1,3,24-triol
(5*Z*,7*E*,22*E*)-[1*S*,3*R*,24*R*,25(*S*)]-25-(1-Hydroxy-2,2,3,3,4,4,5,5,6,6,7,7,7-tridecafluorheptyl)-26,27-cyclo-9,10-secocholesta-5,7,10(19),22-tetraen-1,3,24-triol
(5*Z*,7*E*,22*E*)-[1*S*,3*R*,24*R*,25(*R*)]-25-(1-Hydroxy-2,2,3,3,4,4,5,5,6,6,7,7,7-tridecafluorheptyl)-26,27-cyclo-9,10-secocholesta-5,7,10( 19),22-tetraen-1,3,24-triol
(5*Z*,7*E*,22*E*)-(1*S*,3*R*,24*R*)-25-(Trifluoracetyl)-26,27-cyclo-9,10-secocholesta-5,7,10(19),22-tetraen-1,3,24-triol
(5*Z*,7*E*,22*E*)-(1*S*,3*R*,24*S*)-25-(Trifluoracetyl)-26,27-cyclo-9,10-secocholesta-5,7,10(19),22-tetraen-1,3,24-triol
(5*Z*,7*E*,22*E*)-(1*S*,3*R*,24*R*)-25-(Perfluorethylcarbonyl)-26,27-cyclo-9,10-secocholesta-5,7,10(19),22-tetraen-1,3,24-triol
(5*Z*,7*E*,22*E*)-(1*S*,3*R*,24*S*)-25-(Perfluorethylcarbonyl)-26,27-cyclo-9,10-secocholesta-5,7,10(19),22-tetraen-1,3,24-triol
(5*Z*,7*E*,22*E*)-(1*S*,3*R*,24*R*)-25-(Perfluorpropylcarbonyl)-26,27-cyclo-9,10-secocholesta-5,7,10( 19),22-tetraen-1,3,24-triol
(5*Z*,7*E*,22*E*)-(1*S*,3*R*,24*S*)-25-(Perfluorpropylcarbonyl)-26,27-cyclo-9,10-secocholesta-5,7,10(19),22-tetraen-1,3,24-triol
(5*Z*,7*E*,22*E*)-(1*S*,3*R*,24*R*)-25-(Perfluorbutylcarbonyl)-26,27-cyclo-9,10-secocholesta-5,7,10(19),22-tetraen-1,3,24-triol
(5*Z*,7*E*,22*E*)-(1*S*,3*R*,24*S*)-25-(Perfluorbutylcarbonyl)-26,27-cyclo-9,10-secocholesta-5,7,10(19).22-tetraen-1,3,24-triol
(5*Z*,7*E*,22*E*)-(1*S*,3*R*,24*R*)-25-(Perfluorpentylcarbonyl)-26,27-cyclo-9,10-secocholesta-5,7,10(19),22-tetraen-1,3,24-triol
(5*Z*,7*E*,22*E*)-(1*S*,3*R*,24*S*)-25-(Perfluorpentylcarbonyl )-26,27-cyclo-9,10-secocholesta-5,7,10(19),22-tetraen-1,3,24-triol
(5*Z*,7*E*,22*E*)-(1*S*,3*R*,24*R*)-25-(Perfluorhexylcarbonyl )-26,27-cyclo-9,10-secocholesta-5,7,10(19),22-tetraen-1,3,24-triol
(5*Z*,7*E*,22*E*)-(1*S*,3*R*,24*S*)-25-(Perfluorhexylcarbonyl)-26,27-cyclo-9,10-secocholesta-5,7,10(19),22-tetraen-1,3,24-triol
(5*Z*,7*E*,22*E*)-(1*S*,3*R*,24*R*)-25-Acetyl-20-methyl-26,27-cyclo-9,10-secocholesta-5,7,10(19),22-tetraen-1,3,24-triol
(5*Z*,7*E*,22*E*)-(1*S*,3*R*,24*S*)-25-Acetyl-20-methyl-26,27-cyclo-9,10-secocholesta-5,7,10(19),22-tetraen-1,3,24-triol

(5*Z*,7*E*,22*E*)-(1*S*,3*R*,24*R*)-20-Methyl-25-(1-oxopropyl)-26,27-cyclo-9,10-secocholesta-5,7,10(19),22-tetraen-1,3,24-triol
(5*Z*,7*E*,22*E*]-(1*S*,3*R*,24*S*)-20-Methyl-25-(1-oxopropyl)-26,27-cyclo-9,10-secocholesta-5.7,10( 19),22-tetraen-1,3,24-triol
(5*Z*,7*E*,22*E*)-(1*S*,3*R*,24*R*)-20-Methyl-25-(1-oxobutyl)-26,27-cyclo-9,10-secocholesta-5,7,10(19),22-tetraen-1,3,24-triol
(5*Z*,7*E*,22*E*)-(1*S*,3*R*,24*S*)-20-Methyl-25-(1-oxobutyl )-26,27-cyclo-9,10-secocholesta-5,7,10(19),22-tetraen-1,3,24-triol
(5*Z*,7*E*,22*E*)-(1*S*,3*R*,24*R*)-20-Methyl-25-(1-oxopentyl)-26,27-cyclo-9,10-secocholesta-5,7,10(19),22-tetraen-1,3,24-triol
(5*Z*,7*E*,22*E*)-(1*S*,3*R*,24*S*)-20-Methyl-25-(1-oxopentyl)-26,27-cyclo-9,10-secocholesta-5,7,10(19),22-tetraen-1,3,24-triol
(5*Z*,7*E*,22*E*)-(1*S*,3*R*,24*R*)-20-Methyl-25-(1-oxohexyl)-26,27-cyclo-9,10-secocholesta-5,7,10(19),22-tetraen-1,3,24-triol
(5*Z*,7*E*,22*E*)-(1*S*,3*R*,24*S*)-20-Methyl-25-(1-oxohexyl)-26,27-cyclo-9,10-secocholesta-5,7,10(19),22-tetraen-1,3,24-triol
(5*Z*,7*E*,22*E*)-(1*S*,3*R*,24*R*)-20-Methyl-25-(1-oxoheptyl)-26,27-cyclo-9,10-secocholesta-5,7,10(19),22-tetraen-1,3,24-triol
(5*Z*,7*E*,22*E*)-(1*S*,3*R*,24*S*)-20-Methyl-25-(1-oxoheptyl)-26,27-cyclo-9,10-secocholesta-5,7,10(19),22-tetraen-1,3,24-triol
(5*Z*,7*E*,22*E*)-(1*S*,3*R*,24*R*)-20-Methyl-25-(1-oxooctyl)-26,27-cyclo-9,10-secocholesta-5,7,10(19),22-tetraen-1,3,24-triol
(5*Z*,7*E*,22*E*)-(1*S*,3*R*,24*S*)-20-Methyl-25-(1-oxooctyl)-26,27-cyclo-9,10-secocholesta-5,7,10(19),22-tetraen-1,3,24-triol
(5*Z*,7*E*,22*E*)-(1*S*,3*R*,24*R*)-20-Methyl-25-(1-oxononyl)-26,27-cyclo-9,10-secocholesta-5,7,10(19),22-tetraen-1,3,24-triol
(5,*Z*,7*E*,22*E*)-(1*S*,3*R*,24*S*)-20-Methyl-25-(1-oxononyl)-26,27-cyclo-9,10-secocholesta-5,7,10(19),22-tetraen-1,3,24-triol
(7*E*,22*E*)-(1*R*,3*R*,24*R*)-25-Acetyl-26,27-cyclo-19-nor-9,10-secocholesta-5,7,22-trien-1,3,24-triol
(7*E*,22*E*)-(1*R*,3*R*,24*S*)-25-Acetyl-26,27-cyclo-19-nor-9,10-secocholesta-5,7,22-trien-1,3,24-triol
(7*E*,22*E*)-(1*R*,3*R*,24*R*)-25-(1-Oxopropyl)-26.27-cyclo-19-nor-9,10-secocholesta-5,7,22-trien-1,3,24-triol
(7*E*,22*E*)-(1*R*,3*R*,24*S*)-25-(1-Oxopropyl)-26.27-cyclo-19-nor-9,10-secocholesta-5,7,22-trien-1,3,24-triol
(7*E*,22*E*)-(1*R*,3*R*,24*R*)-25-(1-Oxobutyl)-26,27-cyclo-19-nor-9,10-secocholesta-5,7,22-trien-1,3,24-triol
(7*E*,22*E*)-(1*R*,3*R*,24*S*)-25-(1-Oxobutyl)-26,27-cyclo-19-nor-9,10-secocholesta-5,7,22-trien-1,3,24-triol
(7*E*,22*E*)-(1*R*,3*R*,24*R*)-25-(1-Oxopentyl)-26,27-cyclo-19-nor-9,10-secocholesta-5,7,22-trien-1,3,24-triol
(7*E*,22*E*)-(1*R*,3*R*,24*S*)-25-(1-Oxopentyl)-26,27-cyclo-19-nor-9,10-secocholesta-5,7,22-trien-1,3,24-triol
(7E,22*E*)-(1*R*,3*R*,24*R*)-25-(1-Oxohexyl)-26,27-cyclo-19-nor-9,10-secocholesta-5,7,22-trien-1,3,24-triol
(7*E*,22*E*)-(1*R*,3*R*,24*S*)-25-(1-Oxohexyl)-26,27-cyclo-19-nor-9,10-secocholesta-5,7,22-trien-1,3,24-triol
(7*E*,22*E*)-(1*R*,3*R*,24*R*)-25-(1-Oxoheptyl )-26,27-cyclo-19-nor-9,10-secocholesta-5,7,22-trien-1,3,24-triol
(7*E*,22*E*)-(1*R*,3*R*,24*S*)-25-(1-Oxoheptyl )-26,27-cyclo-19-nor-9,10-secocholesta-5,7,22-trien-1,3,24-triol
(7*E*,22*E*)-(1*R*,3*R*,24*R*)-25-(1-Oxooctyl )-26,27-cyclo-19-nor-9,10-secocholesta-5,7,22-trien-1,3,24-triol
(7*E*,22*E*)-(1*R*,3*R*,24*S*)-25-(1-Oxooctyl)-26,27-cyclo-19-nor-9,10-secocholesta-5,7,22-trien-1,3,24-triol
(7*E*,22*E*)-(1*R*,3*R*,24*R*)-25-(1-Oxononyl)-26,27-cyclo-19-nor-9,10-secocholesta-5,7,22-trien-1,3,24-triol
(7*E*,22*E*)-(1*R*,3*R*,24*S*)-25-(1-Oxononyl)-26,27-cyclo-19-nor-9,10-secocholesta-5,7,22-trien-1,3,24-triol
(7*E*,22*E*)-(1*R*,3*R*,24*R*)-25-Acetyl-20-methyl-26,27-cyclo-19-nor-9,10-secocholesta-5,7,22-trien-1,3,24-triol
(7*E*,22*E*)-(1*R*,3*R*,24*S*)-25-Acetyl-20-methyl-26,27-cyclo-19-nor-9,10-secocholesta-5,7,22-trien-1,3,24-triol
(7*E*,22*E*)-(1*R*,3*R*,24*R*)-20-Methyl-25-(1-oxopropyl)-26,27-cyclo-19-nor-9,10-secocholesta-5,7,22-trien-1,3,24-triol
(7*E*,22*E*)-(1*R*,3*R*,24*S*)-20-Methyl-25-(1-oxopropyl)-26,27-cyclo-19-nor-9,10-secocholesta-5,7,22-trien-1,3,24-triol
(7*E*,22*E*)-(1*R*,3*R*,24*R*)-20-Methyl-25-(1-oxobutyl)-26,27-cyclo-19-nor-9,10-secocholesta-5,7,22-trien-1,3,24-triol
(7*E*,22*E*)-(1*R*,3*R*,24*S*)-20-Methyl-25-(1-oxobutyl)-26,27-cyclo-19-nor-9,10-secocholesta-5,7,22-trien-1,3,24-triol
(7*E*,22*E*)-(1*R*,3*R*,24*R*)-20-Methyl-25-(1-oxopentyl)-26,27-cyclo-19-nor-9,10-secocholesta-5,7,22-trien-1,3,24-triol
(7*E*,22*E*)-(1*R*,3*R*,24*S*)-20-Methyl-25-(1-oxopentyl)-26,27-cyclo-19-nor-9,10-secocholesta-5,7,22-trien-1,3,24-triol
(7*E*,22*E*)-(1*R*,3*R*,24*R*)-20-Methyl-25-(1-oxohexyl)-26,27-cyclo-19-nor-9,10-secocholesta-5,7,22-trien-1,3,24-triol
(7*E*,22*E*)-(1*R*,3*R*,24*S*)-20-Methyl-25-(1-oxohexyl)-26,27-cyclo-19-nor-9,10-secocholesta-5,7,22-trien-1,3,24-triol

(7*E*,22*E*)-(1*R*,3*R*,24*R*)-20-Methyl-25-(1-oxoheptyl)-26,27-cyclo-19-nor-9,10-secocholesta-5,7,22-trien-1,3,24-triol
(7*E*,22*E*)-(1*R*,3*R*,24*S*)-20-Methyl-25-(1-oxoheptyl)-26,27-cyclo-19-nor-9,10-secocholesta-5,7,22-trien-1,3,24-triol
(7*E*,22*E*)-(1*R*,3*R*,24*R*)-20-Methyl-25-(1-oxooctyl)-26,27-cyclo-19-nor-9,10-secocholesta-5,7,22-trien-1,3,24-triol
(7*E*,22*E*)-(1*R*,3*R*,24*S*)-20-Methyl-25-(1-oxooctyl)-26,27-cyclo-19-nor-9,10-secocholesta-5,7,22-trien-1,3,24-triol
(7*E*,22*E*)-(1*R*,3*R*,24*R*)-20-Methyl-25-(1-oxononyl)-26,27-cyclo-19-nor-9,10-secocholesta-5,7,22-trien-1,3,24-triol
(7*E*,22*E*)-(1*R*,3*R*,24*S*)-20-Methyl-25-(1-oxononyl)-26,27-cyclo-19-nor-9,10-secocholesta-5,7,22-trien-1,3,24-triol
(5*Z*,7*E*,22*E*)-[1*S*,3*R*,25(*R*)]-1,3-Dihydroxy-25-(1-hydroxy-2,2,3,3,4,4,5,5,5-nonafluorpentyl)-26,27-cyclo-9,10-secocholesta-5,7,10(19),22-tetraen-24-on
(5*Z*,7*E*,22E)-[1*S*,3*R*,25(*S*)]-1,3-Dihydroxy-25-(1-hydroxy-2,2,3,3,4,4,5,5,5-nonafluorpentyl)-26,27-cyclo-9,10-secocholesta-5,7,10(19),22-tetraen-24-on
(5*Z*,7*E*,22*E*)-[1*S*,3*R*,25(*R*)]-1,3-Dihydroxy-25-(1-hydroxy-2,2,3,3,4,4,5,5,6,6,7,7,7-tridecafluorheptyl)-26,27-cyclo-9,10-secocholesta-5,7,10(19),22-tetraen-24-on
(5*Z*,7*E*,22*E*)-[1*S*,3*R*,25(S)]-1,3-Dihydroxy-25-(1-hydroxy-2,2,3,3,4,4,5,5,6,6,7,7,7-tridecafluorheptyl)-26,27-cyclo-9,10-secocholesta-5,7,10( 19),22-tetraen-24-on
(5*Z*,7*E*,22*E*)-(3*S*,24*R*)-25-Acetyl-26,27-cyclo-9,10-secocholesta-5,7,10(19),22-tetraen-3,24-diol
(5*Z*,7*E*,22*E*)-(3*S*,24*S*)-25-Acetyl-26.27-cyclo-9,10-secocholesta-5,7,10(19),22-tetraen-3,24-diol
(5*Z*,7*E*,22*E*)-(3*S*,24*R*)-25-(1-Oxopropyl)-26.27-cyclo-9,10-secocholesta-5,7,10(19),22-tetraen-3,24-diol
(5*Z*,7*E*,22*E*)-(3*S*,24*S*)-25-(1-Oxopropyl)-26,27-cyclo-9,10-secocholesta-5,7,10(19),22-tetraen-3,24-diol
(5*Z*,7*E*,22*E*)-(3*S*,24*R*)-25-(1-Oxobutyl)-26,27-cyc]o-9,10-secocholesta-5,7,10(19),22-tetraen-3,24-diol
(5*Z*,7*E*,22*E*)-(3*S*,24*S*)-25-(1-Oxobutyl)-26,27-cyclo-9,10-secocholesta-5,7,10(19),22-tetraen-3,24-diol
(5*Z*,7*E*,22*E*)-(3*S*,24*R*)-25-(1-Oxopentyl)-26,27-cyclo-9,10-secocholesta-5,7,10(19),22-tetraen-3,24-diol
(5*Z*,7*E*,22*E*)-(3*S*,24*R*)-25-(1-Oxopentyl)-26,27-cyclo-9,10-secocholesta-5,7,10(19),22-tetraen-3,24-diol
(5*Z*,7*E*,22*E*)-(3*S*,24*S*)-25-(1-Oxopentyl)-26,27-cyclo-9,10-secocholesta-5,7,10(19),22-tetraen-3,24-diol
(5*Z*,7*E*,22*E*)-(3*S*,24*R*)-25-(1-Oxohexyl)-26,27-cyclo-9,10-secocholesta-5,7,10(19),22-tetraen-3,24-diol
(5*Z*,7*E*,22*E*)-(3*S*,24*S*)-25-(1-Oxohexyl)-26,27-cyclo-9,10-secocholesta-5,7,10(19),22-tetraen-3,24-diol
(5*Z*,7*E*,22*E*)-(3*S*,24*R*)-25-(1-Oxoheptyl)-26,27-cyclo-9,10-secocholesta-5,7,10(19),22-tetraen-3,24-diol
(5*Z*,7*E*,22*E*)-(3*S*,24*S*)-25-(1-Oxoheptyl)-26,27-cyclo-9,10-secocholesta-5,7,10(19),22-tetraen-3,24-diol
(5*Z*,7*E*,22*E*)-(3*S*, 24*R*)-25-(1-Oxooctyl)-26,27-cyclo-9,10-secocholesta-5,7,10(19),22-tetraen-3,24-diol
(5*Z*,7*E*,22*E*)-(3*S*, 24*S*)-25-(1-Oxooctyl)-26, 27-cyclo-9,10-secocholesta-5,7,10(19),22-tetraen-3,24-diol
(5*Z*,7*E*,22*E*)-(3*S*, 24*R*)-25-(1-Oxononyl)-26,27-cyclo-9,10-secocholesta-5,7,10(19),22-tetraen-3,24-diol
(5*Z*,7*E*,22*E*)-(3*S*,24*S*)-25-(1-Oxononyl)-26,27-cyclo-9,10-secocholesta-5,7,10(19),22-tetraen-3,24-diol
(5*Z*,7*E*,22*E*)-(1*S*,3*R*,24*R*)-25-Hydroxymethyl-26, 27-cyclo-9,10-secocholesta-5,7,10(19),22-tetraen-1,3,24-triol
(5*Z*,7*E*,22*E*)-(1*S*,3*R*, 24*S*)-25-Hydroxymethyl-26,27-cyclo-9,10-secocholesta-5,7,10(19),22-tetraen-1,3,24-triol
[5*Z*,7*E*,22*E*,25(*E*)]-(1*S*,3*R*,24*R*)-25-(3-Oxo-1-heptenyl)-26,27-cyclo-9,10-secocholesta-5,7,10(19),22-tetraen-1,3,24-triol
[5*Z*,7*E*,22*E*,25(*E*)]-(1*S*,3*R*,24*S*)-25-(3-Oxo-1-heptenyl)-26,27-cyclo-9,10-secocholesta-5,7,10(19),22-tetraen-1,3,24-triol
[5*Z*,7*E*,22*E*,25(*E,E*)]-(1*S*,3*R*,24*R*)-25-(1-Oxo-2,4-hexadienyl)-26,27-cyclo-9,10-secocholesta-5,7,10(19),22-tetraen-1,3,24-triol
[5*Z*,7*E*,22*E*,25(*E,E*)]-(1*S*,3*R*,24*S*)-25-(1-Oxo-2,4-hexadienyl)-26,27-cyclo-9,10-secocholesta-5,7,10(19),22-tetraen-1,3,24-triol

Die natürlichen Vitamine $D_2$ und $D_3$ (vergl. allgemeine Formel Vitamin D) sind an sich biologisch inaktiv und werden erst nach Hydroxylierung am C-Atom 25 in der Leber und am C-Atom 1 in der Niere in biologisch aktive Metaboliten [1α,25-Dihydroxyvitamin $D_3$ (Calcitriol) bzw. -$D_2$] umgewandelt. Die Wirkung der aktiven Metaboliten besteht in der Regulation der Calcium- und Phosphatkonzentration im Serum; sie wirken einem Absinken der Calciumkonzentration im Serum entgegen, indem sie die Calciumabsorption im Darm erhöhen und unter bestimmten Umständen die Calciummobilisation aus dem Knochen fördern.

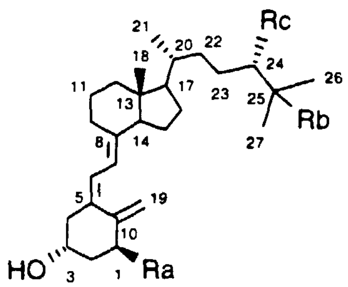

| | |
|---|---|
| Ergocalciferol: Ra=Rb=H, Rc=$CH_3$<br>Doppelbindung C-22/23 | Vitamin $D_2$ |
| Cholecalciferol: Ra=Rb=Rc=H<br>25-Hydroxycholecalciferol: Ra=Rc=H, Rb=OH<br>1α-Hydroxycholecalciferol: Ra=OH, Rb=Rc=H | Vitamin $D_3$ |
| 1α,25-Dihydroxycholecalciferol: Ra=Rb=OH, Rc=H | Calcitriol |

Neben ihrer ausgeprägten Wirkung auf den Calcium- und Phosphatstoffwechsel besitzen die aktiven Metaboliten von Vitamin $D_2$ und $D_3$ und seine synthetischen Abkömmlinge eine proliferationshemmende und differenzierungsstimuliernde Wirkung auf Tumorzellen und normale Zellen, wie zum Bespiel Hautzellen. Weiterhin wurde eine ausgeprägte Wirkung auf Zellen des Immunsystems (Hemmung der Proliferation und Interleukin 2-Synthese von Lymphocyten, Steigerung der Cytotoxizität und Phagocytose in vitro von Monocyten) gefunden, die sich in einer immunmodulatorischen Wirkung äußert, schließlich wird infolge einer fördernden Wirkung auf knochenbildende Zellen eine vermehrte Knochenbildung bei normalen und osteoporotischen Ratten gefunden [R. Bouillon et al. "Short term course of 1,25$(OH)_2D_3$ stimulates osteoblasts but not osteoclasts" . *Calc. Tissue Int.* **49**, 168-173 (1991)]

Alle Wirkungen werden durch Bindung an den Vitamin D-Rezeptor vermittelt. Infolge der Bindung wird die Aktivität von spezifischen Genen reguliert.

Bei Anwendung der biologisch aktiven Metabolite von Vitamin $D_2$ und $D_3$ wird eine toxische Wirkung auf den Calciumstoffwechsel hervorgerufen (Hypercalcämie).

Durch strukturelle Manipulationen der Seitenkette können therapeutisch nutzbare Wirkqualitäten von der unerwünschten hypercalcämischen Aktivität abgetrennt werden. Eine geeignete Strukturvariation ist die Einführung von 24-Hydroxy-Derivaten.

In 24-Stellung hydroxylierte 1α-Cholecalciferole gehen bereits aus der DE 25 26 981 hervor. Sie besitzen eine geringere Toxizität als das entsprechende nicht-hydroxylierte 1α-Cholecalciferol. Darüberhinaus sind 24-Hydroxy-Derivate in folgenden Patentanmeldungen beschrieben: DE 39 33 034, DE 40 03 854, DE 40 34 730, EP 0 421 561, EP 0 441 467, WO 91/12238.

Schließlich werden in der WO 94/07853 an C 24 hydroxylierte 25-Carbonsäure-Derivate von Calcitriol beschrieben, die ein günstigeres Wirkspektrum als Calcitriol aufweisen. Während die Fähigkeit zur Auslösung einer Hypercalcämie deutlich abgeschwächt ist, bleiben die proliferationshemmenden und differenzierungsstimuliernden Wirkungen erhalten.

Gegenüber diesen strukturell verwandten Verbindungen zeichnen sich die erfindungsgemäßen Substanzen dadurch aus, daß sie eine stärkere Wirkung auf die Zelldifferenzierung zeigen, wobei die Wirkung auf den Calciumhaushalt nicht zunimmt.

Die Vitamin D-Aktivität der erfindungsgemäßen Substanzen wird mittels des Calcitriol-Rezeptortests bestimmt. Er wird unter Verwendung eines spezifischen Rezeptorproteins aus dem Darm von jungen Schweinen durchgeführt.

Rezeptorhaltiges Bindungsprotein wird mit $^3$H-Calcitriol ($5x10^{-10}$ mol/l) in einem Reaktionsvolumen von 0,270 ml in Abwesenheit und in Anwesenheit der Prüfsubstanzen für zwei Stunden bei 4°C in einem Teströhrchen inkubiert. Zur Trennung von freiem und rezeptorgebundenem Calcitriol wird eine Charcoal-Dextran-Absorption durchgeführt. Dazu werden 250 µl einer Charcoal-Dextran-Suspension jedem Teströhrchen zugeführt und bei 4°C für 20 Min. inkubiert. Anschließend werden die Proben bei 10 000 x g 5 Minuten bei 4°C zentrifugiert. Der Überstand wird dekantiert und nach lstündiger Äquilibrierung in Picofluor 15 ™ in einem β-Zähler gemessen.

Die mit verschiedenen Konzentrationen der Prüfsubstanz sowie der Referenzsubstanz (unmarkiertes Calcitriol) bei konstanter Konzentration der Bezugssubstanz ($^3$H-Calcitriol) erhaltenen Kompetitionskurven werden in Beziehung zueinander gesetzt und ein Kompetitionsfaktor (KF) ermittelt.

Er ist definiert als Quotient aus den Konzentrationen der jeweiligen Prüfsubstanz und der Referenzsubstanz, die für 50%ige Kompetition erforderlich sind:

$$KF = \frac{\text{Konzentration Prüfsubstanz bei 50\% Kompetition}}{\text{Konzentration Referenzsubstanz bei 50\% Kompetition}}$$

Den erfindungsgemäßen Verbindungen ist gemein, daß sie alle über eine beträchtliche Affinität zum Calcitriol-Rezeptor verfügen.

Zur Bestimmung der akuten hypercalcämischen Wirkung verschiedener Calcitriolderivate wird nachfolgend beschriebener Test durchgeführt:

Die Wirkung von Kontrolle (Lösungsgrundlage), Referenzsubstanz (1,25$(OH)_2$-$D_3$=Calcitriol) und Testsubstanz wird jeweils nach einmaliger subcutaner Applikation in Gruppen von 10 gesunden männlichen Ratten (140-170 g) getestet. Die Ratten werden während der Versuchszeit in speziellen Käfigen zur Bestimmung der Exkretion von Wasser und Mineralstoffen gehalten. Der Harn wird in 2 Fraktionen (0-16 h und 16-22 h) gesammelt. Eine orale Calciumgabe (0.1 mM Calcium in 6,5% Alpha-hydroxypropylcellulose. 5 ml/Tier) ersetzt zum Zeitpunkt 16 h die durch Futterentzug fehlende Calciumaufnahme. Zu Versuchsende werden die Tiere durch Dekapitieren getötet und für die Bestimmung der Serum-Calciumwerte entblutet. Für die primäre Screen-Untersuchung in vivo wird eine einzelne Standarddosis (200 µg/kg) getestet. Für ausgewählte Substanzen wird das Ergebnis durch Erstellung einer Dosis-Wirkungs-Beziehung abgesichert.

Eine hypercalcämische Wirkung zeigt sich in im Vergleich zur Kontrolle erhöhten Serum-Calciumspiegel-Werten.

Die Signifikanz auftretender Unterschiede zwischen Substanzgruppen und Kontrollen sowie zwischen Testsubstanz und Referenzsubstanz werden mit geeigneten statistischen Verfahren abgesichert. Das Ergebnis wird als Dosisrelation DR (DR = Faktor Testsubstanzdosis/Referenzsubstanzdosis für vergleichbare Wirkungen) angegeben.

Die differenzierungsstimulierende Wirkung von Calcitriolanaloga wird ebenfalls quantitativ erfaßt. Es ist literaturbekannt (Mangelsdorf, D.J. et al., *J. Cell. Biol.* <u>98</u>: 391-398 (1984)), daß die Behandlung humaner Leukämiezellen (Promyelozytenzellinie HL 60) in vitro mit Calcitriol die Differenzierung der Zellen zu Makrophagen induziert.

HL 60-Zellen werden in Gewebekulturmedium (RPMI 10% fetales Kälberserum) bei 37°C in einer Atmosphäre 5% $CO_2$ in Luft kultiviert.

Zur Substanztestung werden die Zellen abzentrifugiert und 2,0 x $10^5$ Zellen/ml in phenolrotfreiem Gewebekulturmedium aufgenommen. Die Testsubstanzen werden in Ethanol gelöst und mit Gewebekulturmedium ohne Phenolrot auf die gewünschte Konzentration verdünnt. Die Verdünnungsstufen werden mit der Zellsuspension in einem Verhältnis von 1:10 gemischt und je 100 µl dieser mit Substanz versetzten Zellsuspension in eine Vertiefung einer 96-Loch-Platte pipettiert. Zur Kontrolle wird eine Zellsuspension analog mit dem Lösungsmittel versetzt.

Nach Inkubation über 96 Stunden bei 37°C in 5% $CO_2$ in Luft wird in jede Vertiefung der 96-Loch-Platte zu der Zellsuspension 100 µl einer NBT-TPA-Lösung (Nitroblautetrazolium (NBT), Endkonzentration im Ansatz 1 mg/ml, Tetradecanoylphorbolmyristat-13-acetat (TPA), Endkonzentration im Ansatz 2 x $10^{-7}$ mol/l) pipettiert.

Durch Inkubation über 2 Stunden bei 37°C und 5% $CO_2$ in Luft wird infolge der intrazellulären Sauerstoffradikalfreisetzung, stimuliert durch TPA, in den zu Makrophagen differenzierten Zellen NBT zu unlöslichem Formazan reduziert.

Zur Beendigung der Reaktion werden die Vertiefungen der 96-Loch-Platte abgesaugt und die Zellen durch Zugabe von Methanol am Plattenboden fixiert und nach Fixation getrocknet. Zur Lösung der gebildeten intrazellulären Formazankristalle werden in jede Vertiefung 100 µl Kaliumhydroxid (2 mol/l) und 100 µl Dimethylsulfoxid pipettiert und 1 Minute ultrabeschallt. Die Konzentration von Formazan wird spektralphotometrisch bei 650 nm gemessen.
Als Maß für die Differenzierungsinduktion der HL 60-Zellen zu Makrophagen gilt die Konzentration an gebildetem Formazan. Das Ergebnis wird als Dosisrelation (DR = Faktor Testsubstanzdosis/Referenzsubstanzdosis für vergleichbare halbmaximale Wirkungen) angegeben.

Die Ergebnisse des Calcitriol-Rezeptortests sowie der Bestimmung der Dosisrelation der Differenzierungsinduktion von HL 60-Zellen und der Dosisrelation für Hypercalcämie sind nachfolgend zusammengefaßt (Tab. 1):

Ausgewählte Testverbindungen :

(5*Z*,7*E*,22*E*)-(1*S*,3*R*,24*R*)-25-Acetyl-26,27-cyclo-9,10-secocholesta-5,7,10(19),22-tetraen-1,3,24-triol **<u>7b</u>**
(5*Z*,7*E*,22*E*)-(1*S*,3*R*,24*R*)-25-(1-Oxobutyl)-26,27-cyclo-9,10-secocholesta-5,7,10(19),22-tetraen-1,3,24-triol **<u>8b</u>**
(5*Z*,7*E*,22*E*)-(1*S*,3*R*,24*R*)-25-Benzoyl-26,27-cyclo-9,10-secocholesta-5,7,10(19),22-tetraen-1,3,24-triol **<u>10b</u>**
(5*Z*,7*E*,22*E*)-(1*S*,3*R*,24*R*)-25-(Cyclopropylcarbonyl)-26,27-cyclo-9,10-secocholesta-5,7,10(19),22-tetraen-1,3,24-triol **<u>12b</u>**
(5*Z*,7*E*,22*E*)-(1*S*,3*R*,24*R*)-25-(2,2-Dimethyl-1-oxopropyl)-26,27-cyclo-9,10-secocholesta-5,7,10(19),22-tetraen-1,3,24-triol **<u>15b</u>**
(5*Z*,7*E*,22*E*)-(1*S*,3*R*,24*R*)-25-(1-Oxo-2-hexinyl)-26,27-cyclo-9,10-secocholesta-5,7,10(19),22-tetraen-1,3,24-triol **<u>19b</u>**
(5*Z*,7*E*,22*E*)-(1*S*,3*R*,24*S*)-25-Acetyl-20-methyl-26,27-cyclo-9,10-secocholesta-5,7,10(19),22-tetraen-1,3,24-triol **<u>33a</u>**
(5*Z*,7*E*,22*E*)-(1*S*,3*R*,24*R*)-25-Acetyl-20-methyl-26,27-cyclo-9,10-secocholesta-5,7,10(19),22-tetraen-1,3,24-triol **<u>33b</u>**

(5*Z*,7*E*,22*E*)-(1*S*,3*R*,24*S*)-20-Methyl-25-(1-oxobutyl)-26,27-cyclo-9,10-secocholesta-5,7,10(19),22-tetraen-1,3,24-triol **35a**
(5*Z*,7*E*,22*E*)-(1*S*,3*R*,24*R*)-20-Methyl-25-(1-oxobutyl)-26,27-cyclo-9,10-secocholesta-5,7,10(19),22-tetraen-1,3,24-triol **35b**
(7*E*,22*E*)-(1*R*,3*R*,24*R*)-25-Acetyl-26,27-cyclo-19-nor-9,10-secocholesta-5,7,22-trien-1,3,24-triol **55b**
(7*E*,22*E*)-(1*R*,3*R*,24*S*)-25-Acetyl-20-methyl-26,27-cyclo-19-nor-9,10-secocholesta-5,7,22-trien-1,3,24-triol **81a**
(7*E*,22*E*)-(1*R*,3*R*,24*R*)-25-Acetyl-20-methyl-26,27-cyclo-19-nor-9,10-secocholesta-5,7,22-trien-1,3,24-triol **81b**

Vergleichsverbindung :
Calcitriol

Tab. 1

| Verbindung | Kompetitionsfaktor KF für Rezeptorbindung | Dosisrelation für Differenzierungsinduktion in HL 60-Zellen |
|---|---|---|
| **7b** | 1 | 0,5 |
| **8b** | 2 | 0,9 |
| **10b** | 2 | 6 |
| **12b** | 2 | 0,2 |
| **15b** | 6 | 1,1 |
| **19b** | 2 | 1,5 |
| **33a** | 4 | 0,5 |
| **33b** | 4 | 0,4 |
| **35a** | 7 | 0,3 |
| **35b** | 2 | 0,2 |
| **55b** | 4,5 | 0,2 |
| **81a** | 10 | 0,2 |
| **81b** | 3 | 0,1 |
| Calcitriol | 1 | 1 |

Die aufgeführten Verbindungen zeigen neben einer Affinität zum Vitamin D-Rezeptor, die der von Calcitriol vergleichbar ist, zum Teil eine stärkere zelldifferenzierende Aktivität.
Die Induktion einer Hypercalcämie erfolgt dagegen erst bei sehr viel höheren Dosen als bei Calcitriol (z. B. Dosisrelation für **7b** = 300, **8b** = 100, **15b** = 300, **19b** > 300, Calcitriol DR =1).
Durch die verminderte Eigenschaft, eine Hypercalcämie auszulösen, eignen sich die erfindungsgemäßen Substanzen in besonderer Weise zur Herstellung von Arzneimitteln für die Behandlung von Erkrankungen. die durch eine Hyperproliferation und fehlende Zelldifferenzierung gekennzeichnet sind. Dazu zählen zum Beispiel hyperproliferative Erkrankungen der Haut (Psoriasis, Pituriasis subia pilasis, Akne, Ichthyosis) sowie Tumorerkrankungen und Präkanzerosen (zum Beispiel Darmtumoren, Mammakarzinom, Lungentumoren, Prostatakarzinom, Leukämien, T-Zell-Lymphome, Melanome, Betazell Karzinom, Squamous Carcinoma, aktinische Keratosen, Cervixdysplasien, metastasierende Tumore jeglicher Art).
Auch zur Behandlung und Prophylaxe von Erkrankungen, die durch eine Störung des Gleichgewichts des Immunsystems gekennzeichnet sind, eignen sich die erfindungsgemäßen Substanzen. Hierzu zählen Ekzeme und Erkrankungen des atopischen Formonkreises, sowie Autoimmunerkrankungen wie zum Beispiel Multiple Sklerose, Diabetes mellitus Typ I, Myasthenia gravis, Lupus erythematodes, Sklerodermie, bullöse Hauterkrankungen (Pemphigus, Pemphigoid), weiterhin Abstoßungsreaktionen bei autologen, allogenen oder xenogenen Transplantaten, sowie AIDS. Bei all diesen Erkrankungen können die neuen Verbindung der allgemeinen Formel **I** vorteilhaft mit anderen immunsuppressiv wirksamen Stoffen wie Cyclosporin A, FK 506, Rapamycin und Anti-CD 4-Antikörpern kombiniert werden.
Ebenso sind die Substanzen geeignet zur Therapie von sekundärem Hyperparathyreoidismus und renaler Osteodystrophie infolge der Eigenschaft von Calcitriolen, die Parathormonsynthese zu senken.
Aufgrund der Präsenz des Vitamin D-Konzeptor in den insulinproduzierenden Zellen der Bauchspeicheldrüse eignen sich die Substanzen durch Erhöhung der Insulinsekretion zur Therapie des Diabetes mellitus Typ II.
Weiterhin wurde überraschenderweise gefunden, daß durch topische Applikation der erfindungsgemäßen Verbindungen auf die Haut von Mäusen, Ratten und Meerschweinchen eine vermehrte Hautrötung und Zunahme der Epidermisdicke induziert werden kann. Die Zunahme der Hautrötung wird anhand der Erhöhung des mit einem Farbmeßgerät quantifizierbaren Rotwertes der Hautoberfläche ermittelt. Der Rotwert ist nach dreimaliger Substanzapplikation (Dosis 0,003%) im Abstand von 24 Stunden typischerweise um das 1,5-fache erhöht. Die Zunahme der Epidermisdicke wird im histologischen Präparat quantifiziert. Sie ist typischerweise um das 2,5-fache erhöht. Die Anzahl der proliferierenden Epidermiszellen (Zellen in der S-Phase des Zellcyclus) wird durchflußcytometrisch ermittelt und ist typischerweise um den Faktor 6 erhöht.
Diese Eigenschaften der erfindungsgemäßen 25-Carbonsäure-Derivate in der Vitamin D-Reihe läßt sie zum therapeutischen Einsatz bei atrophischer Haut, wie sie bei natürlicher Hautalterung infolge erhöhter Lichtexposition oder medikamentös induzierter Hautatrophie durch Behandlung mit Glucocorticoiden auftritt, geeignet erscheinen.
Weiterhin ist anzunehmen, daß die Wundheilung durch topische Applikation mit den neuen Verbindungen beschleunigt werden kann.

In Zellpopulationen das Haarfollikels, die entscheidend zum Haarwachstum bzw. der Haarzyklusregulation beitragen, konnten Vitamin $D_3$-Rezeptorproteine nachgewiesen werden (Stumpf, W. E. et al., *Cell Tissue Res.* **238**: 489-496; Milde, P. et al., *J. Invest..* **97**: 230-239, 1991). Außerdem zeigen in vitro-Befunde an isolierten Haarfollikelkeratinozyten einen proliferationsinhibierenden und differenzierungsstimulierenden Einfluß von 1,25-$(OH)_2$-$D_3$.
Aus klinischen Beobachtungen ist bekannt, daß die Vitamin $D_3$-resistente Rachitis häufig mit einer Alopezie einhergeht, die sich im frühen Kindesalter ausprägt. Experimentelle Befunde zeigen, daß die Vitamin $D_3$-Bindungsstelle des VDR bei dieser Erkrankung mutiert, d. h. defekt ist (Kristjansson. K. et al., *J. Clin. Invest.* **92**: 12-16, 1993). Keratinozyten, die aus den Haarfollikeln dieser patienten isoliert wurden, reagieren in vitro nicht auf die Zugabe von 1,25-$(OH)_2$-$D_3$ (Arase, S. et al., *J. Dermatol. Science* **2**: 353-360, 1991).

Aus diesen Befunden läßt sich eine entscheidende Rolle von 1,25 D3 auf die Regulation des Haarwuchstums ableiten.

Daher eignen sich diese Analoga besonders zur Herstellung von Arzneimitteln zur Behandlung von Erkrankungen, die mit einem gestörten Haarwachstum einhergehen (androgenetische Alopezie, Alopezia areata/totalis, chemotherapie-induzierte Alopezie), oder zur Unterstützung des physiologischen Haarwachstums.

Die senile und postmenopausale Osteoporose ist gekennzeichnet durch einen erhöhten Knochenumsatz mit einer insgesamt negativen Bilanz. Aufgrund des Knochenschwundes insbesondere von trabekulärem Knochen kommt es in verstärktem Maße zu Knochenbrüchen. Aufgrund der fördernden Wirkung von Calcitriol sowohl auf die Anzahl als auch die Syntheseleitung von knochenneubildenden Zellen (Osteoblasten) eignen sich die erfindungsgemäßen Substanzen zur Therapie und Prophylaxe der senilen und postmenopausalen Osteoporose (EP 0 634 173 A1), der steroidinduzierten Osteoporose sowie zur beschleunigten Einheilung von Gelenkplastiken. Für die Therapie der verschiedenen Formen der Osteoporose können sie vorteilhaft mit Estradiol oder anderen Abkömmlingen des Östrogens kombiniert werden.

Schließlich konnte gezeigt werden, daß Calcitriol die Synthese eines Wuchsstoffes für Nervenzellen (nerve growth factor) steigert [M.S. Saporito et al. *Brain Res.* **633**, 189-196 (1994)]. Daher eignen sich die erfindungsgemäßen Verbindungen auch zur Behandlung von degenerativen Erkrankungen des peripheren und zentralen Nervensystems, wie der Alzheimerschen Erkrankung und der amyotrophen Lateralsklerose.

Es wurde außerdem gefunden, daß bestimmte Verbindungen der allgemeinen Formel **I** in HL 60-Zellen überraschenderweise die Wirkung von Calcitriol antagonisieren. In der Reihe der 25-Alkyl-Derivate zeigen die Verbindungen mit zunehmender Kettenlänge an der Carbonylgruppe bei gleichbleibend guter Rezeptoraffinität deutlich schwächere differenzierungsstimulierende agonistische Aktivität in HL 60-Zellen (Tab. 2).

Ausgewählte Testverbindungen mit antagonistischer Wirkung:

(5*Z*,7*E*,22*E*)-(1*S*,3*R*,24*R*)-25-(1-Oxopentyl)-26,27-cyclo-9,10-secocholesta-5,7,10(19),22-tetraen-1,3,24-triol **6b**
(5*Z*,7*E*,22*E*)-(1*S*,3*R*,24*R*)-25-(1-Oxohexyl)-26,27-cyclo-9,10-secocholesta-5,7,10(19),22-tetraen-1,3,24-triol **9b**
[5*Z*,7*E*,22*E*,25(*E*)]-(1*S*,3*R*,24*R*)-25-(1-Oxo-2-hexenyl)-26,27-cyclo-9,10-secocholesta-5,7,10(19),22-tetraen-1,3,24-triol **18b**
[5*Z*,7*E*,22*E*,25(*E*)]-(1*S*,3*R*,24*R*)-25-(3-Ethoxy-3-oxo-1-propenyl)-26,27-cyclo-9,10-secocholesta-5,7,10(19),22-tetraen-1,3,24-triol **24b**
[5*Z*,7*E*,22*E*,25(*E*)]-(1*S*,3*R*,24*R*)-25-[3-(1,1-Dimethylethoxy)-3-oxo-1-propenyl]-26,27-cyclo-9,10-secocholesta-5,7,10(19),22-tetraen-1,3,24-triol **26b**
(5*Z*,7*E*,22*E*)-(1*S*,3*R*,24*S*)-20-Methyl-25-(1-oxooctyl)-26,27-cyclo-9,10-secocholesta-5,7,10(19),22-tetraen-1,3,24-triol **43a**
(5*Z*,7*E*,22*E*)-(1*S*,3*R*,24*R*)-20-Methyl-25-(1-oxooctyl)-26,27-cyclo-9,10-secocholesta-5,7,10(19),22-tetraen-1,3,24-triol **43b**
(7*E*,22*E*)-(1*R*,3*R*,24*R*)-25-(1-Oxopentyl)-26,27-cyclo-19-nor-9,10-secocholesta-5,7,22-trien-1,3,24-triol **58b**
(7*E*,22*E*)-(1*R*,3*R*,24*R*)-25-(1-Oxohexyl)-26,27-cyclo-19-nor-9,10-secocholesta-5,7,22-trien-1,3,24-triol **61b**
(7*E*,22*E*)-(1*R*,3*R*,24*S*)-25-(1-Oxoheptyl)-26.27-cyclo-19-nor-9,l0-secocholesta-5,7,22-trien-1,3,24-triol **64a**
(7*E*,22*E*)-(1*R*,3*R*,24*R*)-20-Methyl-25-(1-oxohexyl)-26,27-cyclo-19-nor-9,10-secocholesta-5,7,22-trien-1,3,24-triol **87b**

Vergleichsverbindung:
Calcitriol

Tab. 2

| Verbindung | Kompetitionsfaktor KF für Rezeptorbindung | Dosisrelation für Differenzierungsinduktion in HL 60-Zellen |
|---|---|---|
| **6b** | 2 | 58 |
| **9b** | 3 | >180 |
| **18b** | 2 | 330 |
| **24b** | 4 | >1000 |
| **26b** | 4 | >1000 |
| **43a** | 48 | >1000 |
| **43b** | 33 | >1000 |
| **58b** | 3 | 160 |
| **61b** | 3 | >1000 |
| **64a** | 3,5 | 120 |
| **87b** | 3 | 116 |
| Calcitriol | 1 | 1 |

Die Verbindungen **6b**, **9b**, **18b**, **24b, 26b**, **43a**, **43b**, **58b**, **61b**, **64a** und **87b** antagonisieren die Wirkung von Calcitriol in HL 60-Zellen. Diese Eigenschaft setzt sich mit zunehmender Kettenlänge im Rest Z der allgemeinen Formel **I** fort.

Solche Verbindungen, die die Wirkung von Calcitriol antagonisieren, können bei der Therapie von Hypercalcämien eingesetzt werden, wie zum Beispiel bei Hypervitaminose D oder Intoxikation mit Calcitriol und calcitriolartig wirksamen Substanzen, oder bei erhöhter extrarenaler Calcitriolsynthese bei granulomatösen Erkrankungen (Sarkoidose, Tuberkulose). Ferner sind sie bei paraneoplastischen Hypercalcämien (zum Beispiel bei osteolytischen Metastasen und Tumoren mit erhöhter Synthese von Parathormon-related peptide) sowie bei Hypercalcämie bei Hyperparathyreoidismus anwendbar.

Weiterhin sind Calcitriolantagonisten zur Fertilitätskontrolle einzusetzen. Im Reproduktionstrakt weiblicher und männlicher Tiere wird der Vitamin D-Rezeptor exprimiert. Es ist bekannt, daß die weibliche und männliche Fertilität Vitamin D-defizienter Tiere herabgesetzt ist. Durch kurzfristige Substitution von Calcitriol kann die Reproduktionsleistung erhöht werden. Daher sind Calcitriolantagonisten in der Lage, die weibliche und männliche Fertilität zu beeinflussen.

Da Calcitriol unter bestimmten Bedingungen eine immunsuppressive Wirkung zeigt, sind Calcitriolrezeptorantagonisten auch als Immunstimulantien, z. B. bei Infektabwehrschwäche, einzusetzen.

Von Calcitriol ist bekannt, daß es das Haarwachstum modulieren kann. Calcitriolantagonisten können daher bei unerwünschtem Haarwachstum, z. B. beim Hirsutismus, therapeutische Verwendung finden.

Eine fördernde Rolle von Vitamin D auf die Bildung von arteriosklerotischen Plaques ist seit langem bekannt. In solchen Gefäßläsionen wird ein Calcitriol-reguliertes Protein, das Osteopontin, vermehrt gefunden, dem eine Rolle bei der Gefäßverkalkung zugeschrieben wird [R. Eisenstein et al. *Arch. Path.* **77**, 27-35 (1964), L.A. Fitzpatrick et al. *J. Clin. Invest.* **94,** 1597-1604 (1994)] Deshalb eignen sich Calcitriolantagonisten zur Therapie und Prophylaxe aller Erscheinungsformen der Arteriosklerose.

Schließlich eignen sich Calcitriolantagonisten infolge der Eigenschaft von Calcitriol, unspezifische Immunreaktionen von monocytären Zellen zu steigern, zur Therapie von entzündlichen Erkrankungen insbesondere chronischer Natur, wie rheumatoide Arthritis, Morbus Crohn, Colitis ulcerosa, und granulomatösen Erkrankungen wie Sarkoidose und anderen Fremdkörperreaktionen.

Die vorliegende Erfindung bezieht sich somit auf pharmazeutische Präparate, die mindestens eine Verbindung gemäß der allgemeinen Formel **I** zusammen mit einem pharmazeutisch vertäglichen Träger enthalten.

Die Verbindungen können formuliert werden als Lösungen in pharmazeutisch verträglichen Solventien oder als Emulsionen, Suspensionen oder Dispensionen in geeigneten pharmazeutischen Solventien oder Trägern oder als Pillen, Tabletten oder Kapseln, die in an sich bekannter Weise feste Trägerstoffe enthalten. Für eine topische Anwendung werden die Verbindungen vorteilhafterweise als Cremes oder Salben oder in einer ähnlichen, zur topischen Anwendung geeigneten Arzneimittelform formuliert. Jede derartige Formulierung kann auch andere pharmazeutisch verträgliche und nichttoxische Hilfsstoffe enthalten, wie z. B. Stabilisatoren, Antioxidantien, Bindemittel, Farbstoffe, Emulgatoren oder Geschmackskorrigentien. Die Verbindungen werden vorteilhafterweise durch Injektion oder intravenöse Infusion geeigneter steriler Lösungen oder als orale Dosierung über den Ernährungstrakt oder topisch in der Form von Cremes, Salben, Lotions oder geeigneter transdermaler Pflaster appliziert, wie in der EP-A 0 387 077 beschrieben ist. Die tägliche Dosis liegt bei 0,1 µg/Patient/Tag - 1000 µg (1 mg)/Patient/Tag,
vorzugsweise 1,0 µg/Patient/Tag - 500 µg/Patient/Tag.

Die Herstellung der Vitamin D-Derivate der allgemeinen Formel **I** erfolgt erfindungsgemäß aus einer Verbindung der allgemeinen Formel **II**,

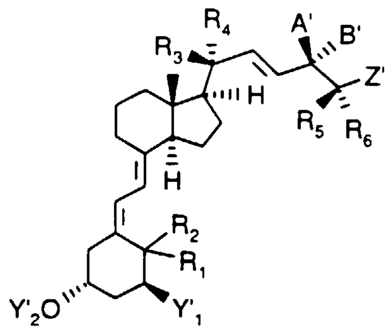

**II**

worin $Y'_1$ ein Wasserstoffatom oder eine geschützte Hydroxygruppe und $Y'_2$ eine Hydroxyschutzgruppe bedeuten.
Bei den Schutzgruppen handelt es sich vorzugsweise um alkyl-, aryl- oder gemischt alkylarylsubstituierte Silylgruppen, z.B. die Trimethylsilyl- (TMS), Triethylsilyl- (TES), tert.-Butyldimethylsilyl- (TBDMS), tert.-Butyldiphenylsilyl- (TBDPS) oder Triisopropylsilylgruppen (TIPS) oder eine andere gängige Hydroxyschutzgruppe (siehe T.W. Greene, P.G.M. Wuts "Protective Groups in Organic Synthesis", 2nd Edition, John Wiley & Sons, 1991).
A' und B' können gemeinsam eine Ketogruppe oder einer der beiden Substituenten eine gegebenenfalls geschützte Hydroxygruppe und der andere ein Wasserstoffatom bedeuten (z.B Silylschutzgruppe obiger Definition, Tetrahydrofuranyl-, Tetrahydropyranyl-, Methoxymethyl-, Methoxyethoxymethyl- oder Trimethylsilylethoxymethylgruppe).
Z' kann analoge Bedeutung wie Z haben oder gegebenenfalls schutzgruppentragende Substituenten (z.B. Hydroxyschutzgruppen gemäß obiger Definition) aufweisen.
Durch gleichzeitige oder sukzessive Abspaltung der Hydroxyschutzgruppen und gegebenenfalls durch partielle. sukzessive oder vollständige Veresterung der freien Hydroxygruppen wird **II** in eine Verbindung der allgemeinen Formel **I** überführt.
Im Falle der Silylschutzgruppen oder der Trimethylsilylethoxymethylgruppe verwendet man zu deren Abspaltung Tetrabutylammoniumfluorid, Fluorwasserstoffsäure oder Fluorwasserstoffsäure/Pyridin; im Falle der übrigen Ethergruppen werden diese unter katalytischer Einwirkung von Säure, beispielsweise p-Toluolsulfonsäure, Pyridinium-p-toluolsulfonat, Essigsäure, Salzsäure, Phosphorsäure oder einem sauren Ionenaustauscher abgespalten.
Die Veresterung der freien Hydroxygruppen kann nach gängigen Verfahren mit den entsprechenden Carbonsäurechloriden, -bromiden oder -anhydriden erfolgen.
Trennungen von Diastereomeren (z.B. bezüglich C-24) können auf der Endstufe oder jeder anderen Vorstufe erfolgen.
Die Herstellung der Ausgangsverbindungen für die allgemeine Formel **II** geht je nach letztendlich gewünschtem Substitutionsmuster in 10- und 20-Position von verschiedenen Startverbindungen aus.

Für die Herstellung von Verbindungen der allgemeinen Formel **II**, worin $R_1$ und $R_2$ gemeinsam eine exocyclische Methylengruppe bedeuten, wird von dem bekannten Aldehyd **III** ausgegangen [M. Calverley Tetrahedron **43**, 4609 (1987), WO 87/00834].

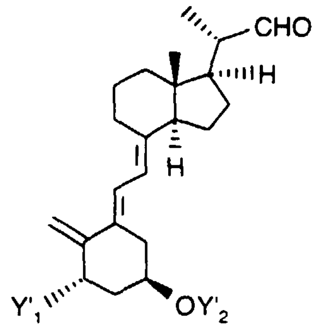

**III**

Für $Y'_1$ und $Y'_2$ gelten die schon erwähnten Definitionen. Andere Schutzgruppen als die in den Literaturstellen erwähnten lassen sich durch analoge Vorgehensweise unter Verwendung entsprechend modifizierter Silylchloride (z. B. tert.-Butyldiphenylsilylchlorid anstelle von tert.-Butyldimethylsilylchlorid) erhalten. Durch Verzicht auf die entsprechenden Stufen zur 1$\alpha$-Hydroxylierung lassen sich Derivate vom Typ $Y'_1$=H erhalten.
Die Verbindungen der allgemeinen Formel **III** werden nun analog bekannter Verfahren in Aldehyde der allgemeinen Formel **IV** überführt (WO 94/07853).

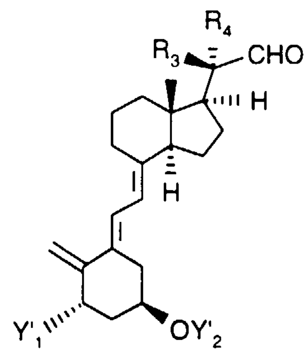

**IV**

Für $R_3$ und $R_4$ gelten die schon eingangs erwähnten Definitionen.
Zum Aufbau der Seitenkette können nun sowohl Verbindungen der allgemeinen Formel **III** als auch Verbindungen der allgemeinen Formel **IV** eingesetzt werden. Exemplarisch wird im folgenden die Umsetzung von Verbindungen der allgemeinen Formel **III** beschrieben. Analog der etablierten Sequenz (WO 94/07853) können so Carbonsäureamide der allgemeinen Formel **V** generiert werden,

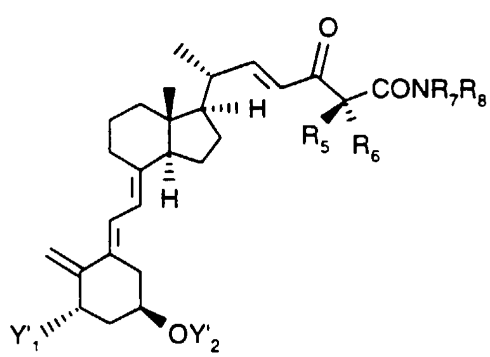

**V**

wobei für $Y'_1$, $Y'_2$, $R_5$ und $R_6$ die bereits gegebenen Definitionen gelten. Vorzugsweise sollen $R_5$ und $R_6$ je eine Me-

thylgruppe oder beide zusammen mit dem Kohlenstoffatom 25 einen Cyclopropylring bedeuten. $R_7$ und $R_8$ bedeuten gerad- oder verzweigtkettige Alkylgruppen mit 1-9 Kohlenstoffatomen. wobei besonders Methyl- und Ethylgruppen bevorzugt sind.
Reduktion der Ketogruppe mit Reduktionsmitteln wie z.B. $NaBH_4$ oder $NaBH_4/CeCl_3$ führt dann zu Alkoholen der allgemeinen Formel **VI**.

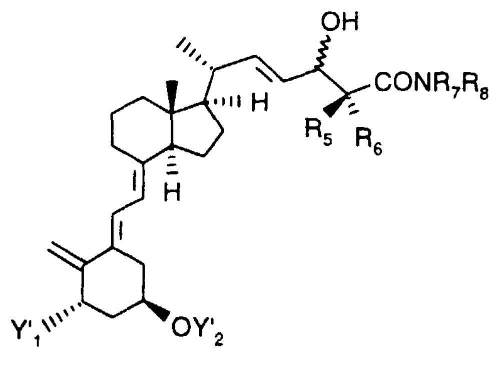

**VI**

Zur Etablierung des natürlichen Vitamin D-Triensystems wird eine photochemische Isomerisierung von Verbindungen der allgemeinen Formel **VI** vorgenommen. Bestrahlung mit ultraviolettem Licht erfolgt in Gegenwart eines sogenannten Triplettsensibilisators. Im Rahmen der vorliegenden Erfindung wird dafür Anthracen verwendet. Durch Spaltung der $\pi$-Bindung der 5,6-Doppelbindung, Rotation des A-Ringes um 180° um die 5,6-Einfachbindung und Reetablierung der 5,6-Doppelbindung wird die Stereoisomerie an der 5,6-Doppelbindung umgekehrt, wobei Verbindungen der allgemeinen Formel **VII** anfallen,

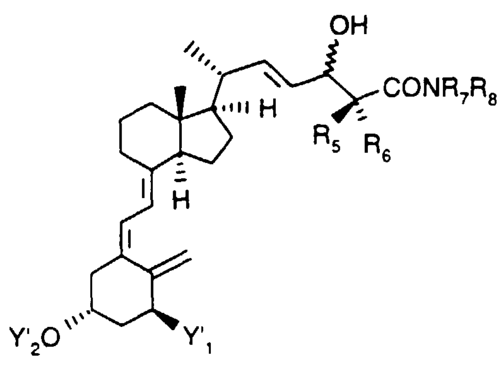

**VII**

wobei $Y'_1$, $Y'_2$, $R_5$, $R_6$, $R_7$ und $R_8$ die genannten Bedeutungen haben. Die diastereomeren Alkohole an C-24 können chromatographisch getrennt werden.
Zum Aufbau des Restes Z' werden Verbindungen der allgemeinen Formel **VII** mit geeigneten Lithiumorganylen der allgemeinen Formel **VIII**

$$LiR_9 \qquad \text{VIII}$$

bei tiefer Temperatur (-100 bis 0°C) umgesetzt. Die Lithiumorganyle können unter Standardbedingungen generiert werden (Halogen-Lithiumtausch bei Halogenalkanen, Metallierungen von aromatischen oder heteroaromatischen Systemen, Metall-Lithiumaustausch, Definitionen für $R_9$ wurden bereits genannt). Dabei entstehen Verbindungen der allgemeinen Formel **II**, wobei für $Y'_1$, $Y'_2$, $R_5$ und $R_6$ die genannten Bedeutungen gelten, $R_1$ und $R_2$ zusammen eine

exocyclische Methylengruppe bedeuten, $R_3$ und $R_4$ je nach Wahl des Aldehydes **III** oder **IV** die daraus abgeleiteten Bedeutungen haben, A' eine Hydroxygruppe und B' ein Wasserstoffatom oder A' ein Wasserstoffatom und B' eine Hydroxygruppe und Z'= C(O)-$R_9$ ist. Die Hydroxylgruppe in 24-Position (A' oder B') kann vor der abschließenden Schutzgruppenabspaltung gegebenfalls mit einem Oxidationsmittel wie z.B. PCC, PDC, $BaMnO_4$, $MnO_2$, Swern-Bedingungen, Dess-Martin-Reagenz in ein 24-Keton der allgemeinen Formel **II** überführt werden, wobei A' und B' zusammen eine Ketogruppe bilden. Die nachfolgende Schutzgruppenabspaltung muß dann aber unter sauren Reaktionsbedingungen erfolgen (z.B. saure Ionentauscher, Essigsäure, p-Toluolsulfonsäure, Pyridinium-p-Toluolsulfonat), da bei Verwendung der üblichen Fluorid-Reagenzien konjugierte Additionen der Nukleophile an das Enon-System zu befürchten sind. Ein temporärer Schutz der 24-Hydroxygruppe mit einer Schutzgruppe wie in $Y'_1$ und $Y'_2$ kann erfolgen, um in einigen Fällen die Ausbeute bei der Addition der lithiumorganischen Verbindung **VIII** zu erhöhen.
Sollen sterisch anspruchsvolle, verzweigte Reste für $R_9$ etabliert werden, so erfolgt die Reaktion des bekannten Esters der allgemeinen Formel **IX** (WO 94/07853) anstelle des Amides **VII** mit der lithiumorganischen Verbindung **VIII**, wobei eine Verbindung der allgemeinen Formel **II** anfällt.

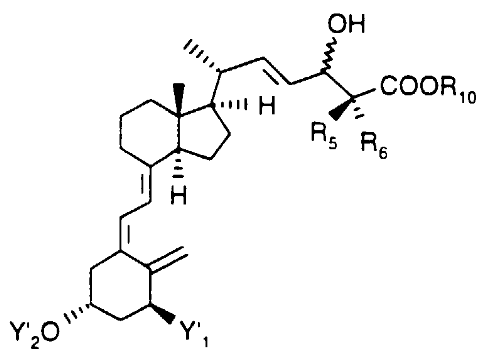

**IX**

Der Rest $R_{10}$ bedeutet eine gerad- oder verzweigtkettige Alkylgruppe mit 1-9-Kohlenstoffatomen. Grundsätzlich können die diastereomeren Alkohole (bzgl. C-24) bei den genannten Sequenzen im Vorfeld getrennt und separat umgesetzt werden.
Für die Synthese weiterer modifizierter Derivate wird die Verbindung der allgemeinen Formel **IX** durch Schutz der 24-Hydroxygruppe in eine Verbindung der allgemeinen Formel **X** überführt,

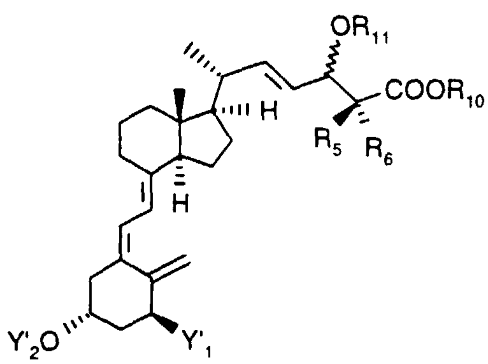

**X**

wobei $R_{11}$ eine säurelabile Schutzgruppe analoger Definition wie in $Y'_1$ oder $Y'_2$ oder die Tetrahydropyranyl-, Tetrahydrofuranyl-, Ethoxyethyl-, Methoxymethyl- oder Methoxyethoxymethylgruppe bedeutet. Durch Reduktion der Estereinheit der allgemeinen Formel **X** mit einem Reduktionsmittel wie z.B. DIBAH, TIBA, $LiAlH_4$, RedAl erhält man Verbindungen der allgemeinen Formel **XI**.

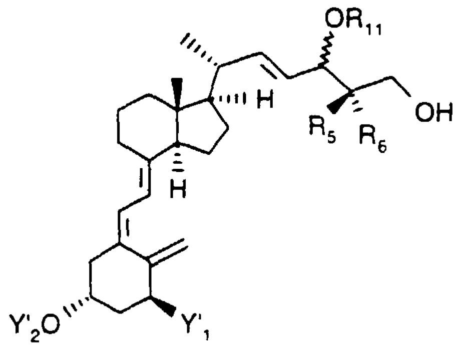

XI

Unter den bekannten Reaktionsbedingungen lassen sich nun Ether, Sulfide und Amine generieren, wobei Verbindungen der allgemeinen Formel **II** anfallen, für die Z'= X-$R_9$ mit X=O, S, NH, N-Alkyl. N-Acyl sein kann.

Zur weiteren Strukturvariation können die Verbindungen der allgemeinen Formel **XI** zu den Aldehyden der allgemeinen Formel **XII** umgesetzt werden.

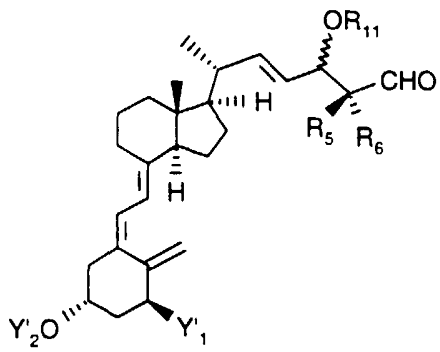

XII

Diese Reaktion kann mit den schon für die Oxidation der Hydroxygruppe in Position 24 angegebenen Reagenzien oder Methoden erfolgen. Neben den schon erwähnten Lithiumorganylen der allgemeinen Formel **VIII**, deren Einsatz hier zu Verbindungen der allgemeinen Formel **II** führt, wobei Z'= CH(OH)-$R_9$ ist, können hier nach literaturbekannten Methoden perfluorierte Alkylreste eingeführt werden [G.K. Surya Prakash J. Org. Chem. **56**, 984 (1991), H. Uno et al. Bull. Chem. Soc. Jpn. **62**, 2636 (1989)]. Durch katalytische Einwirkung von Tetrabutylammoniumfluorid auf die leicht zugänglichen Perfluoralkyltrimethylsilane (Synthese aus den kommerziell erhältlichen Perfluoralkyliodiden) oder durch Jod-Lithiumaustausch der Perfluoralkyliodide mit Methyllithium/Lithiumbromid-Komplex kann ein Angriff an die Carbonylgruppe erfolgen, wobei nach hydrolytischer Aufarbeitung Verbindungen der allgemeinen Formel **XIII** anfallen,

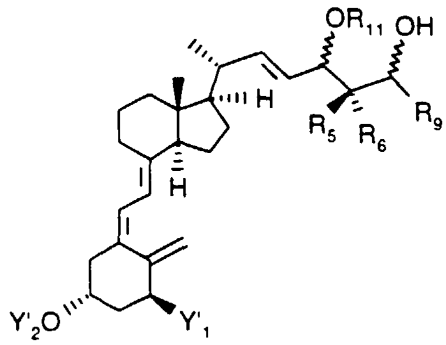

XIII

wobei $R_9$ gerad- oder verzweigtkettige perfluorierte Alkylreste von 1-9 Kohlenstoffatomen bedeuten kann. Die diastereomeren Alkohole werden chromatographisch getrennt. Die Verbindung der allgemeinen Formel **XIII** können einerseits als Spezialfall der allgemeinen Formel **II** angesehen und wie dort beschrieben weiter behandelt werden oder andererseits durch Oxidation mit einem der schon vorher erwähnten Oxidationsmittel (vorzugsweise Swern-Bedingungen oder Dess-Martin-Reagenz) in eine Verbindung der allgemeinen Formel **II**, wobei Z'= C(O)-$R_9$ bedeutet, überführt werden.

Ferner kann der Aldehyd **XII** mit Wittig-, Wittig-Horner- oder Wadsworth-Emmons-Reagenzien vom Typ **XIV**,

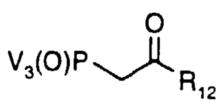

XIV

wobei V=C1-C8-Alkyl oder Alkoxy (gerad oder verzweigtkettig oder cyclisch) vorzugsweise Methyl, Methoxy, Ethyl, Ethoxy, Butyl, Butoxy, Phenyl, Phenoxy bedeuten und die Definition für $R_{12}$ bereits eingangs gegeben wurde, in Gegenwart von Basen (z.B. NaH, KH, LDA, Butyllithium, LiHMDS, NaHMDS, KHMDS) zu Verbindungen der allgemeinen Formel **XV** umgesetzt werden,

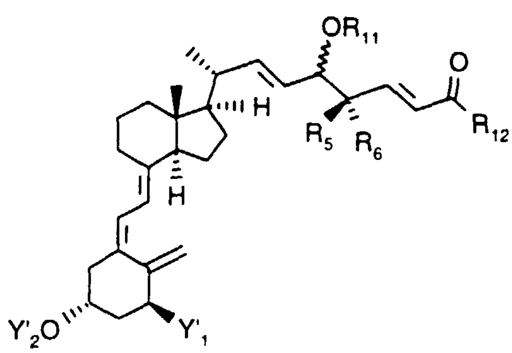

XV

welche als Spezialfall der allgemeinen Formel **II** betrachtet werden kann, für die gilt: Z'=

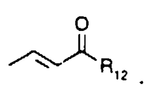

Die Herstellung von Verbindungen der allgemeinen Formel **I,** für den Fall das $R_1$ und $R_2$ Wasserstoffatome bedeuten, erfolgt dadurch, daß eine Verbindung der allgemeinen Formel **II'**,

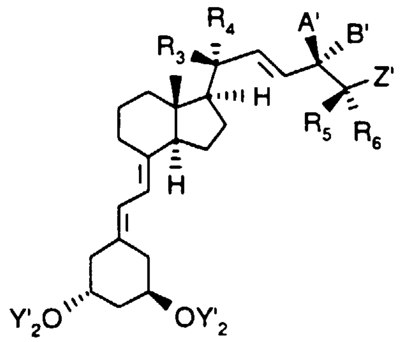

II'

wobei für $Y'_2$, $R_3$, $R_4$, $R_5$, $R_6$, A', B' und Z' die schon genannten Bedeutungen bestehen, analog den für die Umsetzung von **II** beschriebenen Bedingungen behandelt wird.
Die Herstellung von Verbindungen der allgemeinen Formel **II'** erfolgt auf einem konvergenten Syntheseweg, wobei CD- und A-Ring-Fragmente separat aufgebaut werden. Zur Synthese der CD-Fragmente wird der literaturbekannte Aldehyd **XVII** [H.H. Inhoffen et al. Chem. Ber. **91**, 780 (1958), Chem. Ber. **92**, 1772 (1959), W.G. Dauben **30**, 677 (1989)] verwendet,

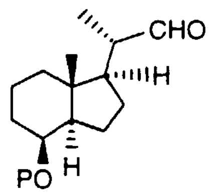

XVII

worin P eine acyl-, alkyl- oder arylsubstituierte Silyl- oder Tetrahydropyranyl-, Tetrahydrofuranyl-, Methoxymethyl-, Ethoxyethylgruppe, eine Acylgruppe (z.B. Acetyl-, Benzoyl-) oder eine andere Alkoholschutzgruppe bedeuten (siehe T.W. Greene, P.G.M. Wuts "Protective Groups in Organic Synthesis", 2nd Edition, John Wiley & Sons, Inc., 1991).
Nach den bekannten Verfahren (WO 94/07853) können die schon für die Normalreihe beschriebenen Modifikationen an C-20 eingeführt werden, wobei eine Verbindung der allgemeinen Formel **XVIII** anfällt.

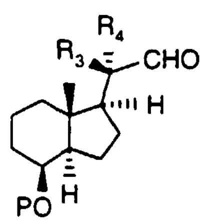

XVIII

Für $R_3$ und $R_4$ gelten die eingangs erwähnten Definitionen.
Zur Vereinfachung wird im Folgenden exemplarisch die Umsetzung der Verbindung der allgemeinen Formel **XVII** beschrieben.
Für den Fall, daß $R_5$ und $R_6$ gemeinsam mit dem tertiären Kohlenstoffatom 25 einen Cyclopropylring bilden, kann wie für die Normalreihe bekannt (WO 94/07853) durch Aldolreaktion mit einem Acetessigesterbaustein der allgemeinen Formel **XIX**,

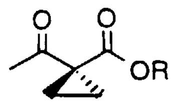

**XIX**

wobei R eine geradkettige Alkylgruppe mit 1-6 Kohlenstoffatomen bedeutet, eine Verbindung der allgemeinen Formel **XX** erhalten werden.

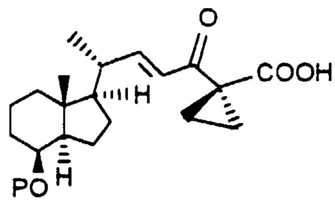

**XX**

Über die Zwischenprodukte **XXI**, **XXII** sowie **XXIII** wird dann die Verbindung der allgemeinen Formel **XXIV** zugänglich.

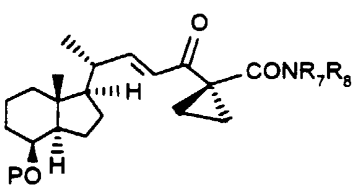

**XXI**

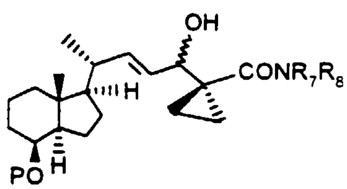

**XXII**

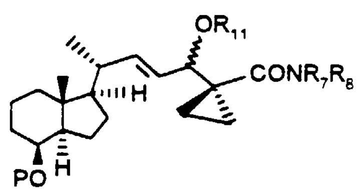

**XXIII**

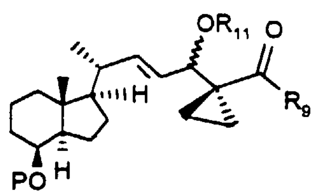

**XXIV**

Die dazu notwendigen chemischen Manipulationen sowie die Bedeutungen von P, $R_7$, $R_8$, $R_9$ und $R_{11}$ sind bereits an anderer Stelle beschrieben worden. Durch Reduktion der Ketogruppe mit einem Reduktionsmittel (z.B. $NaBH_4$, $NaBH_4/CeCl_3$, $LiAlH_4$, DIBAH, TIBA, RedAl) wird eine Verbindung der allgemeinen Formel **XXV** zugänglich, deren Hydroxygruppe mit einer säurestabilen, durch Baseneinwirkung zu entfernenden, Schutzgruppe versehen wird (z.B. $R_{14}$= Acetyl-, Propionyl-, Pivaloyl-, Benzoylgruppe), wobei man eine Verbindung der allgemeinen Formel **XXVI** erhält. Trennungen der diastereomeren Hydroxygruppen erfolgen jeweils auf den geeigneten Zwischenstufen.

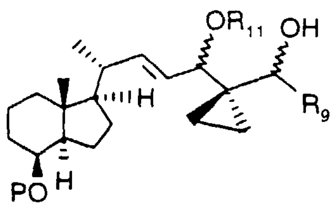

XXV

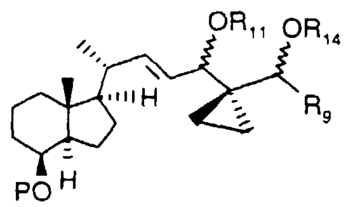

XXVI

Bei Wahl geeigneter Schutzgruppen (z.B. P=$Et_3Si$, $R_{11}$=THP, $R_{14}$=Ac) läßt sich die Gruppe P selektiv abspalten und durch Oxidation der Hydroxygruppe der Verbindung der allgemeinen Formel **XXVII** mit einem Oxidationsmittel (PCC, PDC, $BaMnO_4$, Swern-Bedingungen, Dess-Martin-Reagenz) in ein CD-Fragment der allgemeinen Formel **XXVIII** überführen.

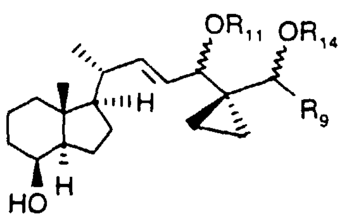

XXVII

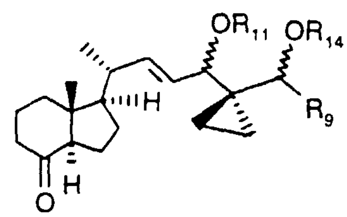

XXVIII

Die Verbindungen der allgemeinen Formel **XXVIII** werden nun durch Reaktion mit dem durch eine Base wie n-Butyllithium oder LDA erzeugten Anion des literaturbekannten Phosphinoxides der allgemeinen Formel **XXIX** [H.F. DeLuca et al. Tetrahedron Lett. 32, 7663, (1991)],

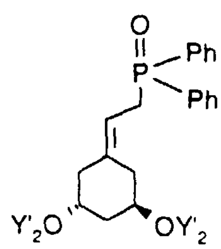

XXIX

worin $Y'_2$ die schon beschriebene Bedeutung hat, in die entsprechenden Verbindungen der allgemeinen Formel **XXX** überführt werden.

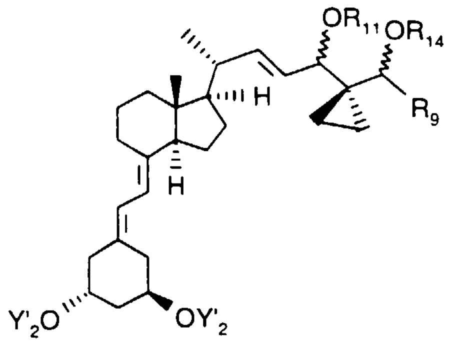

**XXX**

Sukzessive oder gleichzeitig werden jetzt die Schutzgruppen entfernt ($R_{14}$ durch basische Hydrolyse, $R_{11}$ sowie $Y'_2$ durch saure Hydrolyse oder Fluoridreagenzien) und je nach Wunsch eine oder beide der Seitenkettenhydroxygruppen mit den schon häufiger genannten Oxidationsmittel oxidiert, wobei Verbindungen der allgemeinen Formel **I** anfallen, für die gilt: $R_1$ und $R_2$ sind Wasserstoffatome sowie $R_5$ und $R_6$ bilden gemeinsam mit dem tertiären Kohlenstoffatom 25 einen Cyclopropylring. Die weiteren Definitionen wurden bereits genannt.

Alternativ kann die Schutzgruppe P in der allgemeinen Formel **XXIII** selektiv gespalten werden, wenn gilt: P=Silylschutzgruppe, $R_{11}$ = Tetrahydropyranyl- oder Tetrahydrofuranylschutzgruppe. Dies kann z.B. mit Tetrabutylammoniumfluorid erfolgen, wobei Verbindungen der allgemeinen Formel **XXXI** anfällt.

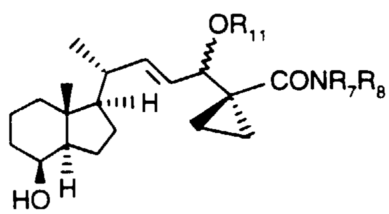

**XXXI**

Mit einem Oxidationsmittel (PCC, PDC, $BaMnO_4$, Swern-Bedingungen, Dess-Martin-Reagenz) kann nun die freie Hydroxygruppe oxidiert werden, wobei Verbindungen der allgemeinen Formel **XXXII** entstehen,

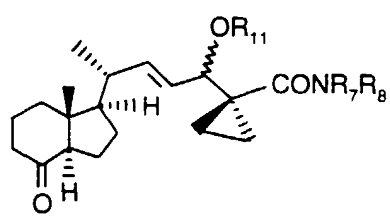

**XXXII**

die mit dem durch eine Base (n-Butyllithium, Lithiumdiisopropylamid) erzeugten Anion des Phosphinoxides **XXIX** in Verbindungen der allgemeinen Formel **XXXIII** überführt werden.

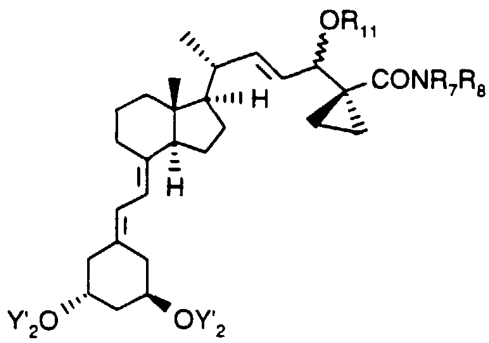

**XXXIII**

Analog der Verbindungen in der Normalreihe (z.B. **VII**) erfolgt nun der Aufbau des Restes Z', wobei Verbindungen der allgemeinen Formel **XXXIV** anfallen.

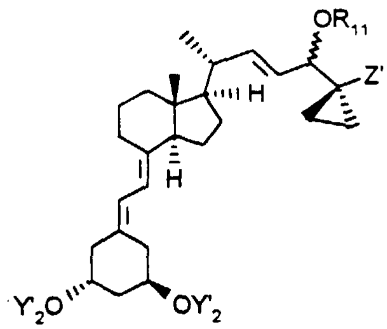

**XXXIV**

Diese können als Spezialfall der allgemeinen Formel **II'** angesehen werden, wobei alle Variablen bereits zuvor beschrieben worden sind. Die Weiterbehandlung der Verbindungen der allgemeinen Formel **II'** ist ebenso vorstehend ausgeführt worden.
Für den Fall, daß $R_5$ und $R_6$ nicht zusammen mit dem tertiären Kohlenstoffatom 25 einen Cyclopropylring bilden, sondern die übrigen eingangs genannten Definitionen gelten sollen, erfolgt der Aufbau der Seitenkette auf einem etwas modifizierten Syntheseweg. Der bekannte CD-Teil der allgemeinen Formel **XXXV** (WO 94/07853) kann analog der Normalreihe in Derivate der allgemeinen Formeln **XXXVI** und **XXXVII** überführt werden,

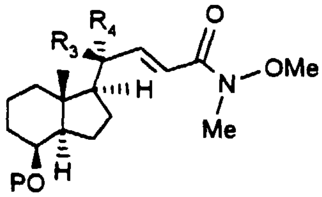

**XXXV**

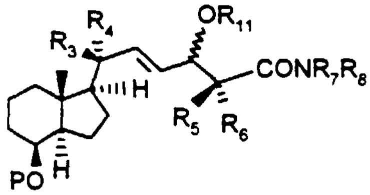

**XXXVI**

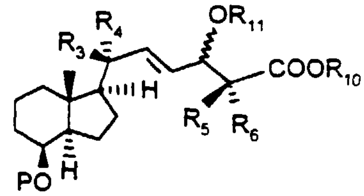

**XXXVII**

wobei sämtliche Variablen die schon genannten Definitionen haben. Die Diastereomeren können auf geeigneten Zwischenstufen getrennt werden.
Durch direkte Umsetzung von Lithiumorganylen der allgemeinen Formel **VIII** ($LiR_9$) mit Verbindungen der allgemeinen Formeln **XXXVI** und **XXXVII** können nun Verbindungen der allgemeinen Formel **XXXVIII** generiert und wie zuvor gezeigt in eine Verbindung der allgemeinen Formel **II'** überführt werden.
Die diastereomeren Alkohole (bzgl. C-24) können im Vorfeld getrennt und separat umgesetzt werden.

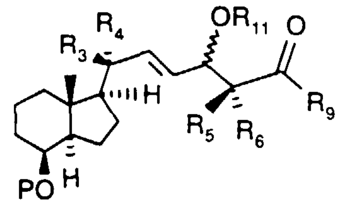

**XXXVIII**

Grundsätzlich kann die Einführung entsprechend substituierter Seitenketten oder deren Vorläufer auch an Aldehyden der allgemeinen Formeln **III** oder **IV** bzw. deren 5Z-Isomeren unter Verwendung etablierter Synthesemethoden erfolgen.

Die nachstehenden Beispiele dienen der näheren Erläuterung der Erfindung.

**Beispiel 1**

(5*Z*,7*E*,22*E*)-(1*S*,3*R*,24*R*)-25-(1-Oxopentyl)-26,27-cyclo-9,10-secocholesta-5,7,10(19),22-tetraen-1,3,24-triol (**6b**)

a) Eine Menge von 5,0 g (5*E*,7*E*,22*E*)-(1*S*,3*R*)-1,3-Bis[[dimethyl(1,1-dimethylethyl)silyl]oxy]-24-oxo-26,27-cyclo-9,10-secocholesta-5,7,10(19),22-tetraen-25-carbonsäure **1** (WO 94/07853) und 1,01 g N-Hydroxysuccinimid werden in 21 ml Methylenchlorid gelöst und bei 0°C mit 1,87 g N,N'-Dicyclohexylcarbodiimid versetzt. Nach 1,5 h werden 2,04 ml einer 40%igen wässrigen Dimethylamin-Lösung zugesetzt und weitere 30 min bei 0°C gerührt. Nach 3 h bei Raumtemperatur wird die Reaktionsmischung an Silicagel mit Essigester/Hexan (1:4) chromatographiert. Es werden 2,08 g (5*E*,7*E*,22*E*)-(1*S*,3*R*)-1,3-Bis-[[dimethyl(1,1-dimethyl-ethyl)silyl]oxy]-N,N-dimethyl-24-oxo-26,27-cyclo-9,10-secocholesta-5,7,10(19),22-tetraen-25-carbonsäureamid **2** als farblose Masse erhalten.

b) 1,39 g des Amides **2** werden in 3,3 ml THF und 7,7 ml Methanol gelöst und mit 7,7 ml einer 0,4 molaren methanolischen Certrichlorid (Hydrat)-Lösung versetzt. Bei 0°C werden nun 210 mg Natriumborhydrid portionsweise hinzugegeben. Man rührt 45 min bei 0°C nach und versetzt dann mit einem Eis/Wasser-Gemisch. Anschließend wird mit Essigester extrahiert, über Natriumsulfat getrocknet und eingeengt. Der ölige Rückstand (1,32 g) ist ein Diastereomerengemisch aus (5*E*,7*E*,22*E*)-(1*S*,3*R*,24*S*)-1,3-Bis[[dimethyl(1,1-dimethylethyl)-silyl]oxy]-N,N-dimethyl-24-hydroxy-26,27-cyclo-9,10-secocholesta-5,7,10(19),22-tetraen-25-carbonsäureamid **3a** und dem 24*R*-Diastereomeren **3b**.

c) 2,48 g des Epimerengemisches **3a** und **3b** werden in 348 ml Toluol gelöst und nach Zugabe von 383 mg Anthracen und 7 Tropfen Triethylamin 19 min unter Stickstoff mit einer Quecksilberhochdrucklampe (Heraeus TQ 150) durch Pyrex-Glas bestrahlt. Die eingeengte Reaktionsmischung wird mit Hexan versetzt, filtriert und erneut eingeengt. Der Rückstand von 2,82 g ist ein Gemisch aus (5*Z*,7*E*,22*E*)-(1*S*,3*R*,24*S*)-1,3-Bis[[dimethyl(1,1-dimethylethyl)silyl]oxy]-N,N-dimethyl-24-hydroxy-26,27-cyclo-9,10-secocholesta-5,7,10(19),22-tetraen-25-carbonsäureamid **4a** und dem korrespondierenden 24*R*-Diastereomeren **4b**.

d) Das Diastereomerengemisch aus **4a** und **4b** (2,82 g) wird in 28 ml THF gelöst und bei 0°C mit 6,53 ml n-Butyllithium-Lösung (1,6 M in Hexan) tropfenweise versetzt. Nach 75 min wird in die Reaktionslösung gesättigte Ammoniumchlorid-Lösung gegeben, mit Essigester extrahiert, über Natriumsulfat getrocknet und eingeengt. Durch Chromatographie des Rückstandes an Silicagel mit Essigester/Hexan erhält man in der Elutionsreihenfolge 0,84 g (5*Z*,7*E*,22*E*)-(1*S*,3*R*,24*S*)-1,3-Bis[[dimethyl(1,1-dimethylethyl)silyl]oxy]-25-(1-oxopentyl)-26,27-cyclo-9,10-secocholesta-5,7,10(19),22-tetraen-24-ol **5a** und 0,64 g (5*Z*,7*E*,22*E*)-(1*S*,3*R*,24*R*)-1,3-Bis[[dimethyl(1,1-dimethylethyl)silyl]oxy]-25-(1-oxopentyl)-26,27-cyclo-9,10-secocholesta-5,7,10(19),22-tetraen-24-ol **5b** als Öle.

e) 0,62 g des Epimeren **5b** werden in 24,9 ml THF mit 1,32 g Tetrabutylammoniumfluorid (Trihydrat) über Nacht bei Raumtemperatur stehengelassen. Die Reaktionsmischung wird danach auf ein Gemisch aus Eis/Natriumhydrogencarbonat-/Natriumchlorid-Lösung gegossen. Nach Extraktion mit Essigester wird die organische Phase über Natriumsulfat getrocknet und eingeengt. Chromatographie des Rückstandes an Kieselgel mit Essigester/Hexan ergeben 125 mg der Titelverbindung **6b** als Schaum.

$^1$H-NMR (300 MHz, $CDCl_3$): δ= 0,57 ppm (s, 3H); 0,91 (t, 3H); 1,00 (m, 2H); 1,05 (d, 3H); 1,22 (m, 2H); 2,15 (t, 2H); 3,29 (brd, 1H); 4,08 (m, 1H); 4,22 (m, 1H); 4,42 (m, 1H); 5,00 (brs, 1H); 5,32 (brs, 1H); 5,35 (dd, 1H), 5,49 (dd, 1H); 6,01 (d, 1H); 6,38 (d, 1H)

**Beispiel 2**

(5*Z*,7*E*,22*E*)-(1*S*,3*R*,24*S*)-25-(1-Oxopentyl)-26,27-cyclo-9,10-secocholesta-5,7,10(19),22-tetraen-1,3,24-triol (**6a**)

Analog zur Durchführung nach 1e) wird das Epimer **5a** umgesetzt, wobei man die Titelverbindung **6a** als kristallisierendes Öl erhält.

$^1$H-NMR (300 MHz, $CDCl_3$): δ= 0,57 ppm (s, 3H); 0,90 (t, 3H); 1,00 (m, 2H); 1,05 (d, 3H); 1,22 (m, 2H); 2,16 (t, 2H); 3,25 (brs, 1H); 4,12 (m, 1H); 4,23 (m, 1H); 4,43 (m, 1H); 5,00 (brs, 1H); 5,32 (brs, 1H); 5,36 (dd, 1H); 5,55 (dd, 1H); 6,01 (d, 1H); 6,38 (d, 1H)

**Beispiel 3**

(5*Z*,7*E*,22*E*)-(1*S*,3*R*,24*R*)-25-Acetyl-26,27-cyclo-9,10-secocholesta-5,7,10(19),22-tetraen-1,3,24-triol (**7b**)

Ausgehend vom Epimerengemisch **4a** und **4b** wird analog Beispiel 1d)-e) mit Methyllithium die Titelverbindung **7b** kristallin erhalten (Schmelzpunkt: 138-140°C).

$^1$H-NMR (300 MHz, $CDCl_3$): δ= 0,57 ppm (s, 3H); 1,00 (m, 2H); 1,05 (d, 3H); 1,22 (m, 2H); 1,96 (s, 3H); 3,16 (brd, 1H); 4,12 (m, 1H); 4,23 (m, 1H); 4,43 (m, 1H); 5,00 (brs, 1H); 5,32 (brs, 1H); 5,36 (dd, 1H); 5,50 (dd, 1H); 6,01 (d, 1H); 6,38 (d, 1H)

**Beispiel 4**

(5*Z*,7*E*,22*E*)-(1*S*,3*R*,24*R*)-25-(1-Oxobutyl)-26,27-cyclo-9,10-secocholesta-5,7,10(19),22-tetraen-1,3,24-triol (**8b**)

Ausgehend vom Epimerengemisch **4a** und **4b** wird analog Beispiel 1d)-e) mit Propyllithium (aus n-Propylbromid und Lithium in Ether) die Titelverbindung **8b** als Schaum erhalten.

$^1$H-NMR (300 MHz, $CDCl_3$): δ= 0,57 ppm (s, 3H); 0,90 (t, 3H); 1,00 (m, 2H); 1,05 (d, 3H); 1,20 (m, 2H); 2,13 (t, 2H); 3,25 (brs, 1H); 4,10 (m, 1H); 4,23 (m, 1H); 4,43 (m, 1H); 5,00 (brs, 1H); 5,32 (brs, 1H); 5,38 (dd, 1H); 5,50 (dd, 1H); 6,01 (d, 1H); 6,38 (d, 1H)

**Beispiel 5**

(5*Z*,7*E*,22*E*)-(1*S*,3*R*,24*R*)-25-(1-Oxohexyl)-26,27-cyclo-9,10-secocholesta-5,7,10(19),22-tetraen-1,3,24-triol (**9b**)

Ausgehend vom Epimerengemisch **4a** und **4b** wird analog Beispiel 1d)-e) mit Pentyllithium (aus n-Pentylbromid und Lithium in Ether) die Titelverbindung **9b** als Schaum erhalten.

$^1$H-NMR (300 MHz, $CDCl_3$): δ= 0,57 ppm (s, 3H); 0,90 (t, 3H); 1,00 (m, 2H); 1,05 (d, 3H); 1,22 (m, 2H); 2,13 (t, 2H); 3,30 (brs, 1H); 4,08 (m, 1H); 4,23 (m, 1H); 4,43 (m, 1H); 5,00 (brs, 1H); 5,32 (brs, 1H); 5,37 (dd, 1H); 5,50 (dd, 1H); 6,01 (d, 1H); 6,38 (d, 1H)

**Beispiel 6 (10b)**

(5*Z*,7*E*,22*E*)-(1*S*,3*R*,24*R*)-25-Benzoyl-26,27-cyclo-9,10-secocholesta-5,7,10(19),22-tetraen-1,3,24-triol

Ausgehend vom Epimerengemisch **4a** und **4b** wird analog Beispiel 1d)-e) mit Phenyllithium die Titelverbindung **10b** als Schaum erhalten.

$^1$H-NMR (300 MHz, $CDCl_3$): δ= 0,57 ppm (s, 3H); 1,01 (d, 3H); 1,08 (m, 2H); 1,22 (m, 2H); 2,20 (brd, 1H); 4,23 (m, 1H); 4,45 (m, 2H); 5,00 (brs, 1H); 5,31 (dd, 1H); 5,32 (dd, 1H); 5,48 (dd, 1H); 6,01 (d, 1H); 6,38 (d, 1H); 7,45 (m, 3H); 7,75 (d, 2H)

**Beispiel 7**

(5*Z*,7*E*,22*E*)-(1*S*,3*R*,24*R*)-25-(2-Furanylcarbonyl)-26,27-cyclo-9,10-secocholesta-5,7,10(19),22-tetraen-1,3,24-triol (**11b**)

Ausgehend vom Epimerengemisch **4a** und **4b** wird analog Beispiel 1d)-e) mit 2-Furyllithium (Darstellung aus Furan mit n-Butyllithium in THF) bei inverser Zugabe (-78°C, dann 0°C, 1 h) die Titelverbindung **11b** als Feststoff erhalten.

$^1$H-NMR (300 MHz, $CDCl_3$): δ= 0,57 ppm (s, 3H); 1,02 (d, 3H); 1,08 (m, 2H); 1,18 (m, 2H); 3,12 (brd, 1H); 4,22 (m, 2H); 4,42 (m, 1H); 5,00 (brs, 1H); 5,32 (dd, 1H); 5,43 (dd, 1H); 5,53 (dd, 1H); 6,01 (d, 1H); 6,38 (d, 1H); 6,50 (m, 1H); 7,20 (d, 1H); 7,51 (brs, 1H)

**Beispiel 8**

(5*Z*,7*E*,22*E*)-(1*S*,3*R*,24*R*)-25-(Cyclopropylcarbonyl)-26,27-cyclo-9,10-secocholesta-5,7,10(19),22-tetraen-1,3,24-triol (**12b**)

Ausgehend vom Epimerengemisch **4a** und **4b** wird analog Beispiel 1d)-e) mit Cyclopropyllithium (aus Cyclopropylbromid und Lithium in Ether) die Titelverbindung als Schaum erhalten.

$^1$H-NMR (300 MHz, $CDCl_3$): δ= 0,57 ppm (s, 3H); 0,83 (m, 2H); 1,05 (m, 10H); 3,48 (brd, 1H); 4,13 (m, 1H); 4,23 (m, 1H); 4,45 (m, 1H); 5,00 (brs, 1H); 5,32 (dd, 1H); 5,40 (dd, 1H); 5,50 (dd, 1H); 6,01 (d, 1H); 6,38 (d, 1H)

**Beispiel 9**

(5*Z*,7*E*,22*E*)-(1*S*,3*R*,24*R*)-25-(2,2-Dimethyl-1-oxopropyl)-26,27-cyclo-9,10-secocholesta-5,7,10(19),22-tetraen-1,3,24-triol (**15b**)

a) 580 mg (5*Z*,7*E*,22*E*)-(1*S*,3*R*,24*R*)-1,3-Bis[[dimethyl(1,1-dimethylethyl)silyl]oxy]-24-hydroxy-26,27-cyclo-9,10-secocholesta-5,7,10(19),22-tetraen-25-carbonsäureethylester **13b** (WO 94/07853) in 6,4 ml Diethylether werden bei -78°C mit 2,04 ml tert.-Butyllithium (1,7 M in Pentan) tropfenweise versetzt. Nach 1 h bei -78°C wird Ammoniumchlorid-Lösung zugegeben, mit Essigester extrahiert, die organische Phase mit Natriumchlorid-Lösung gewaschen, über Natriumsulfat getrocknet und eingeengt. Chromatographie des öligen Rückstandes an Silicagel mit Essigester/Hexan ergeben 220 mg (5*Z*,7*E*,22*E*)-(1*S*,3*R*,24*R*)-1,3-Bis[[dimethyl(1,1-dimethylethyl)silyl]oxy]-25-(2,2-dimethyl-1-oxopropyl)-26,27-cyclo-9,10-secocholesta-5,7,10(19),22-tetraen-24-ol **14b** als Öl.

b) 220 mg **14b** werden in 8,8 ml THF gelöst und mit 467 mg Tetrabutylammoniumfluorid (Trihydrat) über Nacht bei Raumtemperatur stehengelassen. Aufarbeitung und Isolierung erfolgt analog Beispiel 1e), wobei die Titelverbindung **15b** als Feststoff anfällt.

$^1$H-NMR (300 MHz, $CDCl_3$): δ= 0,57 ppm (s, 3H); 0,80-1,00 (m, 4H); 1,05 (d, 3H); 1,20 (s, 9H); 3,00 (brs, 1H); 4,09 (m, 1H); 4,23 (m, 1H); 4,43 (m, 1H); 5,00 (brs, 1H); 5,29 (dd, 1H); 5,32 (brs, 1H); 5,52 (dd, 1H); 6,01 (d, 1H); 6,38 (d, 1H)

**Beispiel 10**

(5*Z*,7*E*,22*E*)-(1*S*,3*R*,24*R*)-25-(1-Oxoheptyl)-26,27-cyclo-9,10-secocholesta-5,7,10(19),22-tetraen-1,3,24-triol (**16b**)

Ausgehend vom Epimerengemisch **4a** und **4b** wird analog Beispiel 1d)-e) mit Hexyllithium (aus 1-Hexylbromid und Lithium in Ether) die Titelverbindung **16b** als farbloser Schaum erhalten.

$^1$H-NMR (300 MHz, $CDCl_3$): δ= 0,57 ppm (s, 3H); 0,90 (m, 5H); 1,05 (d, 3H); 2,13 (t, 2H); 3,30 (brd, 1H); 4,08 (m, 1H); 4,23 (m, 1H); 4,43 (m, 1H); 5,00 (brs, 1H); 5,32 (brs, 1H); 5,37 (dd, 1H); 5,50 (dd, 1H); 6,01 (d, 1H); 6,38 (d, 1H)

**Beispiel 11**

(5*Z*,7*E*,22*E*)-(1*S*,3*R*,24*R*)-25-(2-Pyridinylcarbonyl)-26,27-cyclo-9,10-secocholesta-5,7,10(19),22-tetraen-1,3,24-triol (**17b**)

2,03 ml 2-Brompyridin in 38 ml Diethylether werden bei -78°C mit 13,3 ml n-Butyllithium (1.6 M in Hexan) tropfenweise versetzt. Nach 30 min werden 1,98 g (5*Z*,7*E*,22*E*)-(1*S*,3*R*,24*R*)-1,3-Bis-[[dimethyl(1,1-dimethylethyl)silyl]oxy]-24-hydroxy-26,27-cyclo-9,10-secocholesta-5,7,10(19),22-tetraen-25-carbonsäureethylester **13b** (WO 94/07853) in 19 ml Diethylether zugetropft. Nach 1,5 h bei -78°C wird die Reaktionslösung mit ges. Ammoniumchlorid-Lösung versetzt, danach mit Essigester extrahiert, die organische Phase über Natriumsulfat getrocknet und eingeengt. Durch Chromatographie an Silicagel mit Essigester/Hexan werden 1,52 g eines farblosen Öls erhalten, die in 60,2 ml THF gelöst und mit 3,19 g Tetrabutylammoniumfluorid (Trihydrat) über Nacht bei Raumtemperatur stehengelassen werden. Aufarbeitung und Isolierung erfolgen analog Beispiel 1e). Nach Umkristallisation aus Isopropanol/Wasser erhält man die Titelverbindung vom Schmelzpunkt 120-121°C.

**Beispiel 12**

[5*Z*,7*E*,22*E*,25(*E*)]-(1*S*,3*R*,24*R*)-25-(1-Oxo-2-hexenyl)-26,27-cyclo-9,10-secocholesta-5,7,10(19),22-tetraen-1,3,24-triol (**18b**)

11,5 ml n-Butyllithium (1.6 M in Hexan) werden bei 0°C zu 3,61 g (*E*)-1-Iod-1-penten [aus 1-Pentin, DIBAH und Iod analog J.K. Stille et al. *J. Am. Chem. Soc.* **109** 2138 (1987), T. Yokoo *Synlett* 645 (1994)] in 90 ml Hexan getropft. Nach 15 min werden 180 mg (5*Z*,7*E*,22*E*)-(1*S*,3*R*,24*R*)-N,N-Dimethyl-1,3,24-trihydroxy-26,27-cyclo-9,10-secocholesta-5,7,10(19),22-tetraen-25-carbonsäureamid [erhalten aus dem Epimerengemisch **4a** und **4b** (Beispiel 1c) durch Chromatographie an Silicagel und Behandlung mit Tetrabutylammoniumfluorid (Trihydrat)] in 18 ml THF zugetropft. Die Reaktionsmischung wird nach 3 h bei 0°C in eiskalte Ammoniumchlorid-Lösung eingerührt. Nach Extraktion mit Essigester, Trocknen der organischen Phase über Natriumsulfat und Chromatographie an Silicagel mit Essigester/Hexan wird die Titelverbindung als farbloser Schaum erhalten.

$^1$H-NMR (300 MHz, $CDCl_3$): δ= 0,57 ppm (s, 3H); 0,90 (t, 3H); 1,05 (d, 5H); 3,45 (brs, 1H); 4,11 (m, 1H); 4,23 (m, 1H); 4,43 (m, 1H); 5,00 (brs, 1H); 5,35 (brs, 1H); 5,40 (dd, 1H); 5,56 (dd, 1H); 5,91 (d, 1H); 6,01 (d, 1H); 6,38 (d, 1H); 6,98 (dt, 1H)

**Beispiel 13**

(5*Z*,7*E*,22*E*)-(1*S*,3*R*,24*R*)-25-(1-Oxo-2-hexinyl)-26,27-cyclo-9,10-secocholesta-5,7,10(19),22-tetraen-1,3,24-triol (**19b**)

5,75 ml n-Butyllithium (1.6 M in Hexan) werden bei -5°C zu 0,9 ml 1-Pentin in 45 ml Hexan getropft. Nach 1 h bei -5°C werden 90 mg (5*Z*,7*E*,22*E*)-(1*S*,3*R*,24*R*)-N,N-Dimethyl-1,3,24-trihydroxy-26,27-cyclo-9,10-secocholesta-5,7,10(19),22-tetraen-25-carbonsäureamid in 9 ml THF zugetropft. Nach 3 h bei 0°C wird die Reaktionsmischung in gesättigte Ammoniumchlorid-Lösung eingerührt. Nach Extraktion mit Essigester, Trocknung der organischen Phase über Natriumsulfat und Chromatographie an Silicagel mit Essigester/Hexan wird die Titelverbindung als farbloser Feststoff erhalten.

$^1$H-NMR (300 MHz, $CDCl_3$): δ= 0,57 ppm (s, 3H); 1,00 (m, 6H); 1,10 (m, 2H); 1,45 (m, 2H); 2,32 (t, 4H); 3,12 (brd, 1H); 4,23 (m, 2H); 4,43 (m, 1H); 5,00 (brs, 1H); 5,32 (brs, 1H); 5,37 (dd, 1H); 5,51 (dd, 1H); 6,01 (d, 1H); 6,38 (d, 1H);

**Beispiel 14**

[5*Z*,7*E*,22*E*,25(*E*)]-(1*S*,3*R*,24*R*)-25-(3-Ethoxy-3-oxo-1-propenyl)-26,27-cyclo-9,10-secocholesta-5,7,10(19),22-tetraen-1,3,24-triol (**24b**)

a) 2,02 g (5*Z*,7*E*,22*E*)-(1*S*,3*R*,24*R*)-1,3-Bis[[dimethyl(1,1-dimethylethyl)silyl]oxy]-24-hydroxy-26,27-cyclo-9,10-secocholesta-5,7,10(19),22-tetraen-25-carbonsäureethylester **13b** (WO 94/07853) werden in 20 ml DMF gelöst und mit 761 mg Imidazol sowie 843 mg t-Butyldimethylsilylchlorid versetzt. Man rührt über Nacht bei Raumtemperatur und arbeitet wässrig auf (Zugabe von Natriumchlorid-Lösung, Essigester-Extraktion, Waschen der organischen Phase mit Natriumchlorid-Lösung, Trocknen über Natriumsulfat, Einengen). Durch Chromatographie an Silicagel mit Essigester/Hexan werden 2,12 g (5*Z*,7*E*,22*E*)-(1*S*,3*R*,24*R*)-1,3,24-Tris[[dimethyl(1,1-dimethylethyl)sily]]oxy]-26,27-cyclo-9,10-secocholesta-5,7,10(19),22-tetraen-25-carbonsäureethylester **20b** als farbloser Schaum erhalten.

$^1$H-NMR (300 MHz, $CDCl_3$): δ= 0,05 ppm (s, 18H); 0,52 (s, 3H); 0,87 (s, 27H); 1,01 (d, 3H); 1,22 (t, 3H); 4,08 (q, 2H); 4,18 (m, 1H); 4,37 (m, 1H); 4,68 (d, 1H); 4,85 (brs, 1H); 5,08 (brs, 1H); 5,22 (dd, 1H); 5,43 (dd, 1H); 6,00 (d, 1H); 6,22 (d, 1H)

b) 2,10 g des Trisilylethers **20b** werden in 15 ml THF gelöst und bei 0°C werden 12 ml DIBAH-Lösung (1 M in Toluol) zugetropft. Man rührt 1 h bei 0°C nach und fügt dann 4 ml Wasser zu. Der Niederschlag wird durch Filtration entfernt, mit Essigester nachgewaschen, die organische Phase mit Wasser gewaschen, über Natriumsulfat getrocknet und eingeengt. Chromatographie an Silicagel mit Essigester/Hexan liefert 1,53 g (5*Z*,7*E*,22*E*)-(1*S*,3*R*,24*R*)-1,3,24-Tris[[dimethyl(1,1-dimethylethyl)silyl]oxy]-26,27-cyclo-9,10-secocholesta-5,7,10(19),22-tetraen-25-methanol **21b** als farblosen Schaum.

$^1$H-NMR (300 MHz, $CDCl_3$): δ= 0,06 ppm (s, 18H); 0,54 (s, 3H); 0,90 (s, 27H); 1,05 (d, 3H); 3,05 (m, 2H); 3,61 (d, 1H); 4,00 (d, 1H); 4,19 (m, 1H); 4,38 (m, 1H); 4,86 (brs, 1H); 5,08 (brs, 1H); 5,47 (m, 2H); 6,01 (d, 1H); 6,22 (d, 1H)

c) 1,5 g des Alkohols **21b** werden in 50 ml Methylenchlorid gelöst und bei Raumtemperatur portionsweise mit insgesamt 1,2 g Pyridiniumchlorochromat versetzt. Man rührt 3 h bei Raumtemperatur nach, verdünnt mit Ether, filtriert, engt ein und chromatographiert den Rückstand an Silicagel mit Essigester/Hexan, wobei man 570 mg (5*Z*,7*E*,22*E*)-(1*S*,3*R*,24*R*)-1,3,24-Tris[[dimethyl(1,1-dimethylethyl)silyl]oxy]-26,27-cyclo-9,10-secocholesta-5,7,10(19),22-tetraen-25-carbaldehyd **22b** als farblosen Schaum erhält.

$^1$H-NMR (300 MHz, $CDCl_3$): δ= 0,05 ppm (s, 18H); 0,55 (s, 3H); 0,90 (s, 27H); 1,05 (d, 3H); 4,19 (m, 1H); 4,38 (m, 1H); 4,45 (d, 1H); 4,87 (brs, 1H); 5,08 (brs, 1H); 5,32 (dd, 1H); 5,53 (dd, 1H); 6,01 (d, 1H); 6,23 (d, 1H); 9,29 (s, 1H)

d) Man legt 34 mg Natriumhydrid (65%) in 5 ml THF vor und gibt 216 mg Diethylphosphonoessigsäureethylester hinzu. Danach werden 100 mg des Aldehydes **22b** in 5 ml THF zugetropft und 1 h auf 50°C erhitzt. Nach dem Abkühlen wird analog 14a) wässrig aufgearbeitet und an Silicagel mit Essigester/Hexan chromatographiert, wobei man 100 mg [5*Z*,7*E*,22*E*,25(*E*)]-(1*S*,3*R*,24*R*)-25-(3-Ethoxy-3-oxo-1-propenyl)-1,3,24-tris[[dimethyl(1,1-dimethylethyl)silyl]oxy]-26,27-cyclo-9,10-secocholesta-5,7,10(19),22-tetraen **23b** als farblosen Schaum erhält.

$^1$H-NMR (300 MHz, $CDCl_3$): δ= 0,06 ppm (s, 18H); 0,53 (s, 3H); 0,89 (s, 27H); 1,02 (d, 3H); 1,25 (t, 3H); 3,89 (d, 1H); 4,16 (q, 2H); 4,18 (m, 1H); 4,38 (m, 1H); 4,85 (brs, 1H); 5,18 (brs, 1H); 5,30 (dd, 1H); 5,41 (dd, 1H); 5,68 (d, 1H); 6,00 (d, 1H); 6,22 (d, 1H); 6,98 (d, 1H)

e) Man löst 100 mg des Esters **23b** in 10 ml THF, gibt 287 mg Tetrabutylammoniumfluorid (Trihydrat) zu und rührt über Nacht bei Raumtemperatur. Analog 14a) wird wässrig aufgearbeitet und an Silicagel mit Essigester/Hexan chromatographiert, wobei 39 mg der Titelverbindung **24b** als farbloser Schaum anfallen.

$^1$H-NMR (300 MHz, $CDCl_3$): δ= 0,53 ppm (s, 3H); 1,05 (d, 3H); 1,27 (t, 3H); 3,91 (d, 1H); 4,15 (q, 2H); 4,20 (m, 1H); 4,41 (m, 1H); 4,97 (brs, 1H); 5,30 (brs, 1H); 5,40 (dd, 1H); 5,58 (dd, 1H); 5,79 (d, 1H); 5,99 (d, 1H); 6,37 (d, 1H); 6,93 (d, 1H)

**Beispiel 15**

[5*Z*,7*E*,22*E*,25(*E*)]-(1*S*,3*R*,24*S*)-25-(3-Ethoxy-3-oxo-1-propenyl)-26,27-cyclo-9,10-secocholesta-5,7,10(19),22-tetraen-1,3,24-triol (**24a**)

3,1 g (5*Z*,7*E*,22*E*)-(1*S*,3*R*,24*S*)-1,3-Bis[[dimethyl(1,1-dimethylethyl)silyl]oxy]-24-hydroxy-26,27-cyclo-9,10-secocholesta-5,7,10(19),22-tetraen-25-carbonsäureethylester **13a** (WO 94/07853) werden analog 14a), b), c), d) und e) zur Titelverbindung **24a** umgesetzt, welche als farbloser Schaum anfällt.

$^1$H-NMR (300 MHz, $CDCl_3$): δ= 0,56 ppm (s, 3H); 1,02 (d, 3H); 1,28 (t, 3H); 3,93 (d, 1H); 4,16 (q, 2H); 4,21 (m, 1H); 4,41 (m, 1H); 4,99 (brs, 1H); 5,31 (brs, 1H); 5,41 (dd, 1H); 5,61 (dd, 1H); 5,80 (d, 1H); 6,00 (d, 1H); 6,38 (d, 1H); 6,93 (d, 1H)

**Beispiel 16**

[5*Z*,7*E*,22*E*,25(*E*)]-(1*S*,3*R*,24*R*)-25-[3-(1,1-Dimethylethoxy)-3-oxo-1-propenyl]-26,27-cyclo-9,10-secocholesta-5,7,10(19),22-tetraen-1,3,24-triol **(26b)**

a) Analog 14d) wird der Aldehyd **22b** mit Diethylphosphonoessigsäure-t-butylester umgesetzt, wobei [5*Z*,7*E*,22*E*,25(*E*)]-(1*S*,3*R*,24*R*)-25-[3-(1,1-Dimethylethoxy)-3-oxo-1-propenyl]-1,3,24-tris-[[dimethyl(1,1-dimethylethyl)silyl]oxy]-26,27-cyclo-9,10-secocholesta-5,7,10(19),22-tetraen **25b** als farbloser Schaum anfällt.

$^1$H-NMR (300 MHz, $CDCl_3$): δ= 0,06 ppm (s, 18H); 0,53 (s, 3H); 0,89 (s, 27H); 1,02 (d, 3H); 1,46 (s, 9H); 3,91 (d, 1H); 4,18 (m, 1H); 4,37 (m, 1H); 4,87 (brs, 1H); 5,18 (brs, 1H); 5,29 (dd, 1H); 5,41 (dd, 1H); 5,59 (d, 1H); 6,00 (d, 1H); 6,22 (d, 1H); 6,81 (d, 1H)

b) Analog 14e) wird die Titelverbindung **26b** als farbloser Schaum erhalten.

$^1$H-NMR (300 MHz, $CDCl_3$): δ= 0,58 ppm (s, 3H); 1,06 (d, 3H); 1,47 (s, 9H); 3,39 (dd, 1H); 4,22 (m, 1H); 4,42 (m, 1H); 5,00 (brs, 1H); 5,23 (dd, 1H); 5,31 (brs, 1H); 5,52 (dd, 1H); 5,68 (d, 1H); 6,01 (d, 1H); 6,38 (d, 1H); 6,79 (d, 1H)

**Beispiel 17**

(5*Z*,7*E*,22*E*)-[1*S*,3*R*,24*S*,25(*S*)]-25-(1-Hydroxy-2,2,3,3,4,4,5,5,6,6,7,7,7-tridecafluorheptyl)-26,27-cyclo-9,10-secocholesta-5,7,10(19),22-tetraen-1,3,24-triol (**27a**α) und
(5*Z*,7*E*,22*E*)-[1*S*,3*R*,24*S*,25(*R*)]-25-(1-Hydroxy-2,2,3,3,4,4,5,5,6,6,7,7,7-tridecafluorheptyl)-26,27-cyclo-9,10-secocholesta-5,7,10(19),22-tetraen-1,3,24-triol (**27a**β)

Man legt 60 mg des Aldehydes **22a** [Synthese analog zu **22b** aus **13a** an Stelle von **13b**, siehe 14a)-c)] mit 107 mg Perfluorhexyliodid in Diethylether bei -78°C vor und tropft 0,12 ml Methyllithium/Lithiumbromid-Komplex (1,6 M in Ether) zu. Nach 30 Minuten bei -78°C wird analog 14a) wässrig aufgearbeitet und der Rückstand chromatographisch an Silicagel mit Essigester/Hexan gereinigt. Das anfallende Produkt (22 mg) wird in 10 ml THF gelöst, mit 60 mg Tetrabutylammoniumfluorid (Trihydrat) versetzt und über Nacht bei Raumtemperatur gerührt. Nach erneuter wässriger Aufarbeitung werden nun die diastereomeren Alkohole durch präparative Dünnschichtchromatographie mit Essigester/Hexan als Laufmittel getrennt, wobei 0,9 mg der Titelverbindung **27a**α und 2,45 mg der Titelverbindung **27a**β als farblose Schäume anfallen.

$^1$H-NMR (300 MHz, $CD_2Cl_2$):

**27a**α δ= 0,54 ppm (s, 3H); 1,00 (d, 3H); 3,35 (dd, 1H); 4,15 (m, 2H); 4,35 (m, 1H); 4,75 (brs, 1H); 4,93 (brs, 1H); 4,97 (d, 1H); 5,08 (dd, 1H); 5,27 (dd, 1H); 5,53 (dd, 1H); 6,00 (d, 1H); 6,34 (d, 1H)

**27a**β δ= 0,55 ppm (s, 3H); 1,02 (d, 3H); 3,36 (d, 1H); 3,76 (d, 1H); 4,07 (dd, 1H); 4,16 (m, 1H); 4,36 (m, 1H); 4,95 (brs, 1H); 5,18 (brs, 1H); 5,51 (dd, 1H); 5,62 (dd, 1H); 6,00 (d, 1H); 6,35 (d, 1H)

**Beispiel 18**

(5*Z*,7*E*,22*E*)-[1*S*,3*R*,24*R*,25(*S*)]-25-(1-Hydroxy-2,2,3,3,4,4,5,5,6,6,7,7,7-tridecafluorheptyl)-26,27-cyclo-9,10-secocholesta-5,7,10(19),22-tetraen-1,3,24-triol (**27b**α) und
(5*Z*,7*E*,22*E*)-[1*S*,3*R*,24*R*,25(*R*)]-25-(1-Hydroxy-2,2,3,3,4,4,5,5,6,6,7,7,7-tridecafluorheptyl)-26,27-cyclo-9,10-secocholesta-5,7,10(19),22-tetraen-1,3,24-triol (**27b**β)

In Analogie zu 17) werden 50 mg des Aldehydes **22b** umgesetzt, wobei schließlich 4,50 mg der Titelverbindung **27b**α und 3,15 mg der Titelverbindung **27b**β als farblose Schäume anfallen.

$^1$H-NMR (300 MHz, $CD_2Cl_2$):

**27b**α δ= 0,54 ppm (s, 3H); 1,02 (d, 3H); 3,33 (dd, 1H); 4,15 (m, 1H); 4,16 (m, 1H); 4,36 (m, 1H); 4,84 (brs, 1H); 4,95 (brs, 1H); 4,99 (d, 1H); 5,07 (dd, 1H); 5,17 (dd, 1H); 5,51 (dd, 1H); 6,00 (d, 1H); 6,35 (d, 1H)

**27b**β δ= 0,55 ppm (s, 3H); 1,03 (d, 3H); 3,52 (d, 1H); 3,73 (dd, 1H); 3,98 (s, 1H); 4,17 (m, 2H); 4,36 (m, 1H); 4,95 (brs, 1H); 5,18 (brs, 1H); 5,56 (m, 2H); 6,00 (d, 1H); 6,35 (d, 1H)

**Beispiel 19**

(5*Z*,7*E*,22*E*)-(1*S*,3*R*,24*R*)-25-Acetyl-20-methyl-26,27-cyclo-9,10-secocholesta-5,7,10(19),22-tetraen-1,3,24-triol **(33b)** und (5*Z*,7*E*,22*E*)-(1*S*,3*R*,24*S*)-25-Acetyl-20-methyl-26,27-cyclo-9,10-secocholesta-5,7,10(19),22-tetraen-1,3,24-triol (**33a**)

a) Aus 3,4 ml Diisopropylamin und 8,7 ml n-Butyllithium-Lösung (2.5 M in Hexan) in 250 ml THF wird bei 0°C unter Argon Lithiumdiisopropylamid (LDA) bereitet und die Lösung dann auf-78°C abgekühlt. Nun tropft man 3,5 g 1-Acetylcyclopropancarbonsäuremethylester [D.F. Taber et al. *J. Org. Chem.* **57**, 456 (1992)] zu und rührt 1 h. Anschließend werden 3,2 g (5*Z*,7*E*)-(1*S*,3*R*)-1,3-Bis[[dimethyl(1,1-dimethylethyl)silyl]oxy]-20-methyl-9,10-secopregna-5,7,10(19)-trien-20-carbaldehyd (WO 93/12081) **28** in 20 ml THF zugetropft und 2 h bei 0°C gerührt. Nach Zugabe von gesättigter Ammoniumchlorid-Lösung bei -20°C wird mit gesättigter Natriumchlorid-Lösung verdünnt, mit Essigester unter Zusatz von 5% Oxalsäure extrahiert über Natriumsulfat getrocknet und eingeengt. Das so erhaltene Rohprodukt (5*Z*,7*E*,22*E*)-(1*S*,3*R*)-1,3-Bis[[dimethyl(1,1-dimethylethyl)silyl]oxy]-20-methyl-24-oxo-26,27-cyclo-9,10-secocholesta-5,7,10(19),22-tetraen-25-carbonsäure **29** (4,3 g feste Masse) wird ohne weitere Reinigung weiter umgesetzt.

b) Eine Menge von 2,23 g **29**, 990 mg Dicyclohexylcarbodiimid und 552 mg N-Hydroxysuccinimid werden in 30 ml Methylenchlorid gelöst und unter Argon 2 h gerührt. Nun werden 0,81 ml Dimethylamin zugegeben und die Mischung über Nacht bei Raumtemperatur gerührt. Man verdünnt mit Natriumchlorid-Lösung, extrahiert mit Methylenchlorid, trocknet über Natriumsulfat, entfernt das Solvens und reinigt den Rückstand durch Chromatographie an Kieselgel mit Essigester/Hexan, wobei 1,5 g (5*Z*,7*E*,22*E*)-(1*S*,3*R*)-1,3-Bis[[dimethy](1,1-dimethylethyl)silyl]oxy]-24-oxo-N,N,20-trimethyl-26,27-cyclo-9,10-secocholesta-5,7,10(19),22-tetraen-25-carbonsäureamid **30** als farbloser Schaum anfallen.

$^1$H-NMR (300 MHz, $CDCl_3$): δ= 0,07 ppm (s, 12H); 0,48 (s, 3H); 0,86 (s, 18H); 1,07 (s, 3H); 1,10 (s, 3H); 2,91 (s, 3H); 2,98 (s, 3H); 4,18 (m, 1H); 4,37 (m, 1H); 4,82 (brs, 1H); 5,18 (brs, 1H); 5,97 (d, 1H); 6,13 (d, 1H); 6,19 (d, 1H); 7,20 (d, 1H)

c) Man setzt 3,2 g **30** analog 1b) um und erhält nach Chromatographie an Kieselgel mit Essigester/Hexan 2,7 g (5*Z*,7*E*,22*E*)-(1*S*,3*R*)-1,3-Bis[[dimethyl(1,1-dimethylethyl)silyl]oxy]-24-hydroxy-N,N,20-trimethyl-26,27-cyclo-9,10-secocholesta-5,7,10(19),22-tetraen-25-carbonsäuredimethylamid **31** als Diastereomerengemisch bezüglich C-24 als farblosen Schaum.

$^1$H-NMR (300 MHz, $CDCl_3$): δ= 0,07 ppm (s, 12H); 0,55 (s, 3H); 0,89 (s, 18H); 1,02/1,03 (2x s, 3H); 1,08/1,09 (2x s, 3H); 3,04 (brs, 6H); 4,02 (m, 1H); 4,19 (m, 1H); 4,39 (m, 1H); 4,87 (brs, 1H); 5,20 (brs, 1H); 5,27 (d, 1H); 5,88 (d, 1H); 6,00 (d, 1H); 6,22 (d, 1H)

d) Man legt 300 mg **31** in 2 ml Diethylether vor und tropft bei -78°C unter Argon 1,03 ml Methyllithium-Lösung (1.3 M in Diethylether) hinzu. Man rührt 30 min bei -78°C und weitere 30 min bei -30°C. Anschließend quencht man mit Natriumchlorid-Lösung, extrahiert mit Essigester, wäscht die vereinigten organischen Phasen mit Natriumchlorid-Lösung, trocknet über Natriumsulfat und entfernt das Solvens. Chromatographie des Rohproduktes an Kieselgel mit Essigester/Hexan ergibt 165 mg (5*Z*,7*E*,22*E*)-(1*S*,3*R*)-25-Acetyl-1,3-bis[[dimethyl(1,1-dimethylethyl)silyl]oxy]-20-methyl-26,27-cyclo-9,10-secocholesta-5,7,10(19),22-tetraen-24-ol **32** als farblosen Schaum.

$^1$H-NMR (300 MHz, $CD_2Cl_2$): δ= 0,04 ppm (s, 12H); 0,53 (s, 3H); 0,84 (s, 18H); 1,02/1,03 (2x s, 3H); 1,05/1,06 (2x s, 3H); 1,93/1,94 (2x s, 3H); 2,90/2,94 (2x d, OH); 4,04/4,09 (2x t, 1H); 4,16 (m, 1H); 4,35 (m, 1H); 4,82 (brs, 1H); 5,17 (brs, 1H); 5,29/5,30 (2x dd, 1H); 5,79 (d, 1H); 5,98 (d, 1H); 6,22 (d, 1H)

e) Man behandelt 160 mg **32** analog 1e) und erhält nach Chromatographie an Kieselgel mit Essigester/Hexan sowie Diastereomerentrennung über HPLC an chiraler Phase mit Hexan/Isopropanol/Ethanol nacheinander 12 mg **33b** und 21 mg **33a** als farblose Schäume.

$^1$H-NMR (300 MHz, $CD_2Cl_2$):

**33b** δ= 0,56 ppm (s, 3H); 1,02 (s, 3H); 1,07 (s, 3H); 1,95 (s, 3H); 3,00 (d, OH); 4,08 (m, 1H); 4,17 (m, 1H); 4,38 (m, 1H); 4,95 (brs, 1H); 5,29 (brs, 1H); 5,30 (dd, 1H); 5,80 (d, 1H); 5,99 (d, 1H); 6,35 (d, 1H)
**33a** δ= 0,57 ppm (s, 3H); 1,01 (s, 3H); 1,08 (s, 3H); 1,95 (s, 3H); 2,95 (d, OH); 4,13 (m, 1H); 4,16 (m, 1H); 4,38 (m, 1H); 4,96 (brs, 1H); 5,29 (brs, 1H); 5,30 (dd, 1H); 5,82 (d, 1H); 5,99 (d, 1H); 6,35 (d, 1H)

**Beispiel 20**

(5*Z*,7*E*,22*E*)-(1*S*,3*R*,24*R*)-20-Methyl-25-(1-oxobutyl)-26,27-cyclo-9,10-secocholesta-5,7,10(19),22-tetraen-1,3,24-triol (**35b**) und (5*Z*,7*E*,22*E*)-(1*S*,3*R*,24*S*)-20-Methyl-25-(1-oxobutyl)-26,27-cyclo-9,10-secocholesta-5,7,10(19),22-tetraen-1,3,24-triol (**35a**)

a) Man legt 0,9 ml Propyliodid in 20 ml Diethylether vor und tropft bei -78°C unter Argon 8 ml t-Butyllithium-Lösung (1,5 M in Pentan) zu. Diese Mischung wird 30 min bei -78°C gerührt und anschließend zu einer Lösung von 300 mg **31** in 2 ml Diethylether bei -78°C unter Argon getropft. Man rührt nun 3 h bei -78°C und quencht dann mit Natriumchlorid-Lösung. Es wird dann mit Essigester extrahiert, die vereinigten organischen Phasen werden mit Natriumchlorid-Lösung gewaschen über Natriumsulfat getrocknet und das Lösungsmittel entfernt. Der Rückstand wird an Kieselgel mit Essigester/Hexan chromatographiert, wobei 180 mg (5*Z*,7*E*,22*E*)-(1*S*,3*R*)-1,3-Bis[[dimethyl(1,1-dimethylethyl)silyl]oxy]-20-methyl-25-(1-oxobutyl)-26,27-cyclo-9,10-secocholesta-5,7,10(19),22-tetraen-24-ol **34** als farbloser Schaum anfallen.

$^1$H-NMR (300 MHz, $CD_2Cl_2$): δ= 0,03 ppm (s, 12H); 0,53 (s, 3H); 0,86 (s, 18H); 0,86 (t, 3H); 1,00/1,01 (2x s, 3H); 1,05/1,06 (2x s, 3H); 2,14 (t, 2H); 3,02/3,07 (2x d, OH); 4,02/4,05 (2x t, 1H); 4,17 (m, 1H); 4,37 (m, 1H); 4,83 (brs, 1H); 5,17 (brs, 1H); 5,29/5,30 (2x dd, 1H); 5,78 (d, 1H); 5,98 (d, 1H); 6,23 (d, 1H)

b) Man behandelt 160 mg **34** analog 1e) und erhält nach Chromatographie an Kieselgel mit Essigester/Hexan sowie Diastereomerentrennung über HPLC an chiraler Phase mit Hexan/Isopropanol/Ethanol nacheinander 14 mg **35b** und 27 mg **35a** als farblose Schäume.

$^1$H-NMR (300 MHz, $CD_2Cl_2$):

**35b** δ= 0,55 ppm (s, 3H); 0,88 (t, 3H); 1,02 (s, 3H); 1,07 (s, 3H); 2,14 (t, 2H); 3,10 (brs, OH); 4,05 (m, 1H); 4,17 (m, 1H); 4,38 (m, 1H); 4,95 (brs, 1H); 5,29 (brs, 1H); 5,30 (dd, 1H); 5,79 (d, 1H); 5,99 (d, 1H); 6,35 (d, 1H)
**35a** δ= 0,55 ppm (s, 3H); 0,88 (t, 3H); 1,00 (s, 3H); 1,09 (s, 3H); 2,16 (t, 2H); 3,06 (brs, OH); 4,08 (m, 1H); 4,17 (m, 1H); 4,37 (m, 1H); 4,95 (brs, 1H); 5,29 (brs, 1H); 5,30 (dd, 1H); 5,81 (d, 1H); 5,99 (d, 1H); 6,35 (d, 1H)

**Beispiel 21**

(5*Z*,7*E*,22*E*)-(1*S*,3*R*,24*R*)-20-Methyl-25-(1-oxopentyl)-26,27-cyclo-9,10-secocholesta-5,7,10(19),22-tetraen-1,3,24-triol **(37b)** und (5*Z*,7*E*,22*E*)-(1*S*,3*R*,24*S*)-20-Methyl-25-(1-oxopentyl)-26,27-cyclo-9,10-secocholesta-5,7,10(19),22-tetraen-1,3,24-triol (**37a**)

a) Man legt 250 mg **31** in 10 ml THF unter Argon vor und kühlt auf -78°C. Bei dieser Temperatur tropft man 1 ml n-Butyllithium-Lösung (1,6 M in Hexan) zu und rührt 4 h nach. Es wird dann mit Natriumchlorid-Lösung gequencht, mit Essigester extrahiert, über Natriumsulfat getrocknet, das Solvens entfernt und der Rückstand an Kieselgel mit Essigester/Hexan chromatographiert, wobei man 160 mg (5*Z*,7*E*,22*E*)-(1*S*,3*R*)-1,3-Bis[[dimethyl(1,1-dimethylethyl)silyl]oxy]-20-methyl-25-(1-oxopentyl)-26,27-cyclo-9,10-secocholesta-5,7,10(19),22-tetraen-24-ol **36** als farblosen Schaum erhält.

$^1$H-NMR (300 MHz, $CDCl_3$): δ= 0,05 ppm (s, 12H); 0,54 (s, 3H); 0,88 (s, 18H); 0,90 (t, 3H); 1,02/1,03 (2x s, 3H); 1,07/1,08 (2x s, 3H); 3,27/3,28 (2x d, OH); 4,08 (m, 1H); 4,20 (m, 1H); 4,38 (m, 1H); 4,87 (brs, 1H); 5,19 (brs, 1H); 5,32/5,33 (2x dd, 1H); 5,80 (d, 1H); 5,99 (d, 1H); 6,22 (d, 1H)

b) Man behandelt 150 mg **36** analog 1e) und erhält nach Chromatographie an Kieselgel mit Essigester/Hexan sowie Diastereomerentrennung über HPLC an chiraler Phase mit Hexan/Isopropanol/Ethanol nacheinander 12 mg **37b** und 22 mg **37a** als farblose Schäume.

$^1$H-NMR (300 MHz, $CD_2Cl_2$):

**37b** δ= 0,55 ppm (s, 3H); 0,89 (t, 3H); 1,01 (s, 3H); 1,07 (s, 3H); 2,18 (t, 2H); 3,09 (d, OH); 4,04 (t, 1H); 4,17 (m, 1H); 4,38 (m, 1H); 4,96 (brs, 1H); 5,30 (brs, 1H); 5,31 (dd, 1H); 5,80 (d, 1H); 5,99 (d, 1H); 6,35 (d, 1H)
**37a** δ= 0,56 ppm (s, 3H); 0,88 (t, 3H); 1,00 (s, 3H); 1,08 (s, 3H); 2,19 (t, 2H); 2,99 (d, OH); 4,08 (m, 1H); 4,17 (m, 1H); 4,38 (m, 1H); 4,96 (brs, 1H); 5,30 (brs, 1H); 5,31 (dd, 1H); 5,82 (d, 1H); 5,99 (d, 1H); 6,35 (d, 1H)

**Beispiel 22**

(5*Z*,7*E*,22*E*)-(1*S*,3*R*,24*R*)-20-Methyl-25-(1-oxohexyl)-26,27-cyclo-9,10-secocholesta-5,7,10(19),22-tetraen-1,3,24-triol (**39b**)

a) Analog 20a) werden 150 mg **31** mit 1-Pentyllithium (aus 1-Iodpentan und t-Butyllithium) umgesetzt, wobei man 170 mg (5*Z*,7*E*,22*E*)-(1*S*,3*R*)-1,3-Bis[[dimethyl(1,1-dimethylethyl)silyl]oxy]-20-methyl-25-(1-oxohexyl)-26,27-cyclo-9,10-secocholesta-5,7,10(19),22-tetraen-24-ol **38** als farblosen Schaum erhält. Die Trennung der Diastereomeren erfolgte durch mehrmalige Chromatographie an Aluminiumoxid-Platten mit Essigester/Hexan. Man erhielt so 25 mg (5*Z*,7*E*,22*E*)-(1*S*,3*R*,24*R*)-1,3-Bis[[dimethyl(1,1-dimethylethyl)-silyl]oxy]-20-methyl-25-(1-oxohexyl)-26,27-cyclo-9,10-secocholesta-5,7,10(19),22-tetraen-24-ol **38b** und 18 mg (5*Z*,7*E*,22*E*)-(1*S*,3*R*,24*S*)-1,3-Bis[[dimethyl(1,1-dimethylethyl)-silyl]oxy]-20-methyl-25-(1-oxohexyl)-26,27-cyclo-9,10-secocholesta-5,7,10(19),22-tetraen-24-ol **38a** als farblose Schäume.

$^1$H-NMR (300 MHz, $CDCl_3$):

**38b** δ= 0,06 ppm (s, 12H); 0,53 (s, 3H); 0,87 (t, 3H); 0,88 (s, 18H); 0,99 (s, 3H); 1,05 (s, 3H); 3,09 (d, OH); 4,02 (t, 1H); 4,18 (m, 1H); 4,39 (m, 1H); 4,83 (brs, 1H); 5,19 (brs, 1H); 5,31 (dd, 1H); 5,79 (d, 1H); 5,99 (d, 1H); 6,22 (d, 1H)

**38a** δ= 0,06 ppm (s, 12H); 0,52 (s, 3H); 0,87 (t, 3H); 0,88 (s, 18H); 0,99 (s, 3H); 1,08 (s, 3H); 3,03 (d, OH); 4,06 (t, 1H); 4,18 (m, 1H); 4,38 (m, 1H); 4,83 (brs, 1H); 5,19 (brs, 1H); 5,30 (dd, 1H); 5,79 (d, 1H); 5,99 (d, 1H); 6,23 (d, 1H)

b) Man behandelt 24 mg **38b** analog 1e) und erhält nach Chromatographie an Kieselgel mit Essigester/Hexan 6 mg **39b** als farblosen Schaum.

$^1$H-NMR (300 MHz, $CD_2Cl_2$): δ= 0,55 ppm (s, 3H); 0,88 (t, 3H); 1,00 (s, 3H); 1,08 (s, 3H); 3,10 (brs, OH); 4,03 (m, 1H); 4,18 (m, 1H); 4,38 (m, 1H); 4,94 (brs, 1H); 5,29 (brs, 1H); 5,29 (dd, 1H); 5,79 (d, 1H); 5,99 (d, 1H); 6,34 (d, 1H)

**Beispiel 23**

(5*Z*,7*E*,22*E*)-(1*S*,3*R*,24*S*)-20-Methyl-25-(1-oxohexyl)-26,27-cyclo-9,10-secocholesta-5,7,10(19),22-tetraen-1,3,24-triol (**39a**)

Man behandelt 17 mg **38a** analog 1e) und erhält nach Chromatographie an Kieselgel mit Essigester/Hexan 5 mg **39a** als farblosen Schaum.

$^1$H-NMR (300 MHz, $CD_2Cl_2$): δ=0,56 ppm (s, 3H); 0,87 (t, 3H); 1,00 (s, 3H); 1,07 (s, 3H); 3,05 (brs, OH); 4,07 (m, 1H); 4,18 (m, 1H); 4,37 (m, 1H); 4,95 (brs, 1H); 5,29 (brs, 1H); 5,29 (dd, 1H); 5,80 (d, 1H); 5,98 (d, 1H); 6,35 (d, 1H)

**Beispiel 24**

(5*Z*,7*E*,22*E*)-(1*S*,3*R*,24*R*)-20-Methyl-25-(1-oxoheptyl)-26,27-cyclo-9,10-secocholesta-5,7,10(19),22-tetraen-1,3,24-triol (**41b**) und (5*Z*,7*E*,22*E*)-(1*S*,3*R*,24*S*)-20-Methyl-25-(1-oxoheptyl)-26,27-cyclo-9,10-secocholesta-5,7,10(19),22-tetraen-1,3,24-triol (**41a**)

a) Analog 20a) werden 300 mg **31** mit 1-Hexyllithium (aus 1-Iodhexan und t-Butyllithium) umgesetzt, wobei man 150 mg (5*Z*,7*E*,22*E*)-(1*S*,3*R*)-1,3-Bis[[dimethyl(1,1-dimethylethyl)-silyl]oxy]-20-methyl-25-(1-oxoheptyl)-26,27-cyclo-9,10-secocholesta-5,7,10(19),22-tetraen-24-ol **40** neben 230 mg des Ausgangsmaterials als farblose Schäume erhält.

$^1$H-NMR (300 MHz, $CDCl_3$): δ= 0,06 ppm (s, 12H); 0,53 (s, 3H); 0,87 (t, 3H); 0,89 (s, 18H); 1,02/1,03 (2x s, 3H); 1,08/1,09 (2x s, 3H); 3,25/3,29 (2x d, OH); 4,06/4,08 (2x t, 1H); 4,19 (m, 1H); 4,38 (m, 1H); 4,87 (brs, 1H); 5,20 (brs, 1H); 5,32/5,34 (dd, 1H); 5,80 (d, 1H); 5,99 (d, 1H); 6,22 (d, 1H)

b) Man behandelt 145 mg **40** analog le) und erhält nach Chromatographie an Kieselgel mit Essigester/Hexan sowie Diastereomerentrennung über HPLC an chiraler Phase mit Hexan/Isopropanol/Ethanol nacheinander 19 mg **41b**

und 11 mg **41a** als farblose Schäume.

$^1$H-NMR (300 MHz, $CD_2Cl_2$):

**41b** δ= 0,55 ppm (s, 3H); 0,89 (t, 3H); 1,01 (s, 3H); 1,07 (s, 3H); 2,17 (t, 2H); 3,10 (d, OH); 4,03 (t, 1H); 4,17 (m, 1H); 4,38 (m, 1H); 4,95 (brs, 1H); 5,29 (brs, 1H); 5,30 (dd, 1H); 5,79 (d, 1H); 5,99 (d, 1H); 6,35 (d, 1H)
**41a** δ= 0,55 ppm (s, 3H); 0,88 (t, 3H); 1,00 (s, 3H); 1,07 (s, 3H); 2,17 (t, 2H); 3,07 (brs, OH); 4,08 (m, 1H); 4,17 (m, 1H); 4,38 (m, 1H); 4,94 (brs, 1H); 5,28 (brs, 1H); 5,30 (dd, 1H); 5,80 (d, 1H); 5,99 (d, 1H); 6,35 (d, 1H)

**Beispiel 25**

(5*Z*,7*E*,22*E*)-(1*S*,3*R*,24*R*)-20-Methyl-25-(1-oxooctyl)-26,27-cyclo-9,10-secocholesta-5,7,10(19),22-tetraen-1,3,24-triol (**43b**) und (5*Z*,7*E*,22*E*)-(1*S*,3*R*,24*S*)-20-Methyl-25-(1-oxooctyl)-26,27-cyclo-9,10-secocholesta-5,7,10(19),22-tetraen-1,3,24-triol (**43a**)

a) Analog 20a) werden 300 mg **31** mit 1-Heptyllithium (aus 1-Iodheptan und t-Butyllithium) umgesetzt, wobei man 160 mg (5*Z*,7*E*,22*E*)-(1*S*,3*R*)-1,3-Bis[[dimethyl(1,1-dimethylethyl)-silyl]oxy]-20-methyl-25-(1-oxooctyl)-26,27-cyclo-9,10-secocholesta-5, 7,10(19),22-tetraen-24-ol **42** neben 210 mg des Ausgangsmaterials als farblose Schäume erhält.

$^1$H-NMR (300 MHz, $CDCl_3$): δ= 0,06 ppm (s, 12H); 0,52 (s, 3H); 0,88 (t, 3H); 0,89 (s, 18H); 1,00/1,01 (2x s, 3H); 1,05/1,06 (2x s, 3H); 3,21/3,28 (2x d, OH); 4,04/4,07 (2x t, 1H); 4,18 (m, 1H); 4,37 (m, 1H); 4,85 (brs, 1H); 5,18 (brs, 1H); 5,31/5,32 (dd, 1H); 5,79 (d, 1H); 5,98 (d, 1H); 6,21(d, 1H)

b) Man behandelt 155 mg **42** analog le) und erhält nach Chromatographie an Kieselgel mit Essigester/Hexan sowie Diastereomerentrennung über HPLC an chiraler Phase mit Hexan/Isopropanol/Ethanol nacheinander 31 mg **43b** und 25 mg **43a** als farblose Schäume.

$^1$H-NMR (300 MHz, $CD_2Cl_2$):

**43b** δ= 0,55 ppm (s, 3H); 0,88 (t, 3H); 1,01 (s, 3H); 1,06 (s, 3H); 2,17 (t, 2H); 3,12 (d, OH); 4,03 (t, 1H); 4,17 (m, 1H); 4,37 (m, 1H); 4,95 (brs, 1H); 5,29 (brs, 1H); 5,32 (dd, 1H); 5,80 (d, 1H); 5,99 (d, 1H); 6,34 (d, 1H)
**43a** δ= 0,54 ppm (s, 3H); 0,87 (t, 3H); 0,99 (s, 3H); 1,07 (s, 3H); 2,17 (t, 2H); 3,04 (brs, OH); 4,07 (m, 1H); 4,17 (m, 1H); 4,37 (m, 1H); 4,95 (brs, 1H); 5,29 (brs, 1H); 5,31 (dd, 1H); 5,80 (d, 1H); 5,99 (d, 1H); 6,34 (d, 1H)

**Beispiel 26**

(7*E*,22*E*)-(1*R*,3*R*,24*R*)-25-Acetyl-26,27-cyclo-19-nor-9,10-secocholesta-5,7,22-trien-1,3,24-triol (**55b**)

a) Man setzt 6,70 g [1*R*-[1α(*S**),3αβ,4α,7aα]]-α,7a-Dimethyloctahydro-4-[(triethylsilyl)oxy]-1H-inden-1-acetaldehyd **44** [H.H. Inhoffen et al. *Chem. Ber.* **91**, 780 (1958), *Chem. Ber.* **92**, 1772 (1959), W.G. Dauben et al. *Tetrahedron Lett.* **30**, 677 (1989), Triethylsilyl-Schutzgruppe an C-4-OH] analog 19a) um und erhält 11,6 g [1*R*-[1α[R*-(*E*)],3a β,4α,7aα]]-1-[4-[7a-Methyloctahydro-4-[(triethylsilyl)oxy]-1H-inden-1-yl]-1-oxo-2-pentenyl]cyclopropancarbonsäure **45** als gelbes Öl.

b) Man setzt 15 g des Rohproduktes **45** analog 19b) um und erhält 11,9 g [1*R*-[1α[*R**-(*E*)],3aβ,4α,7aα]]-N,N-Dimethyl-1-[4-[7a-methyloctahydro-4-[(triethylsilyl)oxy]-1H-inden-1-yl]-1-oxo-2-pentenyl]cyclopropancarbonsäureamid **46** als farbloses Öl.

$^1$H-NMR (300 MHz, $CDCl_3$): δ= 0,52 ppm (q, 6H); 0,92 (s, 3H); 0,93 (t, 9H); 2,95 (s, 3H); 3,00 (s, 3H); 4,01 (s, 1H); 6,15 (d, 1H); 6,82 (d, 1H)

c) Man setzt 11,6 g **46** analog 1b) um und erhält 8,7 g [1*R*-[1α[*R**-(*E*)],3aβ,4α,7aα]]-N,N-Dimethyl-1-[1-hydroxy-4-[7a-methyloctahydro-4-[(triethylsilyl)oxy]-1H-inden-1-yl]-2-pentenyl]cyclopropancarbonsäureamid **47** als farbloses Öl.

$^1$H-NMR (300 MHz, $CDCl_3$): δ=0,54 ppm (q, 6H); 0,89 (s, 3H); 0,94 (t, 9H); 2,62 (brd, OH); 3,04 (brs, 6H); 4,02 (s, 1H); 5,27/5,29 (2x dd, 1H); 5,51/5,54 (2x dd, 1H)

d) Man rührt 5,34 g **47** in 70 ml Methylenchlorid mit 3,2 ml Dihydropyran und 187 mg Pyridinium-p-Toluolsulfonat unter Argon bei Raumtemperatur für 3 Tage. Dann wird Natriumchlorid-Lösung zugegeben, mit Methylenchlorid extrahiert, die vereinigten organischen Phasen werden mit Natriumhydrogencarbonat-Lösung und Natriumchlorid-Lösung gewaschen, über Natriumsulfat getrocknet und vom Solvens befreit. Der Rückstand wird an Kieselgel mit Essigester/Hexan chromatographiert, wobei man 4,91 g [1*R*-[1α[*R**-(*E*)],3aβ,4α,7aα]]-N,N-Dimethyl-1-[4-[7a-methyloctahydro-4-[(triethylsilyl)oxy]-1H-inden-1-yl]-1-[(tetrahydro-2H-pyran-2-yl)oxy]-2-pentenyl]cyclopropancarbonsäureamid **48** als farbloses Öl erhält.

e) Man behandelt 5,92 g **48** analog le) und erhält 2,99 g [1*R*-[1α[*R**-(*E*)],3aβ,4α,7aα]]-N,N-Dimethyl-1-[4-(4-hydroxy-7a-methyloctahydro-1H-inden-1-yl)-1-[(tetrahydro-2H-pyran-2-yl)oxy]-2-pentenyl]cyclopropancarbonsäureamid **49** als farbloses Öl.

f) Man löst 2,67g **49** in 130 ml Methylenchlorid, gibt portionsweise 1,87 g Pyridiniumchlorochromat hinzu und rührt 2 h unter Argon bei Raumtemperatur. Anschließend wird mit Diethylether verdünnt, filtriert und das Lösungsmittel entfernt. Der Rückstand wird an Kieselgel mit Essigester/Hexan chromatographiert, wobei man 2,84 g [1*R*-[1α[*R**-(*E*)],3a β,7aα]]-N,N-Dimethyl-1-[4-(7a-methyloctahydro-4-oxo-1H-inden-1-yl)-1-[(tetrahydro-2H-pyran-2-yl)oxy]-2-pentenyl]cyclopropancarbonsäureamid **50** als farbloses Öl erhält.

g) Man löst 1,0 g (3R-trans)-[2-[3,5-Bis[[dimethyl(1,1-dimethylethyl)silyl]oxy]cyclohexyliden]ethyl]diphenylphosphinoxid **51** [H.F. DeLuca et al. *Tetrahedron Lett.* **32**, 7663 (1991)] in 10 ml THF und kühlt unter Argon auf -78°C. Nun tropft man 2,1 ml n-Butyllithium-Lösung (2.5 M in Hexan) zu und rührt 5 min nach. Anschließend gibt man 381 mg **50** in 7 ml THF zu und rührt 30 min bei -78°C. Danach quencht man mit Kalium/Natriumtartrat-Lösung, extrahiert mit Essigester, wäscht die organische Phase mit Natriumchlorid-Lösung, trocknet über Natriumsulfat und entfernt das Lösungsmittel. Der Rückstand wird an Kieselgel mit Essigester/Hexan chromatographiert, wobei 648 mg (7*E*,22*E*)-(1*R,*3*R*)-1,3-Bis[[dimethy](1,1-dimethylethyl)silyl]oxy]-N,N-dimethyl-24ξ-[(tetrahydro-2H-pyran-2-yl)oxy]-26,27-cyclo-19-nor-9,10-secocholesta-5,7,22-trien-25-carbonsäureamid **52** als farbloser Schaum verbleiben.

h) Man löst 350 mg **52** in 5 ml THF und tropft bei -78°C unter Argon 0,64 ml Methyllithium-Lösung (1.3 M in Diethylether) zu. Nach 90 min quencht man mit Natriumchlorid-Lösung, extrahiert mit Essigester, wäscht die organische Phase mit Natriumchlorid-Lösung, trocknet über Natriumsulfat und entfernt das Lösungsmittel. Der Rückstand wird an Kieselgel mit Essigester/Hexan chromatographiert, wobei 284 mg (7*E*,22*E*)-(1*R*,3*R*)-25-Acetyl-1,3-bis[[dimethyl(1,1-dimethylethyl)si]yl]oxy]-24ξ-[(tetrahydro-2H-pyran-2-yl)oxy]-26,27-cyclo-19-nor-9,10-secocholesta-5,7,22-trien **53** als farbloser Schaum verbleiben.

i) Man löst 279 mg **53** in 37 ml Methylenchlorid und behandelt bei -25°C unter Argon mit 0,74 ml Dimethylaluminiumchlorid-Lösung (1 M in Hexan), Man rührt 10 h bei dieser Temperatur und stellt den Ansatz gegebenenfalls über Nacht in den Tiefkühlschrank. Anschließend hydrolysiert man mit Natriumhydrogencarbonat-Lösung, extrahiert mit Methylenchlorid, wäscht die organische Phase mit Natriumchlorid-Lösung, trocknet über Natriumsulfat, entfernt das Lösungsmittel und chromatographiert den Rückstand an Kieselgel mit Essigester/Hexan. Die Trennung der diastereomeren Alkohohole (bzgl. C-24) wird an Aluminiumoxid-Platten mit Essigester/Hexan vollzogen. So erhält man 69 mg (7*E*,22*E*)-(1*R*,3*R*,24*S*)-25-Acetyl-1,3-bis[[dimethy](1,1-dimethylethyl)silyl]oxy]-26,27-cyclo-19-nor-9,10-secocholesta-5,7,22-trien-24-ol **54a** neben 36 mg (7*E*,22*E*)-(1*R*,3*R*,24*R*)-25-Acetyl-1,3-bis[[dimethyl (1,1-dimethylethyl)silyl]oxy]-26,27-cyclo-19-nor-9,10-secocholesta-5,7,22-trien-24-ol **54b** als farblose Schäume.

$^1$H-NMR (300 MHz, $CD_2Cl_2$):

**54a** δ= 0,05 ppm (s, 12H); 0,52 (s, 3H); 0,85 (s, 18H); 1,01 (d, 3H); 1,93 (s, 3H); 2,89 (d, OH); 4,07 (m, 2H); 4,16 (t, 1H); 5,34 (dd, 1H); 5,52 (dd, 1H); 5,80 (d, 1H); 6,16 (d, 1H)

**54b** δ= 0,05 ppm (s, 12H); 0,52 (s, 3H); 0,85 (s, 18H); 1,02 (d, 3H); 1,93 (s, 3H); 2,95 (d, OH); 4,06 (m, 3H); 5,33 (dd, 1H); 5,46 (dd, 1H); 5,80 (d, 1H); 6,16 (d, 1H)

j) Man behandelt 36 mg **54b** analog le) und erhält nach Chromatographie an Kieselgel mit Essigester/Hexan 20 mg **55b** als farblosen Schaum.

$^1$H-NMR (300 MHz, $CD_2Cl_2/CD_3OD$): δ= 0,53 ppm (s, 3H); 1,01 (s, 3H); 1,98 (s, 3H); 3,94 (m, 1H); 4,01 (m, 1H); 4,15 (m, 1H); 5,29 (dd, 1H); 5,47 (dd, 1H); 5,83 (d, 1H); 6,23 (d, 1H)

**Beispiel 27**

(7*E*,22*E*)-(1*R*,3*R*,24*S*)-25-Acetyl-26,27-cyclo-19-nor-9,10-secocholesta-5,7,22-trien-1,3,24-triol (**55a**)

a) Man behandelt 69 mg **54a** analog 1e) und erhält nach Chromatographie an Kieselgel mit Essigester/Hexan 25 mg **55a** als farblosen Schaum.

$^1$H-NMR (300 MHz, $CD_2Cl_2/CD_3OD$): δ= 0,53 ppm (s, 3H); 0,99 (s, 3H); 1,98 (s, 3H); 3,96 (m, 1H); 4,01 (m, 1H); 4,20 (t, 1H); 5,30 (dd, 1H); 5,50 (dd, 1H); 5,83 (d, 1H); 6,23 (d, 1H)

**Beispiel 28**

(7*E*,22*E*)-(1*R*,3*R*,24*R*)-25-(1-Oxopentyl)-26,27-cyclo-19-nor-9,10-secocholesta-5,7,22-trien-1,3,24-triol (**58b**)

a) Man behandelt 420 mg **52** analog 21a) und erhält nach Chromatographie an Kieselgel mit Essigester/Hexan 250 mg (7*E*,22*E*)-(1*R*,3*R*)-1,3-Bis[[dimethyl(1,1-dimethylethyl)silyl]oxy]-25-(1-oxopentyl)-24ξ-[(tetrahydro-2H-pyran-2-yl)oxy]-26,27-cyclo-19-nor-9,10-secocholesta-5,7,22-trien **56** als farblosen Schaum.

b) Man behandelt 232 mg **56** analog 26i) und erhält nach Chromatographie an Kieselgel mit Essigester/Hexan nebeneinander 64 mg (7*E*,22*E*)-(1*R*,3*R*,24*S*)-1,3-Bis[[dimethyl(1,1-dimethylethyl)silyl]oxy]-25-(1-oxopentyl)-26,27-cyclo-19-nor-9,10-secocholesta-5,7,22-trien-24-ol **57a** neben 42 mg (7*E*,22*E*)-(1*R*,3*R*,24*R*)-1,3-Bis[[dimethyl(1,1-dimethylethyl)silyl]oxy]-25-(1-oxopentyl)-26,27-cyclo-19-nor-9,10-secocholesta-5,7,22-trien-24-ol **57b** als farblose Schäume.

$^1$H-NMR (300 MHz, $CD_2Cl_2$):

**57a** δ= 0,04 ppm (s, 12H); 0,52 (s, 3H); 0,85 (s, 18H); 0,87 (t, 3H); 1,01 (d, 3H); 3,00 (d, OH); 4,04 (m, 2H); 4,12 (t, 1H); 5,32 (dd, 1H); 5,51 (dd, 1H); 5,81 (d, 1H); 6,15 (d, 1H)
**57b** δ= 0,04 ppm (s, 12H); 0,52 (s, 3H); 0,85 (s, 18H); 0,87 (t, 3H); 1,01 (d, 3H); 3,05 (d, OH); 4,05 (m, 3H); 5,34 (dd, 1H); 5,44 (dd, 1H); 5,81 (d, 1H); 6,15 (d, 1H)

c) Man behandelt 41 mg **57b** analog 1e) und erhält 15 mg **58b** als farblosen Schaum.

$^1$H-NMR (300 MHz, $CD_2Cl_2/CD_3OD$): δ= 0,52 ppm (s, 3H); 0,89 (t, 3H); 1,00 (d, 3H); 2,27 (t, 2H); 3,95 (m, 1H); 4,03 (m, 1H); 4,17 (t, 1H); 5,31 (dd, 1H); 5,48 (dd, 1H); 5,82 (d, 1H); 6,22 (d, 1H)

**Beispiel 29**

(7*E*,22*E*)-(1*R*,3*R*,24*S*)-25-(1-Oxopentyl)-26,27-cyclo-19-nor-9,10-secocholesta-5,7,22-trien-1,3,24-triol (**58a**)

Man behandelt 62 mg **57a** analog le) und erhält 27 mg **58a** als farblosen Schaum.
$^1$H-NMR (300 MHz, $CD_2Cl_2/CD_3OD$): δ= 0,51 ppm (s, 3H); 0,87 (t, 3H); 0,99 (d, 3H); 2,23 (t, 2H); 3,95 (m, 1H); 4,02 (m, 1H); 4,19 (t, 1H); 5,32 (dd, 1H); 5,50 (dd, 1H); 5,82 (d, 1H); 6,23 (d, 1H)

**Beispiel 30**

(7*E*,22*E*)-(1*R*,3*R*,24*R*)-25-(1-Oxohexyl)-26,27-cyclo-19-nor-9,10-secocholesta-5,7,22-trien-1,3,24-triol (**61b**)

a) Analog 20a) werden 500 mg **52** mit 1-Pentyllithium (aus 1-Iodpentan und t-Butyllithium) umgesetzt, wobei man nach Chromatographie mit Essigester/Hexan 321 mg (7*E*,22*E*)-(1*R*,3*R*)-1,3-Bis[[dimethyl(1,1-dimethylethyl)silyl]oxy]-25-(1-oxohexy])-24ξ-[(tetrahydro-2H-pyran-2-yl)oxy]-26,27-cyclo-19-nor-9,10-secocholesta-5,7,22-trien **59** als farblosen Schaum erhält.

b) Man behandelt 213 mg **59** analog 26i) und erhält nach Chromatographie an Kieselgel mit Essigester/Hexan nebeneinander 81 mg (7*E*,22*E*)-(1*R*,3*R*,24*S*)-1,3-Bis[[dimethyl(1,1-dimethylethyl)silyl]oxy]-25-(1-oxohexyl)-26,27-cyclo-19-nor-9,10-secocholesta-5,7,22-trien-24-ol **60a** neben 42 mg (7*E*,22*E*)-(1*R*,3*R*,24*R*)-1,3-Bis[[dimethyl(1,1-dimethylethyl)silyl]oxy]-25-(1-oxohexyl)-26,27-cyclo-19-nor-9,10-secocholesta-5,7,22-trien-24-ol **60b** als

farblose Schäume.
$^1$H-NMR (300 MHz, $CD_2Cl_2$):

**60a** δ= 0,04 ppm (s, 12H); 0,52 (s, 3H); 0,86 (s, 18H); 0,87 (t, 3H); 1,01 (d, 3H); 2,15 (t, 2H); 3,01 (d, OH); 4,05 (m, 2H); 4,12 (t, 1H); 5,33 (dd, 1H); 5,51 (dd, 1H); 5,81 (d, 1H); 6,15 (d, 1H)
**60b** δ= 0,04 ppm (s, 12H); 0,52 (s, 3H); 0,86 (s, 18H); 0,87 (t, 3H); 1,02 (d, 3H); 2,15 (t, 2H); 3,07 (d, OH); 4,02 (t, 1H); 4,06 (m, 2H); 5,34 (dd, 1H); 5,44 (dd, 1H); 5,81 (d, 1H); 6,15 (d, 1H)

c) Man behandelt 51 mg **60b** analog 1e) und erhält 27 mg **61b** als farblosen Schaum.
$^1$H-NMR (300 MHz, $CD_2Cl_2$): δ= 0,54 ppm (s, 3H); 0,87 (t, 3H); 1,02 (d, 3H); 2,16 (t, 2H); 3,11 (d, OH); 3,95 (m, 1H); 4,03 (m, 1H); 4,05 (t, 1H); 5,34 (dd, 1H); 5,46 (dd, 1H); 5,83 (d, 1H); 6,24 (d, 1H)

**Beispiel 31**

(7*E*,22*E*)-(1*R*,3*R*,24*S*)-25-(1-Oxohexyl)-26,27-cyclo-19-nor-9,10-secocholesta-5,7,22-trien-1,3,24-triol (**61a**)

Man behandelt 63 mg **60a** analog 1e) und erhält 32 mg **61a** als farblosen Schaum.
$^1$H-NMR (300 MHz, $CD_2Cl_2$): δ= 0,54 ppm (s, 3H); 0,87 (t, 3H); 1,01 (d, 3H); 2,16 (t, 2H); 3,04 (d, OH); 3,95 (m, 1H); 4,03 (m, 1H); 4,13 (t, 1H); 5,34 (dd, 1H); 5,51 (dd, 1H); 5,83 (d, 1H); 6,24 (d, 1H)

**Beispiel 32**

(7*E*,22*E*)-(1*R*,3*R*,24*R*)-25-(1-Oxoheptyl)-26,27-cyclo-19-nor-9,10-secocholesta-5,7,22-trien-1,3,24-triol (**64b**)

a) Analog 20a) werden 500 mg **52** mit 1-Hexyllithium (aus 1-Iodhexan und t-Butyllithium) umgesetzt, wobei man nach Chromatographie mit Essigester/Hexan 255 mg (7*E*,22*E*)-(1*R*,3*R*)-1,3-Bis[[dimethyl(1,1-dimethylethy])silyl] oxy]-25-(1-oxoheptyl)-24ξ-[(tetrahydro-2H-pyran-2-yl)oxy]-26,27-cyclo-19-nor-9,10-secocholesta-5,7,22-trien **62** als farblosen Schaum erhält.

b) Man behandelt 190 mg **62** analog 26i) und erhält nebeneinander nach Chromatographie an Kieselgel mit Essigester/Hexan 53 mg (7*E*,22*E*)-(1*R*,3*R*,24*S*)-1,3-Bis[[dimethyl(1,1-dimethylethyl)silyl]oxy]-25-(1-oxoheptyl)-26,27-cyclo-19-nor-9,10-secocholesta-5,7,22-trien-24-ol **63a** neben 29 mg (7*E*,22*E*)-(1*R*,3*R*,24*R*)-1,3-Bis[[dimethyl(1,1-dimethylethyl)silyl]oxy]-25-(1-oxoheptyl)-26,27-cyclo-19-nor-9,10-secocholesta-5,7,22-trien-24-ol **63b** als farblose Schäume.
$^1$H-NMR (300 MHz, $CD_2Cl_2$):

**63a** δ= 0,03 ppm (s, 12H); 0,53 (s, 3H); 0,85 (s, 18H); 0,87 (t, 3H); 1,01 (d, 3H); 2,16 (t, 2H); 3,00 (d, OH); 4,05 (m, 2H); 4,12 (t, 1H); 5,32 (dd, 1H); 5,51 (dd, 1H); 5,81 (d, 1H); 6,14 (d, 1H)
**63b** δ= 0,03 ppm (s, 12H); 0,53 (s, 3H); 0,85 (s, 18H); 0,87 (t, 3H); 1,02 (d, 3H); 2,16 (t, 2H); 3,05 (d, OH); 4,03 (t, 1H); 4,05 (m, 2H); 5,34 (dd, 1H); 5,44 (dd, 1H); 5,81 (d, 1H); 6,14 (d, 1H)

c) Man behandelt 29 mg **63b** analog 1e) und erhält 17 mg **64b** als farblosen Schaum.
$^1$H-NMR (300 MHz, $CD_2Cl_2$): δ= 0,53 ppm (s, 3H); 0,88 (t, 3H); 1,03 (d, 3H); 2,17 (t, 2H); 3,12 (d, OH); 3,98 (m, 1H); 4,05 (m, 1H); 4,08 (t, 1H); 5,35 (dd, 1H); 5,48 (dd, 1H); 5,84 (d, 1H); 6,26 (d, 1H)

**Beispiel 33**

(7*E*,22*E*)-(1*R*,3*R*,24*S*)-25-(1-Oxoheptyl)-26,27-cyclo-19-nor-9,10-secocholesta-5,7,22-trien-1,3,24-triol (**64a**)

Man behandelt 52 mg **63a** analog le) und erhält 27 mg **64a** als farblosen Schaum.
$^1$H-NMR (300 MHz, $CD_2Cl_2$): δ= 0,53 ppm (s, 3H); 0,88 (t, 3H); 1,02 (d, 3H); 2,16 (t, 2H); 3,08 (d, OH); 3,98 (m, 1H); 4,05 (m, 1H); 4,12 (t, 1H); 5,33 (dd, 1H); 5,51 (dd, 1H); 5,84 (d, 1H); 6,27 (d, 1H)

**Beispiel 34**

(7*E*,22*E*)-(1*R*,3*R*,24*R*)-25-(1-Oxooctyl)-26,27-cyclo-19-nor-9,10-secocholesta-5,7,22-trien-1,3,24-triol (**67b**)

a) Analog 20a) werden 380 mg **52** mit 1-Heptyllithium (aus 1-Iodheptan und t-Butyllithium) umgesetzt, wobei man nach Chromatographie mit Essigester/Hexan 224 mg (7*E*,22*E*)-(1*R*,3*R*)-1,3-Bis[[dimethyl(1,1-dimethylethyl)silyl] oxy]-25-(1-oxooctyl)-24ξ-[(tetrahydro-2H-pyran-2-yl)oxy]-26,27-cyclo-19-nor-9,10-secocholesta-5,7,22-trien **65** als farblosen Schaum erhält.

b) Man behandelt 103 mg **65** analog 26i) und erhält nach Chromatographie an Kieselgel mit Essigester/Hexan nebeneinander 28 mg (7*E*,22*E*)-(1*R*,3*R*,24*S*)-1,3-Bis[[dimethyl(1,1-dimethylethyl)silyl] oxy]-25-(1-oxooctyl)-26,27-cyclo-19-nor-9,10-secocholesta-5,7,22-trien-24-ol **66a** neben 24 mg (7*E*,22*E*)-(1*R*,3*R*,24*R*)-1,3-Bis[[dimethyl(1,1-dimethylethyl)silyl]oxy]-25-(1-oxooctyl)-26,27-cyclo-19-nor-9,10-secocholesta-5,7,22-trien-24-ol **66b** als farblose Schäume.

$^1$H-NMR (300 MHz, $CD_2Cl_2$):

**66a** δ= 0,04 ppm (s, 12H); 0,53 (s, 3H); 0,85 (s, 18H); 0,86 (t, 3H); 1,00 (d, 3H); 2,17 (t, 2H); 3,01 (d, OH); 4,06 (m, 2H); 4,12 (t, 1H); 5,32 (dd, 1H); 5,51 (dd, 1H); 5,80 (d, 1H); 6,14 (d, 1H)
**66b** δ= 0,04 ppm (s, 12H); 0,53 (s, 3H); 0,85 (s, 18H); 0,86 (t, 3H); 1,02 (d, 3H); 2,15 (t, 2H); 3,05 (d, OH); 4,02 (t, 1H); 4,06 (m, 2H); 5,35 (dd, 1H); 5,45 (dd, 1H); 5,80 (d, 1H); 6,14 (d, 1H)

c) Man behandelt 24 mg **66b** analog le) und erhält 10 mg **67b** als farblosen Schaum.
$^1$H-NMR (300 MHz, $CD_2Cl_2$): δ= 0,53 ppm (s, 3H); 0,87 (t, 3H); 1,01 (d, 3H); 2,17 (t, 2H); 3,08 (brs, OH); 3,98 (m, 1H); 4,06 (m, 2H); 5,36 (dd, 1H); 5,48 (dd, 1H); 5,83 (d, 1H); 6,27 (d, 1H)

**Beispiel 35**

(7*E*,22*E*)-(1*R*,3*R*,24*S*)-25-(1-Oxooctyl)-26,27-cyclo-19-nor-9,10-secocholesta-5,7,22-trien-1,3,24-triol **(67a)**

Man behandelt 38 mg **66a** analog le) und erhält 13 mg **67a** als farblosen Schaum.
$^1$H-NMR (300 MHz, $CD_2Cl_2$): δ= 0,53 ppm (s, 3H); 0,85 (t, 3H); 1,00 (d, 3H); 2,15 (t, 2H); 3,00 (brs, OH); 3,97 (m, 1H); 4,06 (m, 1H); 4,11 (t, 1H); 5,35 (dd, 1H); 5,50 (dd, 1H); 5,83 (d, 1H); 6,26 (d, 1H)

**Beispiel 36**

(7*E*,22*E*)-(1*R*,3*R*,24*R*)-25-(1-Oxononyl)-26,27-cyclo-19-nor-9,10-secocholesta-5,7,22-trien-1,3,24-triol (**70b**)

a) Analog 20a) werden 375 mg **52** mit 1-Octyllithium (aus 1-Iodoctan und t-Butyllithium) umgesetzt, wobei man nach Chromatographie mit Essigester/Hexan 212 mg (7*E*,22*E*)-(1*R*,3*R*)-1,3-Bis[[dimethyl(1,1-dimethylethyl)silyl] oxy]-25-(1-oxononyl)-24ξ-[(tetrahydro-2H-pyran-2-yl)oxy]-26,27-cyclo-19-nor-9,10-secocholesta-5,7,22-trien **68** als farblosen Schaum erhält.

b) Man behandelt 125 mg **68** analog 26i) und erhält nach Chromatographie an Kieselgel mit Essigester/Hexan nebeneinander 36 mg (7*E*,22*E*)-(1*R*,3*R*,24*S*)-1,3-Bis[[dimethyl(1,1-dimethylethyl)silyl]oxy]-25-(1-oxononyl)-26,27-cyclo-19-nor-9,10-secocholesta-5,7,22-trien-24-ol **69a** neben 24 mg (7*E*,22*E*)-(1*R*,3*R*,24*R*)-1,3-Bis[[dimethyl(1,1-dimethylethyl)silyl]oxy]-25-(1-oxononyl)-26,27-cyclo-19-nor-9,10-secocholesta-5,7,22-trien-24-ol **69b** als farblose Schäume.

$^1$H-NMR (300 MHz, $CD_2Cl_2$):

**69a** δ= 0,05 ppm (s, 12H); 0,52 (s, 3H); 0,85 (s, 18H); 0,86 (t, 3H); 1,01 (d, 3H); 2,16 (t, 2H); 3,02 (d, OH); 4,05 (m, 2H); 4,12 (t, 1H); 5,32 (dd, 1H); 5,51 (dd, 1H); 5,80 (d, 1H); 6,14 (d, 1H)
**69b** δ= 0,05 ppm (s, 12H); 0,53 (s, 3H); 0,85 (s, 18H); 0,86 (t, 3H); 1,02 (d, 3H); 2,16 (t, 2H); 3,05 (d, OH); 4,02 (t, 1H); 4,05 (m, 2H); 5,34 (dd, 1H); 5,45 (dd, 1H); 5,80 (d, 1H); 6,14 (d, 1H)

c) Man behandelt 36 mg **69b** analog 1e) und erhält 17 mg **70b** als farblosen Schaum.
$^1$H-NMR (300 MHz, $CD_2Cl_2$): δ= 0,54 ppm (s, 3H); 0,87 (t, 3H); 1,02 (d, 3H); 2,16 (t, 2H); 3,08 (brs, OH); 3,98 (m, 1H); 4,05 (m, 2H); 5,35 (dd, 1H); 5,48 (dd, 1H); 5,83 (d, 1H); 6,28 (d, 1H)

**Beispiel 37**

(7*E*,22*E*)-(1*R*,3*R*,24*S*)-25-(1-Oxononyl)-26,27-cyclo-19-nor-9,10-secocholesta-5,7,22-trien-1,3,24-triol (**70a**)

Man behandelt 24 mg **69a** analog 1e) und erhält 8 mg **70a** als farblosen Schaum.
$^1$H-NMR (300 MHz, $CD_2Cl_2$): δ= 0,54 ppm (s, 3H); 0,87 (t, 3H); 1,02 (d, 3H); 2,17 (t, 2H); 3,07 (d, OH); 3,98 (m, 1H); 4,05 (m, 1H); 4,12 (t, 1H); 5,34 (dd, 1H); 5,51 (dd, 1H); 5,83 (d, 1H); 6,28 (d, 1H)

**Beispiel 38**

(7*E*,22*E*)-(1*R*,3*R*,24*S*)-25-Acetyl-20-methyl-26,27-cyclo-19-nor-9,10-secocholesta-5,7,22-trien-1,3,24-triol (**81a**) und (7*E*,22*E*)-(1*R*,3*R*,24*R*)-25-Acetyl-20-methyl-26,27-cyclo-19-nor-9,10-secocholesta-5,7,22-trien-1,3,24-triol (**81b**)

a) Man legt 1,8 g Natriumhydrid (55% in Mineralöl) in 105 ml THF unter Argon vor und tropft eine Lösung von 10,8 g [1*R*-[1α(*S**),3αβ,4α,7aα]]-α,7a-Dimethyloctahydro-4-[(triethylsilyl)oxy]-1H-inden-1-acetaldehyd **44** [H.H. Inhoffen et al. *Chem. Ber.* **91**, 780 (1958), *Chem. Ber.* **92**, 1772 (1959), W.G. Dauben et al. *Tetrahedron Lett.* **30**, 677 (1989), Triethylsilylschutzgruppe an C-4-OH] in 45 ml THF zu. Anschließend werden 6,24 ml Iodmethan zugetropft und die Mischung wird über Nacht bei Raumtemperatur gerührt. Nun wird die Reaktionsmischung vorsichtig in Eiswasser gegossen und mit Essigester extrahiert. Die vereinigten organischen Phasen wäscht man mit Natriumchlorid-Lösung, trocknet über Natriumsulfat, entfernt das Solvens und chromatographiert den Rückstand an Kieselgel mit Essigester/Hexan, wobei man 7,52 g [1*S*-(1α,3aβ,4α,7aα)]-Octahydro-4-[(triethylsilyl)oxy]-α,α,7a-trimethyl-1H-inden-1-acetaldehyd **71**.
$^1$H-NMR (300 MHz, $CDCl_3$): δ= 0,57 ppm (q, 6H); 0,97 (t, 9H); 0,98 (s, 3H); 1,10 (s, 3H); 1,12 (s, 3H); 4,05 (m, 1H); 9,68 (s, 1H)

b) Man setzt 7,5 g **71** analog 19a) um und erhält 14,6 g [1*R*-[1α(*E*),3aβ,4α,7aα]]-1-[4-Methyl-4-[7a-methyloctahydro-4-[(triethylsilyl)oxy]-1H-inden-1-yl]-1-oxo-2-pentenyl]cyclopropan-carbonsäure **72** als gelbliches Öl.

c) Man setzt 12,9 g **72** analog 19b) um und erhält 5,8 g [1*R*-[1α(*E*),3aβ,4α,7aα]]-N,N-Dimethyl-1-[4-methyl-4-[7a-methyloctahydro-4-[(triethylsilyl)oxy]-1H-inden-1-yl]-1-oxo-2-pentenyl]cyclopropancarbonsäureamid **73** als farbloses Öl.
$^1$H-NMR (300 MHz, $CDCl_3$): δ= 0,54 ppm (q, 6H); 0,92 (t, 9H); 0,93 (s, 3H); 1,03 (s, 3H); 1,10 (s, 3H); 2,94 (s, 3H); 2,99 (s, 3H); 4,01 (m, 1H); 6,15 (d, 1H); 7,22 (d, 1H)

d) Man setzt 1,02 g **73** analog 1b) um und erhält 743 mg [1*R*-[1α(*E*),3aβ,4α,7aα]]-N,N-Dimethyl-1-[1-hydroxy-4-methyl-4-[7a-methyloctahydro-4-[(triethylsilyl)oxy]-1H-inden-1-yl]-2-pentenyl]cyclopropancarbonsäureamid **74** als farbloses Öl.
$^1$H-NMR (300 MHz, $CDCl_3$): δ= 0,54 ppm (q, 6H); 0,92 (t, 9H); 0,94 (s, 3H); 1,00/1,01 (2x s, 3H); 1,05, 1,06 (2x s, 3H); 3,03 (br s, 3H); 4,00 (m, 1H); 4,05 (m, 1H); 5,22 (d, 1H); 5,89 (d, 1H)

e) Man behandelt 1,01 g **74** in Analogie zu 26d) und erhält 938 mg [1*R*-[1α(*E*),3aβ,4α,7aα ]]-N,N-Dimethyl-1-[4-methyl-4-[7a-methyloctahydro-4-[(triethylsilyl)oxy]-1H-inden-1-yl]-1-[(tetrahydro-2H-pyran-2-yl)oxy]-2-pentenyl]cyclopropancarbonsäureamid **75** als farbloses Öl.

f) Man behandelt 1,64 g **75** in Analogie zu 1e) und erhält 1,08 g [1*R*-[1α(*E*),3aβ,4α,7aα]]-N,N-Dimethyl-1-[4-(4-hydroxy-7a-methyloctahydro-1H-inden-1-yl)-4-methyl-1-[(tetrahydro-2H-pyran-2-yl)oxy]-2-pentenyl]cyclopropancarbonsäureamid **76** als farbloses Öl.

g) 1,07 g **76** werden analog 26f) behandelt, wobei man 920 mg [1*R*-[1α(*E*),3aβ,7aα]]-N,N-Dimethyl-1-[4-methyl-4-(7a-methyloctahydro-4-oxo-1H-inden-1-yl)-1-[(tetrahydro-2H-pyran-2-yl)oxy]-2-pentenyl]cyclopropancarbonsäureamid **77** als farblosen Schaum erhält.

h) Analog 26g) setzt man 583 mg **77** mit 1,5 g (3R-trans)-[2-[3,5-Bis[[dimethyl(1,1-dimethylethyl)silyl]oxy]cyclohexyliden]ethyl]diphenylphosphinoxid **51** um und erhält nach chromatographischer Aufreinigung an Kieselgel mit Hexan/Essigester 1,02 g (7*E*,22*E*)-(1*R*,3*R*)-1,3-Bis[[dimethyl(1,1-dimethylethyl)silyl]oxy]-24ξ-[(tetrahydro-2H-pyran-2-yl)oxy]-N,N-20-trimethyl-26,27-cyclo-19-nor-9,10-secocholesta-5,7,22-trien-25-carbonsäureamid **78** als farblosen Schaum.

g) Man setzt 254 mg **78** analog 26h) mit Methyllithium um und erhält 136 mg (7*E*,22*E*)-(1*R*,3*R*)-25-Acetyl-1,3-bis[[dimethyl(1,1-dimethylethyl)silyl]oxy]-20-methyl-24ξ-[(tetrahydro-2H-pyran-2-yl)oxy]-26,27-cyclo-19-nor-9,10-secocholesta-5,7,22-trien **79** als farblosen Schaum.

h) Man behandelt 135 mg **79** analog 26i) und erhält 51 mg (7*E*,22*E*)-(1*R*,3*R*)-25-Acetyl-1,3-bis[[dimethyl(1,1-dimethylethyl)silyl]oxy]-20-methyl-26,27-cyclo-19-nor-9,10-secocholesta-5,7,22-trien-24-ol **80** als farblosen Schaum.

$^1$H-NMR (300 MHz, $CDCl_3$): δ= 0,05 ppm (s, 12H); 0,54 (s, 3H); 0,87 (s, 18H); 1,01/1,02 (2x s, 3H); 1,07/1,08 (2x s, 3H); 1,96/1,97 (2x s, 3H); 3,11/3,15 (m, OH); 4,08 (m, 3H); 5,32/5,34 (2x dd, 1H); 5,78 (d, 1H); 5,82 (d, 1H); 6,15 (d, 1H)

i) Man behandelt 50 mg **80** analog 1e) und erhält nach Chromatographie an Kieselgel mit Essigester/Hexan sowie Diastereomerentrennung über HPLC an chiraler Phase mit Hexan/Isopropanol/Ethanol nacheinander 4 mg **81b** und 5 mg **81a** als farblose Schäume.

$^1$H-NMR (300 MHz, $CD_2Cl_2$):

**81b** δ= 0,55 ppm (s, 3H); 1,02 (s, 3H); 1,08 (s, 3H); 1,95 (s, 3H); 3,98 (m, 1H); 4,07 (m, 1H); 4,08 (d, 1H); 5,31 (dd, 1H); 5,80 (d, 1H); 5,82 (d, 1H); 6,28 (d, 1H)
**81a** δ= 0,55 ppm (s, 3H); 1,01 (s, 3H); 1,08 (s, 3H); 1,96 (s, 3H); 2,94 (brs, OH); 3,99 (m, 1H); 4,08 (m, 1H); 4,12 (d, 1H); 5,31 (dd, 1H); 5,82 (d, 1H); 5,82 (d, 1H); 6,28 (d, 1H)

**Beispiel 39**

(7*E*,22*E*)-(1*R*,3*R*,24*S*)-20-Methyl-25-(1-oxopentyl)-26,27-cyclo-19-nor-9,10-secocholesta-5,7,22-trien-1,3,24-triol **(84a)** und (7*E*,22*E*)-(1*R*,3*R*,24*R*)-20-Methyl-25-(1-oxopentyl)-26,27-cyclo-19-nor-9,10-secocholesta-5,7,22-trien-1,3,24-triol (**84b**)

a) Man setzt 250 mg **78** analog 21a) um und erhält nach Chromatographie an Kieselgel mit Essigester/Hexan 200 mg (7*E*,22*E*)-(1*R*,3*R*)-1,3-Bis[[dimethyl(1,1-dimethylethyl)silyl]oxy]-20-methyl-25-(1-oxopentyl)-24ξ-[(tetrahydro-2H-pyran-2-yl)oxy]-26,27-cyclo-19-nor-9,10-secocholesta-5,7,22-trien **82** als farblosen Schaum.

b) Man behandelt 195 mg **82** analog 26i) und erhält 89 mg (7*E*,22*E*)-(1*R*,3*R*)-1,3-Bis[[dimethyl(1,1-dimethylethyl)silyl]oxy]-20-methyl-25-(1-oxopentyl)-26,27-cyclo-19-nor-9,10-secocholesta-5,7,22-trien-24-ol **83** als farblosen Schaum.

$^1$H-NMR (300 MHz, $CDCl_3$): δ=0,06 ppm (s, 12H); 0,55 (s, 3H); 0,88 (s, 18H); 0,90 (t, 3H); 1,01/1,02 (2x s, 3H); 1,07/1,08 (2x s, 3H); 2,14/2,15 (2x t, 2H); 3,23/3,29 (2x d, OH); 4,08 (m, 3H); 5,33/5,34 (2x dd, 1H); 5,79 (d, 1H); 5,82 (d, 1H); 6,16 (d, 1H)

c) Man behandelt 85 mg **83** analog 1e) und erhält nach Chromatographie an Kieselgel mit Essigester/Hexan sowie Diastereomerentrennung über HPLC an chiraler Phase mit Hexan/Isopropanol/Ethanol nacheinander 12 mg **84b** und 16 mg **84a** als farblose Schäume.

$^1$H-NMR (300 MHz, $CD_2Cl_2$):

**84b** δ= 0,55 ppm (s, 3H); 0,91 (t, 3H); 1,03 (s, 3H); 1,09 (s, 3H); 2,20 (t, 2H); 3,09 (d, OH); 4,00 (m, 1H); 4,07 (m, 2H); 5,31 (dd, 1H); 5,82 (d, 1H); 5,83 (d, 1H); 6,28 (d, 1H)
**84a** δ= 0,55 ppm (s, 3H); 0,91 (t, 3H); 1,02 (s, 3H); 1,10 (s, 3H); 2,20 (t, 2H); 3,09 (d, OH); 4,00 (m, 1H); 4,08 (m, 1H); 4,10 (m, 1H); 5,31 (dd, 1H); 5,82 (d, 1H); 5,82 (d, 1H); 6,28 (d, 1H)

**Beispiel 40**

(7*E*,22*E*)-(1*R*,3*R*,24*S*)-20-Methyl-25-(1-oxohexyl)-26,27-cyclo-19-nor-9,10-secocholesta-5,7,22-trien-1,3,24-triol (**87a**) und (7*E*,22*E*)-(1*R*,3*R*,24*R*)-20-Methyl-25-(1-oxohexyl)-26,27-cyclo-19-nor-9,10-secocholesta-5,7,22-trien-1,3,24-triol **(87b)**

a) Man setzt 250 mg **78** analog 20a) mit 1-Pentyllithium (aus 1-Iodpentan und t-Butyllithium) um und erhält nach Chromatographie an Kieselgel mit Essigester/Hexan 192 mg (7*E*,22*E*)-(1*R*,3*R*)-1,3-Bis[[dimethyl(1,1-dimethylethyl)silyl]oxy]-20-methyl-25-(1-oxohexyl)-24ξ-[(tetrahydro-2H-pyran-2-yl)oxy]-26,27-cyclo-19-nor-9,10-secocholesta-5,7,22-trien **85** als farblosen Schaum.

b) Man behandelt 187 mg **85** analog 26i) und erhält 91 mg (7*E*,22*E*)-(1*R*,3*R*)-1,3-Bis[[dimethyl( 1,1-dimethylethyl) silyl]oxy]-20-methyl-25-(1-oxohexyl)-26,27-cyclo-19-nor-9,10-secocholesta-5,7,22-trien-24-ol **86** als farblosen Schaum.

$^1$H-NMR (300 MHz, $CDCl_3$): δ= 0,06 ppm (s, 12H); 0,55 (s, 3H); 0,88 (s, 18H); 0,89 (t, 3H); 1,01/1,02 (2x s, 3H); 1,07/1,08 (2x s, 3H); 2,14/2,15 (2x t, 2H); 3,25/3,30 (2x d, OH); 4,08 (m, 3H); 5,34/5,35 (2x dd, 1H); 5,79 (d, 1H); 5,80 (d, 1H); 6,17 (d, 1H)

c) Man behandelt 91 mg **86** analog 1e) und erhält nach Chromatographie an Kieselgel mit Essigester/Hexan sowie Diastereomerentrennung über HPLC an chiraler Phase mit Hexan/Isopropanol/Ethanol nacheinander 12 mg **87b** und 13 mg **87a** als farblose Schäume.

$^1$H-NMR (300 MHz, $CD_2Cl_2$):

**87b** δ= 0,53 ppm (s, 3H); 0,87 (t, 3H); 1,01 (s, 3H); 1,07 (s, 3H); 2,17 (t, 2H); 3,12 (d, OH); 3,97 (m, 1H); 4,03 (m, 2H); 5,30 (dd, 1H); 5,78 (d, 1H); 5,80 (d, 1H); 6,26 (d, 1H)

**87a** δ= 0,53 ppm (s, 3H); 0,87 (t, 3H); 0,99 (s, 3H); 1,06 (s, 3H); 2,16 (t, 2H); 3,07 (d, OH); 3,96 (m, 1H); 4,07 (m, 2H); 5,30 (dd, 1H); 5,79 (d, 1H); 5,80 (d, 1H); 6,26 (d, 1H)

**Beispiel 41**

(7*E*,22*E*)-(1*R*,3*R*,24*S*)-20-Methyl-25-(1-oxoheptyl)-26,27-cyclo-19-nor-9,10-secocholesta-5,7,22-trien-1,3,24-triol **(90a)** und (7*E*,22*E*)-(1*R*,3*R*,24*R*)-20-Methyl-25-(1-oxoheptyl)-26,27-cyclo-19-nor-9,10-secocholesta-5,7,22-trien-1,3,24-triol (**90b**)

a) Man setzt 250 mg **78** analog 20a) mit 1-Hexyllithium (aus 1-Iodhexan und t-Butyllithium) um und erhält nach Chromatographie an Kieselgel mit Essigester/Hexan 174 mg (7*E*,22*E*)-(1*R*,3*R*)-1,3-Bis[[dimethyl(1,1-dimethylethyl)silyl]oxy]-20-methyl-25-(1-oxoheptyl)-24ξ-[(tetrahydro-2H-pyran-2-yl)oxy]-26,27-cyclo-19-nor-9,10-secocholesta-5,7,22-trien **88** als farblosen Schaum.

b) Man behandelt 169 mg **88** analog 26i) und erhält 68 mg (7*E*,22*E*)-(1*R*,3*R*)-1,3-Bis[[dimethyl( 1,1-dimethylethyl) silyl]oxy]-20-methyl-25-(1-oxoheptyl)-26,27-cyclo-19-nor-9,10-secocholesta-5,7,22-trien-24-ol **89** als farblosen Schaum.

$^1$H-NMR (300 MHz, $CDCl_3$): δ= 0,06 ppm (s, 12H); 0,56 (s, 3H); 0,88 (s, 18H); 0,89 (t, 3H); 1,01/1,02 (2x s, 3H); 1,07/1,08 (2x s, 3H); 2,14/2,15 (2x t, 2H); 3,25/3,30 (2x d, OH); 4,08 (m, 3H); 5,33/5,34 (2x dd, 1H); 5,79 (d, 1H); 5,81 (d, 1H); 6,17 (d, 1H)

c) Man behandelt 66 mg **89** analog 1e) und erhält nach Chromatographie an Kieselgel mit Essigester/Hexan sowie Diastereomerentrennung über HPLC an chiraler Phase mit Hexan/Isopropanol/Ethanol nacheinander 8 mg **90b** und 11 mg **90a** als farblose Schäume.

$^1$H-NMR (300 MHz, $CD_2Cl_2$):

**90b** δ= 0,54 ppm (s, 3H); 0,88 (t, 3H); 1,01 (s, 3H); 1,07 (s, 3H); 2,17 (t, 2H); 3,11 (brs, OH); 3,97 (m, 1H); 4,04 (m, 2H); 5,30 (dd, 1H); 5,78 (d, 1H); 5,80 (d, 1H); 6,26 (d, 1H)

**90a** δ= 0,54 ppm (s, 3H); 0,88 (t, 3H); 1,02 (s, 3H); 1,08 (s, 3H); 2,17 (t, 2H); 3,06 (d, OH); 3,97 (m, 1H); 4,05

(m, 1H); 4,06 (m, 1H); 5,30 (dd, 1H); 5,79 (d, 1H); 5,80 (d, 1H); 6,26 (d, 1H)

**Beispiel 42**

(7*E*,22*E*)-(1*R*,3*R*,24*S*)-20-Methyl-25-(1-oxooctyl)-26,27-cyclo-19-nor-9,10-secocholesta-5,7,22-trien-1,3,24-triol **(93a)** und (7*E*,22*E*)-(1*R*,3*R*,24*R*)-20-Methyl-25-(1-oxooctyl)-26,27-cyclo-19-nor-9,10-secocholesta-5,7,22-trien-1,3,24-triol (**93b)**

a) Man setzt 250 mg **78** analog 20a) mit 1-Heptyllithium (aus 1-Iodheptan und t-Butyllithium) um und erhält nach Chromatographie an Kieselgel mit Essigester/Hexan 191 mg (7*E*,22*E*)-(1*R*,3*R*)-1,3-Bis[[dimethyl(1,1-dimethylethyl)silyl]oxy]-20-methyl-25-(1-oxooctyl)-24ξ-[(tetrahydro-2H-pyran-2-yl)oxy]-26,27-cyclo-19-nor-9,10-secocholesta-5,7,22-trien **91** als farblosen Schaum.

b) Man behandelt 186 mg **91** analog 26i) und erhält 67 mg (7*E*,22*E*)-(1*R*,3*R*)-1,3-Bis[[dimethyl(1,1-dimethylethyl) silyl]oxy]-20-methyl-25-(1-oxooctyl)-26,27-cyclo-19-nor-9,10-secocholesta-5,7,22-trien-24-ol **92** als farblosen Schaum.

$^1$H-NMR (300 MHz, $CDCl_3$): δ= 0,06 ppm (s, 12H); 0,55 (s, 3H); 0,88 (s, 18H); 0,89 (t, 3H); 1,01/1,02 (2x s, 3H); 1,07/1,08 (2x s, 3H); 2,14/2,15 (2x t, 2H); 3,25/3,30 (2x d, OH); 4,08 (m, 3H); 5,34/5,35 (2x dd, 1H); 5,80 (d, 1H); 5,80 (d, 1H); 6,16 (d, 1H)

c) Man behandelt 65 mg **92** analog 1e) und erhält nach Chromatographie an Kieselgel mit Essigester/Hexan sowie Diastereomerentrennung über HPLC an chiraler Phase mit Hexan/Isopropanol/Ethanol nacheinander 6 mg **93b** und 8 mg **93a** als farblose Schäume.

$^1$H-NMR (300 MHz, $CD_2Cl_2$):

**93b** δ= 0,54 ppm (s, 3H); 0,88 (t, 3H); 1,02 (s, 3H); 1,06 (s, 3H); 2,17 (t, 2H); 3,11 (brs, OH); 3,98 (m, 1H); 4,03 (m, 2H); 5,30 (dd, 1H); 5,79 (d, 1H); 5,80 (d, 1H); 6,26 (d, 1H)

**93a** δ= 0,54 ppm (s, 3H); 0,88 (t, 3H); 1,01 (s, 3H); 1,07 (s, 3H); 2,17 (t, 2H); 3,07 (d, OH); 3,97 (m, 1H); 4,08 (m, 2H); 5,30 (dd, 1H); 5,80 (d, 1H); 5,80 (d, 1H); 6,26 (d, 1H)

**Beispiel 43**

(5*Z*,7*E*,22*E*)-[1*S*,3*R*,25(*S*)]-1,3-Dihydroxy-25-(1-hydroxy-2,2,3,3,4,4,5,5,5-nonafluorpentyl)-26,27-cyclo-9,10-secocholesta-5,7,10(19),22-tetraen-24-on (**106a**) und (5*Z*,7*E*,22*E*)-[1*S*,3*R*,25(*R*)]-1,3-Dihydroxy-25-(1-hydroxy-2,2,3,3,4,4,5,5,5-nonafluorpentyl)-26,27-cyclo-9,10-secocholesta-5,7,10(19),22-tetraen-24-on (**106b**)

a) Man löst 18,7 g 1-(1-Oxoethyl)cyclopropancarbonsäuremethylester **94** [D.F. Taber et a. *J. Org. Chem.* **57**, 436 (1992)] in 500 ml Benzol, fügt 30 ml Ethylenglykol und 500 mg p-Toluolsulfonsäure hinzu und erhitzt unter Argon am Wasserabscheider für 12 h zum Sieden. Nach dem Abkühlen wird die organische Phase mit Natriumhydrogencarbonat-Lösung und Natriumchlorid-Lösung gewaschen, über Natriumsulfat getrocknet und das Solvens entfernt. Der Rückstand wird im Vakuum destilliert, wobei 18,6 g 1-(2-Methyl-1,3-dioxolan-2-yl)cyclopropancarbonsäuremethylester **95** als farbloses Öl anfallen (Sdp.: 90°C, 1 mbar).

$^1$H-NMR (300 MHz, $CDCl_3$): δ= 1,02 ppm (m, 2H); 1,16 (m, 2H); 1,61 (s, 3H); 3,69 (s, 3H); 3,92 (m, 4H)

b) Man löst 24 g **95** in 700 ml Toluol, kühlt unter Argon auf 0°C und tropft dann 620 ml DIBAH-Lösung (1.2 M in Toluol) zu. Man rührt 2 h bei dieser Temperatur und gibt dann 15 ml Isopropanol und 150 ml Wasser zu und läßt über Nacht nachrühren. Anschließend wird filtriert, gründlich mit Toluol nachgewaschen, die organische Phase über Natriumsulfat getrocknet und das Solvens entfernt. Das Produkt 1-(2-Methyl-1,3-dioxolan-2-yl)cyclopropanmethanol **96** (gelbliches Öl) wird direkt weiter umgesetzt.

$^1$H-NMR (300 MHz, $CDCl_3$): δ= 0,47 ppm (m, 2H); 0,72 (m, 2H); 1,41 (s, 3H); 2,92 (t, OH); 3,53 (d, 2H); 3,97 (m, 4H)

c) Man löst 10 g **96** in 500 ml Methylenchlorid und fügt 3,7 g Natriumacetat (wasserfrei) sowie 19,3 g Pyridiniumchlorochromat hinzu. Unter Argon wird nun 2 h gerührt. Man verdünnt mit 1 l Diethylether und filtriert anschließend

über Celite. Einengen des Lösungsmittels gefolgt von chromatographischer Reinigung über Kieselgel mit Essigester/Hexan ergibt 8,1 g 1-(2-Methyl-1,3-dioxolan-2-yl)cyclopropancarbaldehyd **97** als farbloses Öl.

$^1$H-NMR (300 MHz, $CDCl_3$): δ= 1,16 ppm (m, 4H); 1,57 (s, 3H); 3,97 (m, 4H); 9,49 (s, 1H)

d) Man legt 1,2 g **97** und 3,92 ml Perfluorbutyliodid in 40 ml Diethylether unter Argon vor und tropft bei -78°C Methyllithium/Lithiumbromid-Komplex (1.5 M in Diethylether) zu. Nach 30 min wird mit Natriumchlorid-Lösung gequencht und mit Essigester verdünnt. Extraktion mit Essigester, Waschen der vereinigten organischen Phasen mit Natriumchlorid-Lösung, Trocknen über Natriumsulfat, Entfernen des Solvens und Chromatographie an Kieselgel mit Essigester/Hexan ergibt 2,1 g 1-(2-Methyl-1,3-dioxolan-2-yl)-α-(1,1,2,2,3,3,4,4,4-nonafluor-butyl)cyclopropanmethanol **98** als farbloses Öl.

$^1$H-NMR (300 MHz, $CDCl_3$): δ= 0,80 ppm (m, 1H); 0,97 (m, 1H); 1,22 (m, 2H); 1,38 (s, 3H); 3,80 (d, 1H); 3,98 (s, 4H)

e) Man löst 3,2 g **98** in 50 ml Methylenchlorid/Methanol 1:1 und fügt 750 mg p-Toluolsulfonsäure hinzu. Es wird unter Argon 2 h bei Raumtemperatur gerührt. Man gibt Natriumchlorid-Lösung zu, extrahiert mit Methylenchlorid, wäscht die organische Phase mit Natriumhydrogencarbonat- und Natriumchlorid-Lösung, trocknet über Natriumsulfat und engt ein. Der Rückstand wird an Kieselgel mit Essigester/Hexan chromatographiert, wobei man 2,7 g 1-[1-(1-Hydroxy-2,2,3,3,4,4,5,5,5-nonafluorpentyl)cyclopropyl]ethanon **99** als farbloses Öl erhält.

$^1$H-NMR (300 MHz, $CDCl_3$): δ= 1,24 ppm (m, 2H); 1,57 (m, 2H); 1,95 (s, 3H); 3,80 (ddd, 1H); 5,01 (d, OH)

f) Man löst 7,5 g (5*E*,7*E*)-(1*S*,3*R*)-1,3-Bis[[dimethyl(1,1-dimethylethyl)silyl]oxy]-9,10-secopregna-5,7,10(19)-trien-20-carbaldehyd **100** [M. J. Calverley *Tetrahedron* **43**, 4609 (1987)] in 200 ml Toluol, fügt 2 g Anthracen und 0,5 ml Triethylamin zu und bestrahlt unter Stickstoffdurchleitung in einer Pyrex-Apparatur mit einer Quecksilberhochdrucklampe für 30 min. Anschließend filtriert man, engt ein und chromatographiert den Rückstand an Kieselgel mit Essigester/Hexan, wobei man 7,1 g (5*Z*,7*E*)-(1*S*,3*R*)-1,3-Bis[[dimethyl(1,1-dimethylethyl)silyl]oxy]-9,10-secopregna-5,7,10(19)-trien-20-carbaldehyd **101** als farblosen Schaum erhält.

$^1$H-NMR (300 MHz, $CDCl_3$): δ= 0,05 ppm (m, 12H); 0,55 (s, 3H); 0,88 (s, 18H); 1,11 (d, 3H); 2,37 (m, 1H); 4,18 (m, 1H); 4,37 (m, 1H); 4,84 (brs, 1H); 5,17 (brs, 1H); 6,00 (d, 1H); 6,22 (1H); 9,58 (d, 1H)

g) Man stellt aus 5,0 ml Diisopropylamin und 12 ml n-Butyllithium-Lösung (2.5 M in Hexan) in 60 ml THF unter Argon Lithiumdiisopropylamid her und tropft 4 g **99** in 10 ml THF zu. Nach 30 min bei dieser Temperatur werden 3,5 g **101** in 5 ml THF zugetropft und 2 h weitergerührt. Man quencht mit Natriumchlorid-Lösung, extrahiert mit Essigester, wäscht die organische Phase mit Natriumchlorid-Lösung, trocknet über Natriumsulfat und entfernt das Solvens. Chromatographie an Kieselgel mit Essigester/Hexan ergibt 2,9 g (5*Z*,7*E*)-(1*S*,3*R*)-1,3-Bis[[dimethyl (1,1-dimethylethyl)silyl]oxy]-22-hydroxy-25-(1-hydroxy-2,2,3,3,4,4,5,5,5-nona-fluorpentyl)-26,27-cyclo-9,10-secocholesta-5,7,10(19)-trien-24-on **102** als farblosen Schaum.

h) Man rührt eine Mischung aus 1,3 g **102**, 3,8 ml Triethylamin, 2,1 ml Acetanhydrid und einer Spatelspitze DMAP in 50 ml Methylenchlorid für 2 h unter Argon bei Raumtemperatur. Danach wird gesättigte Natriumhydrogencarbonat-Lösung zugegeben und 30 min nachgerührt. Extraktion mit Essigester, Waschen der organischen Phase mit Natriumhydrogencarbonat- und Natriumchlorid-Lösung, Trocknen über Natriumsulfat, Einengen und Chromatographie an Kieselgel mit Essigester/Hexan liefern 850 mg (5*Z*,7*E*)-(1*S*,3*R*)-22-(Acetyloxy)-25-[1-(acetyloxy)-2,2,3,3,4,4,5,5,5-nonafluorpentyl]-1,3-bis[[dimethyl(1,1-dimethylethyl)silyl]oxy]-26,27-cyclo-9,10-secocholesta-5,7,10(19)-trien-24-on **103.**

i) 850 mg **103** werden in 5 ml Toluol gelöst und mit 8 ml Diazabicycloundecan (DBU) versetzt. Man rührt 1 h bei 40°C, verdünnt dann mit Essigester und wäscht die organische Phase mit verdünnter Salzsäure sowie Natriumhydrogencarbonat- und Natriumchlorid-Lösung. Trocknen über Natriumsulfat, Einengen und Chromatographie an Kieselgel mit Essigester/Hexan liefern 460 mg (5*Z*,7*E*,22*E*)-(1*S*,3*R*)-25-[1-(Acetyloxy)-2,2,3,3,4,4,5,5,5-nonafluorpentyl]-1,3-bis[[dimethyl(1,1-dimethylethyl)silyl]oxy]-26,27-cyclo-9,10-secocholesta-5,7,10(19),22-tetraen-24-on **104**.

j) Man löst 110 mg **104** in 5 ml Methanol, gibt 83 mg Kaliumcarbonat zu und rührt 1 h bei Raumtemperatur unter Argon. Anschließend gibt man Natriumchlorid-Lösung zu, extrahiert mit Essigester, wäscht mit Natriumchlorid-Lösung, trocknet über Natriumsulfat, engt ein und chromatographiert an Kieselgel mit Essigester/Hexan, wobei 39 mg (5*Z*,7*E*,22*E*)-(1*S*,3*R*)-1,3-Bis[[dimethyl(1,1-dimethylethyl)silyl]oxy]-25-(1-hydroxy-2,2,3,3,4,4,5,5,5-nonafluorpentyl)-26,27-cyclo-9,10-secocholesta-5,7,10(19),22-tetraen-24-on **105** als farbloser Schaum anfallen.

k) Man rührt eine Mischung aus 35 mg **105** und 350 mg Dowex-Ionentauscher (sauer, vorbehandelt mit Salzsäure und Methanol) in 10 ml Methylenchlorid/Methanol (1:9) unter Argon über Nacht. Man filtriert, wäscht gründlich mit Essigester nach, wäscht dann die organische Phase mit Natriumhydrogencarbonat- sowie Natriumchlorid-Lösung, trocknet über Natriumsulfat, engt ein und chromatographiert an Kieselgel mit Essigester/Hexan, wobei man nacheinander 12 mg **106a** und 9 mg **106b** als farblose Schäume erhält.

$^1$H-NMR (300 MHz, $CD_2Cl_2$):

**106a** δ= 0,58 ppm (s, 3H); 1,08 (d, 3H); 3,63 (m, 1H); 4,17 (m, 1H); 4,38 (m, 1H); 4,97 (s, 1H); 5,19 (d, OH); 5,30 (m, 1H); 5,83 (d, 1H); 6,01 (d, 1H); 6,37 (1H); 6,92 (dd, 1H)
**106b** δ= 0,50 ppm (s, 3H); 0,99 (d, 3H); 3,63 (m, 1H); 4,17 (m, 1H); 4,38 (m, 1H); 4,97 (s, 1H); 5,18 (d, OH); 5,30 (m, 1H); 5,89 (d, 1H); 6,01 (d, 1H); 6,37 (1H); 6,97 (dd, 1H)

**Beispiel 44**

(5*Z*,7*E*,22*E*)-(3*S*,24*R*)-25-(1-Oxopentyl)-26,27-cyclo-9,10-secocholesta-5,7,10(19),22-tetraen-3,24-diol (**113b**)

a) Man unterzieht 5 g (5*E*,7*E*)-(3*S*)-3-[[Dimethyl(1,1-dimethylethyl)silyl]oxy]-9,10-secopregna-5,7,10(19)-trien-20-carbaldehyd **107** [Darstellung siehe M. J. Calverley *Tetrahedron* **43**, 4609 (1987), Verzicht auf die Stufen zur 1α-Funktionalisierung] der in 43f) beschriebenen Prozedur und erhält nach Chromatographie an Kieselgel mit Essigester/Hexan 4,2 g (5*Z*,7*E*)-(3*S*)-3-[[Dimethyl(1,1-dimethylethyl)silyl]oxy]-9,10-secopregna-5,7,10(19)-trien-20-carbaldehyd **108** als farblosen Schaum.

$^1$H-NMR (300 MHz, $CDCl_3$): δ= 0,08 ppm (s, 6H); 0,60 (s, 3H); 0,89 (d, 9H); 1,14 (d, 3H); 3,83 (m, 1H); 4,78 (s, 1H); 5,01 (s, 1H); 6,03 (d, 1H); 6,18 (d, 1H); 9,59 (s, 1H)

b) Analog 19a) werden 4,2 g **108** umgesetzt, wobei man 5,3 g des Rohproduktes (5*Z*,7*E*,22*E*)-(3*S*)-3-[[Dimethyl (1,1-dimethylethyl)silyl]oxy]-24-oxo-26,27-cyclo-9,10-secocholesta-5,7,10(19),22-tetraen-25-carbonsäure **109** als gelbliches Öl erhält.

c) Analog 19b) setzt man 2,5 g **109** um und erhält nach Chromatographie mit Essigester/Hexan an Kieselgel 1,5 g (5*Z*,7*E*,22*E*)-(3*S*)-N,N-Dimethyl-3-[[dimethyl(1,1-dimethylethyl)silyl]oxy]-24-oxo-26,27-cyclo-9,10-secocholesta-5,7,10(19),22-tetraen-25-carbonsäureamid **110** als farblosen Schaum.

$^1$H-NMR (300 MHz, $CDCl_3$): δ= 0,06 ppm (s, 6H); 0,55 (s, 3H); 0,87 (d, 9H); 1,07 (d, 3H); 2,94 (s, 3H); 2,99 (s, 3H); 3,80 (m, 1H); 4,75 (s, 1H); 4,99 (s, 1H); 5,98 (d, 1H); 6,13 (d, 1H); 6,18 (d, 1H); 6,84 (dd, 1H)

d) Man setzt 3,16 g **110** analog 1b) um und erhält nach Chromatographie an Kieselgel mit Essigester/Hexan 2,06 g (5*Z*,7*E*,22*E*)-(3*S*)-N,N-Dimethyl-3-[[dimethyl(1,1-dimethylethyl)-silyl]oxy]-24-hydroxy-26,27-cyclo-9,10-secocholesta-5,7,10(19),22-tetraen-25-carbonsäureamid **111** als farblosen Schaum.

$^1$H-NMR (300 MHz, $CDCl_3$): δ= 0,05 ppm (s, 6H); 0,53 (s, 3H); 0,88 (d, 9H); 1,02 (d, 3H); 3,04 (brs, 6H); 3,80 (m, 1H); 4,00 (m, 1H); 4,77 (s, 1H); 5,00 (s, 1H); 5,30/5,32 (2x dd, 1H); 5,55/5,57 (2x dd, 1H); 5,99 (d, 1H); 6,14 (d, 1H)

e) Man setzt 200 mg **111** analog 21a) mit n-Butyllithium um und erhält nach Chromatographie an Kieselgel mit Essigester/Hexan 132 mg des Diastereomerengemisches bezüglich C-24, das durch mehrmalige Chromatograpie an Aluminiumoxid-Platten mit Essigester/Hexan in 60 mg (5*Z*,7*E*,22*E*)-(3*S*,24*S*)-3-[[Dimethyl(1,1-dimethylethyl) silyl]oxy]-25-(1-oxopentyl)-26,27-cyclo-9,10-secocholesta-5,7,10(19),22-tetraen-24-ol **112a** sowie 42 mg (5*Z*,7*E*,22*E*)-(3*S*,24*R*)-3-[[Dimethyl(1,1-dimethylethyl)silyl]oxy]-25-(1-oxopentyl)-26,27-cyclo-9,10-secocholesta-5,7,10 (19),22-tetraen-24-ol **112b** aufgetrennt wird.

$^1$H-NMR (300 MHz, $CD_2Cl_2$):

**112a** δ= 0,04 ppm (s, 6H); 0,52 (s, 3H); 0,85 (d, 9H); 0,86 (t, 3H); 1,00 (d, 3H); 2,15 (t, 2H); 3,20 (d, OH); 3,81 (m, 1H); 4,10 (t, 1H); 4,72 (s, 1H); 4,99 (s, 1H); 5,30 (dd, 1H); 5,50 (dd, 1H); 5,98 (d, 1H); 6,14 (d, 1H)
**112b** δ= 0,04 ppm (s, 6H); 0,52 (s, 3H); 0,85 (d, 9H); 0,86 (t, 3H); 1,01 (d, 3H); 2,14 (t, 2H); 3,04 (d, OH); 3,81 (m, 1H); 4,03 (t, 1H); 4,72 (s, 1H); 4,99 (s, 1H); 5,33 (dd, 1H); 5,44 (dd, 1H); 5,98 (d, 1H); 6,14 (d, 1H)

f) Man behandelt 42 mg **112b** analog 1e) und erhält nach Chromatographie an Kieselgel mit Essigester/Hexan 18 mg **113b**.

$^1$H-NMR (300 MHz, $CD_2Cl_2$); δ= 0,53 ppm (s, 3H); 0,88 (t, 3H); 2,18 (t, 2H); 3,10 (brs, OH); 3,88 (m, 1H); 4,05 (m, 1H); 4,79 (s, 1H); 5,03 (s, 1H); 5,34 (dd, 1H); 5,48 (dd, 1H); 6,01 (d, 1H); 6,22 (d, 1H)

**Beispiel 45**

(5*Z*,7*E*,22*E*)-(3*S*,24*S*)-25-(1-Oxopentyl)-26,27-cyclo-9,10-secocholesta-5,7,10(19),22-tetraen-3,24-diol (**113a**)

Man behandelt 60 mg **112a** analog 1e) und erhält nach Chromatographie an Kieselgel mit Essigester/Hexan 22 mg **113a.**

$^1$H-NMR (300 MHz, $CD_2Cl_2$); δ= 0,54 ppm (s, 3H); 0,88 (t, 3H); 2,18 (t, 2H); 3,05 (d, OH); 3,88 (m, 1H); 4,13 (m, 1H); 4,78 (s, 1H); 5,03 (s, 1H); 5,33 (dd, 1H); 5,52 (dd, 1H); 6,01 (d, 1H); 6,22 (d, 1H)

**Beispiel 46**

[5*Z*,7*E*,22*E*,25(*E*)]-(1*S*,3*R*,24*S*)-25-[3-(1,1-Dimethylethoxy)-3-oxo-1-propenyl]-24-methoxy-26,27-cyclo-9,10-secocholesta-5,7,10(19),22-tetraen-1,3-diol **(114a**) und [5*Z*,7*E*,22*E*,25(*E*)]-(1*S*,3*R*,24*R*)-25-[3-(1,1-Dimethylethoxy)-3-oxo-1-propenyl]-24-methoxy-26,27-cyclo-9,10-secocholesta-5,7,10(19),22-tetraen-1,3-diol (**114b**)

Man löst 260 mg **25b** (siehe 16a) in 20 ml Methylenchlorid/Methanol (1:9), gibt 2 g Dowex-WX8-Ionentauscher (sauer) hinzu und rührt unter Argon bei Raumtemperatur für 3 Tage. Anschließend wird filtriert, eingeengt und an Kieselgel mit Essigester/Hexan chromatographiert. Der Rückstand wird via HPLC an chiraler Phase mit Hexan/Isopropanol/Ethanol aufgetrennt, wobei 6 mg **114a** neben 5 mg **114b** als farblose Schäume anfielen.

$^1$H-NMR (300 MHz, $CDCl_3$):

**114a:** δ= 0,57 ppm (s, 3H); 1,05 (d, 3H); 1,47 (s, 9H); 3,30 (s, 3H); 3,40 (d, 1H); 4,22 (m, 1H); 4,43 (m, 1H); 5,00 (s, 1H); 5,23 (dd, 1H); 5,32 (s, 1H); 5,52 (dd, 1H); 5,68 (d, 1H); 6,02 (d, 1H); 6,38 (d, 1H); 6,79 (d, 1H)
**114b:** δ= 0,57 ppm (s, 3H); 1,07 (d, 3H); 1,48 (s, 9H); 3,28 (s, 3H); 3,38 (d, 1H); 4,23 (m, 1H); 4,44 (m, 1H); 5,00 (s, 1H); 5,23 (dd, 1H); 5,32 (s, 1H); 5,51 (dd, 1H); 5,68 (d, 1H); 6,02 (d, 1H); 6,38 (d, 1H); 6,79 (d, 1H)

**Beispiel 47**

(5*Z*,7*E*,22*E*)-(1*S*,3*R*,24*R*)-25-Hydroxymethyl-26,27-cyclo-9,10-secocholesta-5,7,10(19),22-tetraen-1,3,24-triol (**115b**)

Man behandelt 100 mg **21b** (siehe 14b) analog le) und erhält nach Chromatographie an Kieselgel mit Essigester/Hexan 36 mg **115b** als farblosen Schaum.

$^1$H-NMR (300 MHz, $CDCl_3$): δ= 0,57 ppm (s, 3H); 1,06 (d, 3H); 3,32 (dd, 1H); 3,84 (br s, OH); 3,88 (dd, 1H); 4,23 (m, 1H); 4,43 (m, 1H); 5,00 (s, 1H); 5,32 (s, 1H); 5,47 (dd, 1H); 5,57 (dd, 1H); 6,02 (d, 1H); 6,38 (d, 1H);

**Beispiel 48**

[5*Z*,7*E*,22*E*,25(*E*)]-(1*S*,3*R*,24*R*)-25-(3-Oxo-1-heptenyl)-26,27-cyclo-9,10-secocholesta-5,7,10(19),22-tetraen-1,3,24-triol (**117b**)

a) Man setzt 70 mg des Aldehydes **22b** analog 14d) mit (2-Oxohexyl)-phosphonsäuredimethylester [P. Mathey *Tetrahedron* **34**, 649 (1978)] und Natriumhydrid um und erhält nach Chromatographie an Kieselgel mit Essigester/Hexan 59 mg [5*Z*,7*E*,22*E*,25(*E*)]-(1*S*,3*R*,24*R*)-25-(3-Oxo-1-heptenyl)-1,3,24-tris-[[dimethyl(1,1-dimethylethyl)silyl]oxy]-26,27-cyclo-9,10-secocholesta-5,7,10(19),22-tetraen **116b** als farblosen Schaum.
$^1$H-NMR (300 MHz, $CDCl_3$): δ= 0,06 ppm (s, 18H); 0,55 (s, 3H); 0,90 (s, 27H); 0,92 (t, 3H); 1,05 (d, 3H); 2,50 (t, 2H); 3,85 (d, 1H); 4,20 (m, 1H); 4,38 (m, 1H); 4,88 (s, 1H); 5,18 (s, 1H); 5,33 (dd, 1H); 5,47 (dd, 1H); 5,97 (d, 1H); 6,02 (d, 1H); 6,23 (d, 1H); 6,93 (d, 1H)

b) Man behandelt 45 mg **116b** analog 1e) und erhält nach Chromatographie an Kieselgel mit Essigester/Hexan 12 mg **117b.**
$^1$H-NMR (300 MHz, $CDCl_3$): δ=0,58 ppm (s, 3H); 0,92 (t, 3H); 1,07 (d, 3H); 2,50 (t, 2H); 3,94 (d, 1H); 4,23 (m, 1H); 4,43 (m, 1H); 5,00 (brs, 1H); 5,22 (brs, 1H); 5,42 (dd, 1H); 5,60 (dd, 1H); 6,00 (d, 1H); 6,08 (d, 1H); 6,38 (d,

1H); 6,89 (d, 1H)

**Beispiel 49**

[5*Z*,7*E*,22*E*,25(*E,E*)]-(1*S*,3*R*,24*R*)-25-(1-Oxo-2,4-hexadienyl)-26,27-cyclo-9,10-secocholesta-5,7,10(19),22-tetraen-1,3,24-triol (**118b**)

5,94 ml n-Butyllithium (1.6 M in Hexan) werden bei 0°C zu 1,46 ml Diisopropylamin in 67 ml THF unter Stickstoff getropft. Nach 15 min wird die Reaktionsmischung auf -78°C gekühlt und es werden 540 mg **7b** (siehe 3) in 2,2 ml THF zugetropft. Nach Erwärmung auf 0°C rührt man die Reaktionsmischung in gesättigte Ammoniumchlorid-Lösung ein, extrahiert mit Essigester, trocknet die organische Phase über Natriumsulfat und engt ein. Chromatographie an Kieselgel mit Essigester/Hexan liefert 65 mg **118b** als farblosen Schaum.

$^1$H-NMR (300 MHz, $CDCl_3$): δ=0,57 ppm (s, 3H); 0,97 (m, 2H); 1,05 (d, 3H); 1,20 (m, 2H); 1,88 (d, 3H); 3,50 (m, 1H); 4,10 (m, 1H); 4,23 (m, 1H); 4,43 (m, 1H); 5,00 (brs, 1H); 5,32 (brs, 1H); 5,42 (dd, 1H); 5,53 (dd, 1H); 5,92 (d, 1H); 6,00 (d, 1H); 6,18 (m, 1H); 6,38 (d, 1H); 7,30 (dd, 1H)

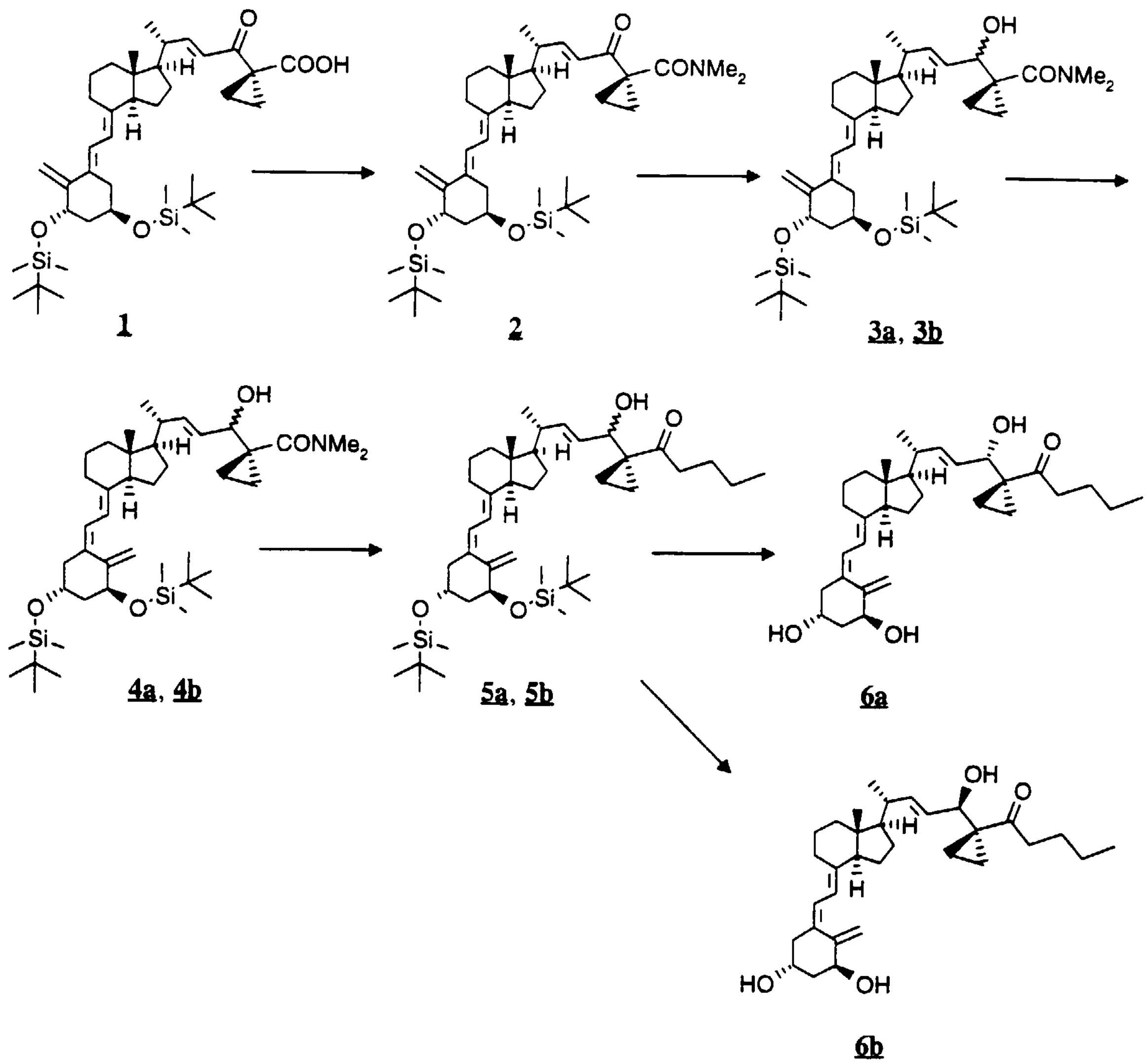

**1** **2** **3a**, **3b**

**4a**, **4b** **5a**, **5b** **6a**

**6b**

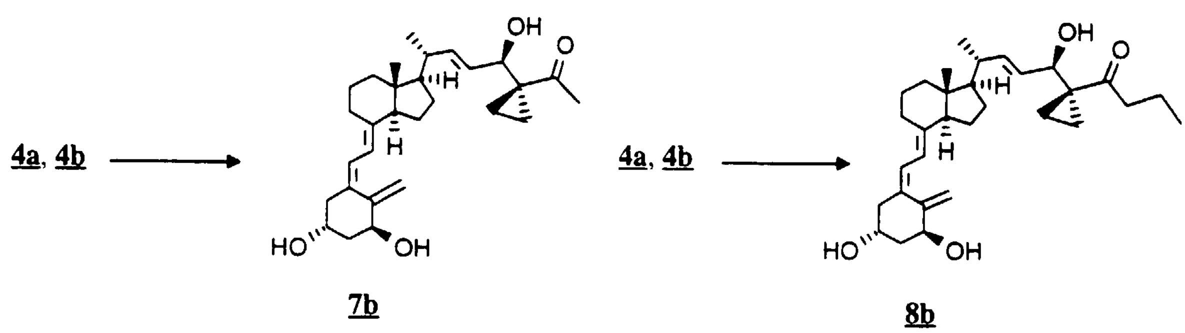

**4a**, **4b** **7b** **4a**, **4b** **8b**

4a, 4b

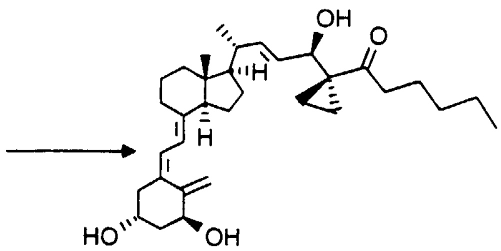

9b

4a, 4b

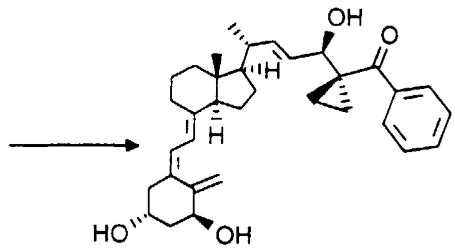

10b

4a, 4b

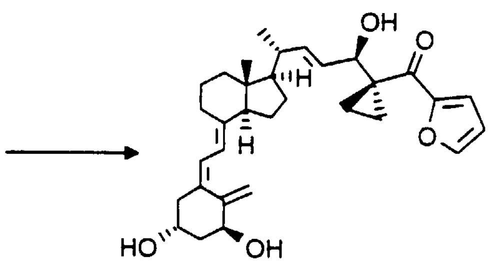

11b

4a, 4b

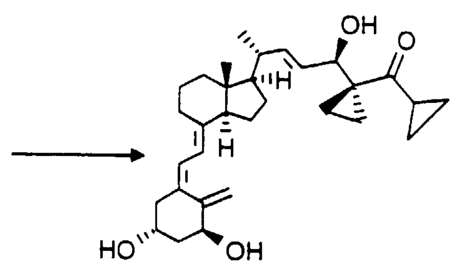

12b

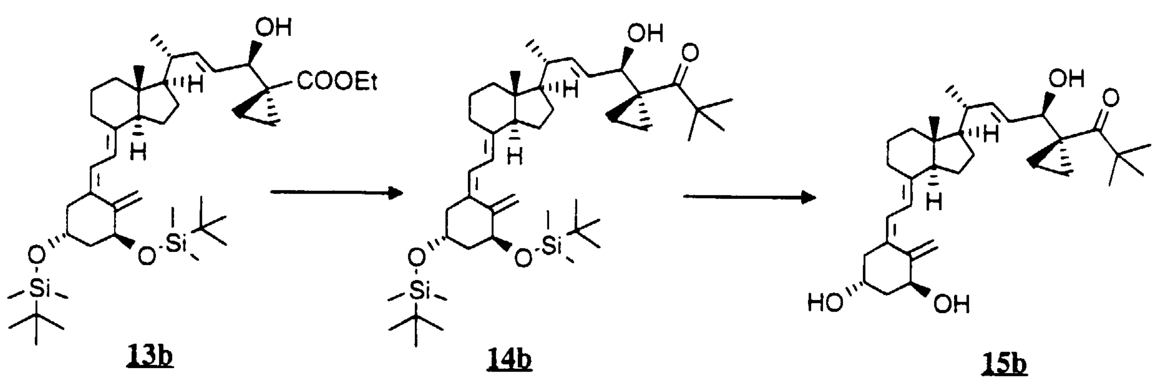

13b 14b 15b

**4a**, **4b**

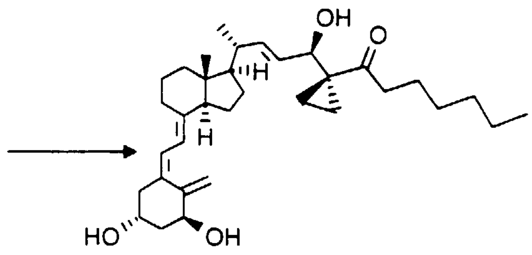

**16b**

**13b**

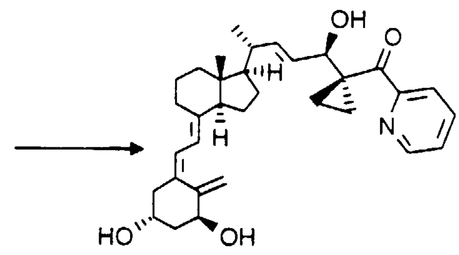

**17b**

**4a**, **4b**

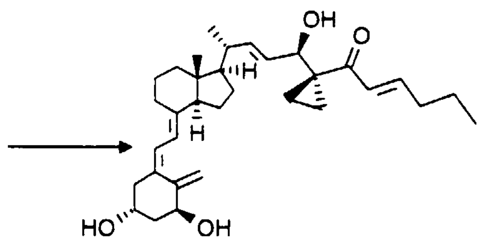

**18b**

**4a**, **4b**

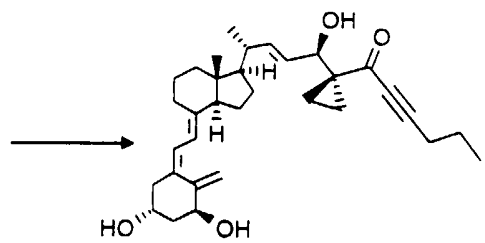

**19b**

**13b**

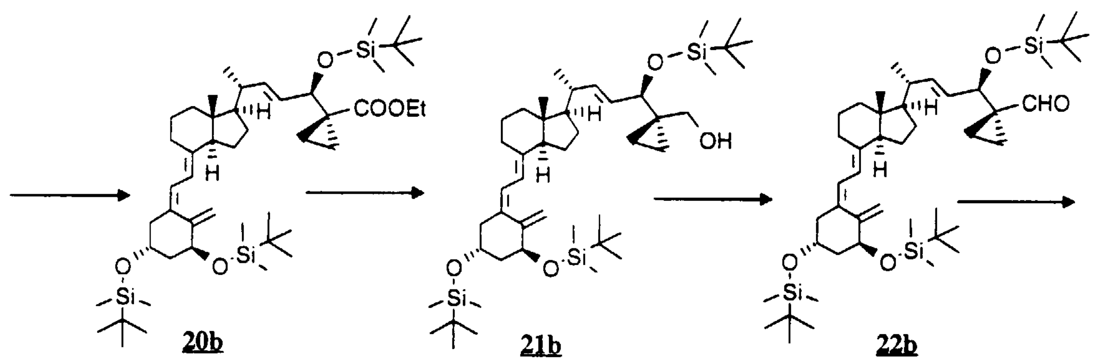

**20b** **21b** **22b**

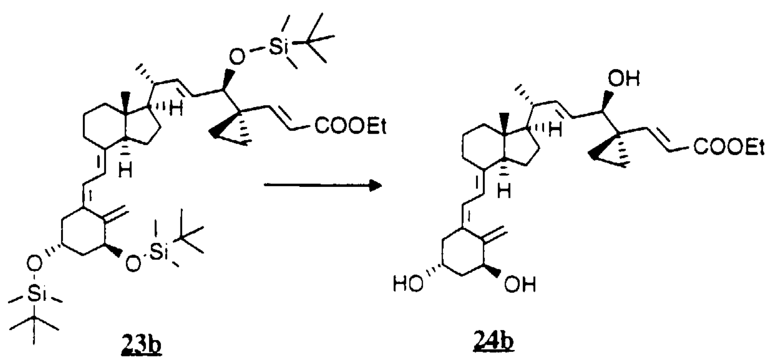

**23b** **24b**

13a

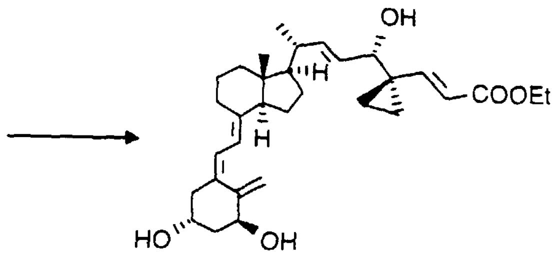

24a

22b

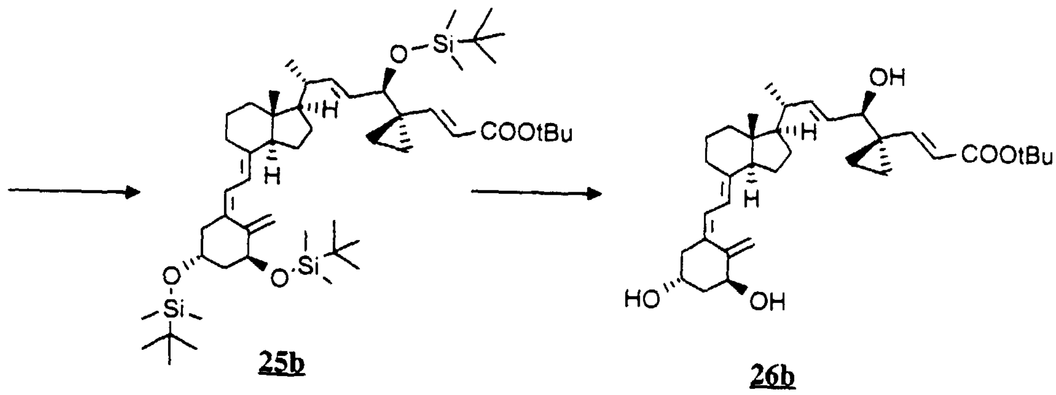

25b

26b

22a

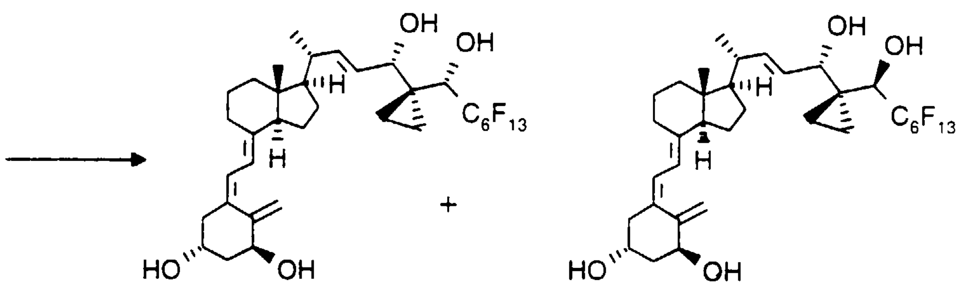

27aα

27aβ

22b

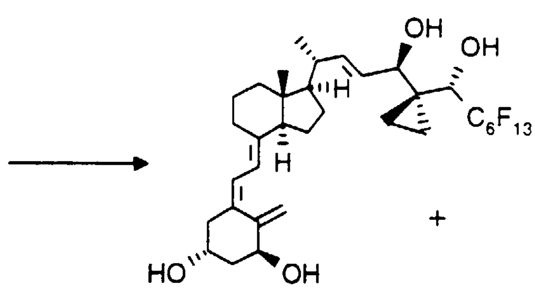

27bα

27bβ

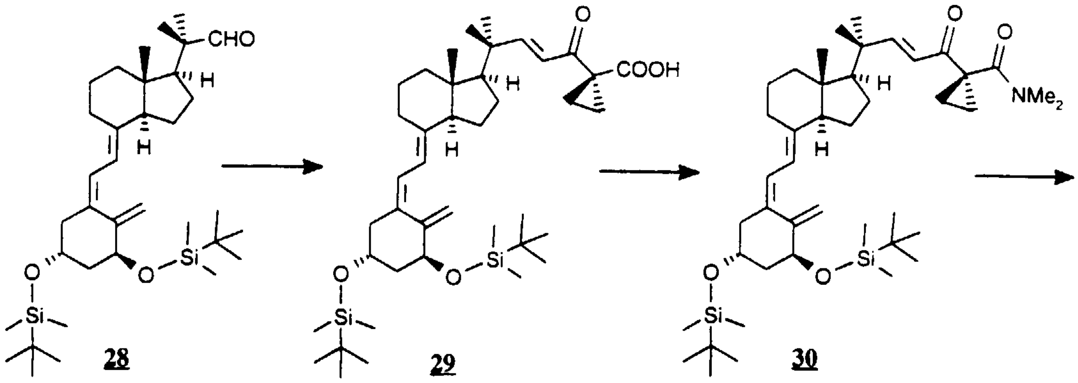

28

29

30

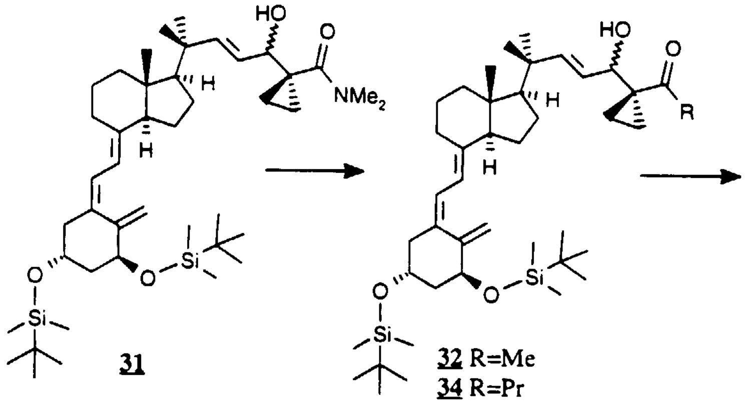

31

32 R=Me
34 R=Pr
36 R=Bu
38 R=Pent
40 R=Hex
42 R=Hept

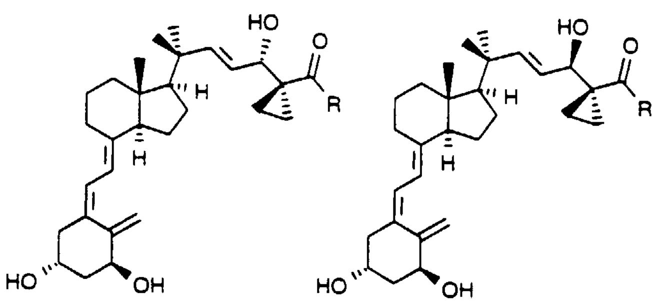

33a R=Me
35a R=Pr
37a R=Bu
39a R=Pent
41a R=Hex
43a R=Hept

33b R=Me
35b R=Pr
37b R=Bu
39b R=Pent
41b R=Hex
43b R=Hept

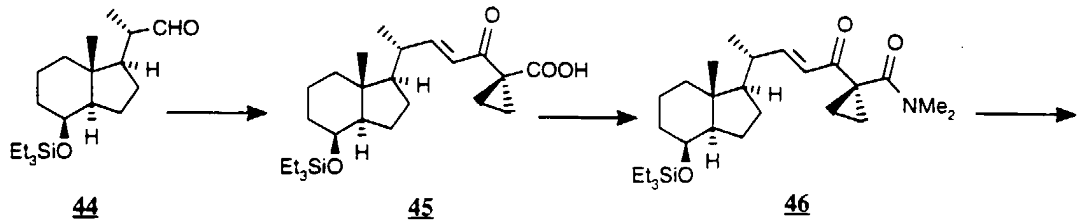

44 45 46

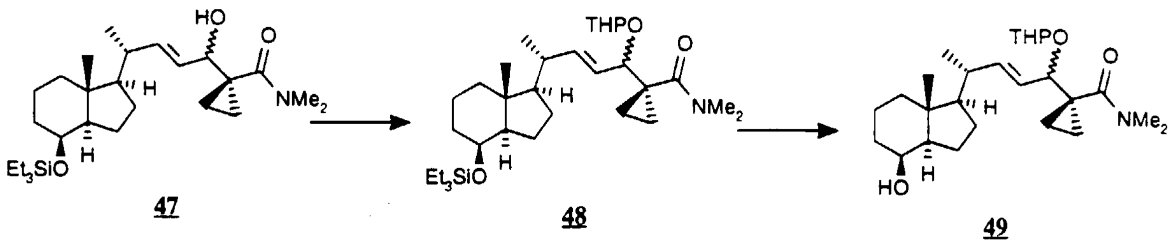

47 48 49

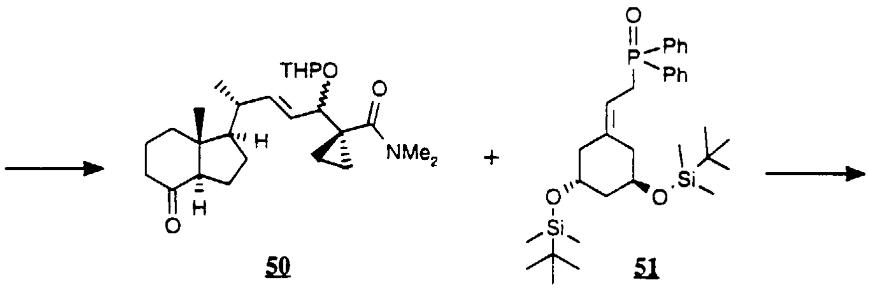

50 51

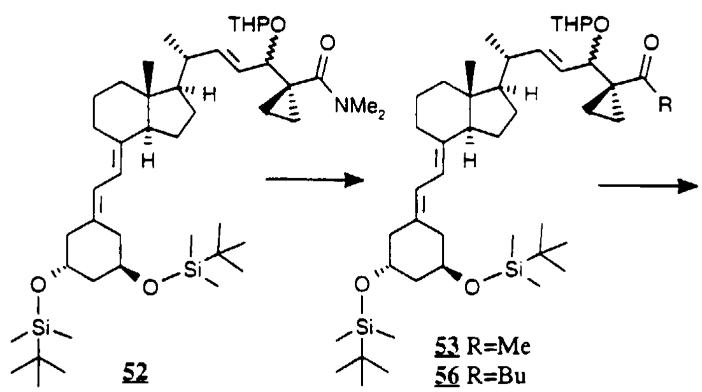

52

53 R=Me
56 R=Bu
59 R=Pent
62 R=Hex
65 R=Hept
68 R=Oct

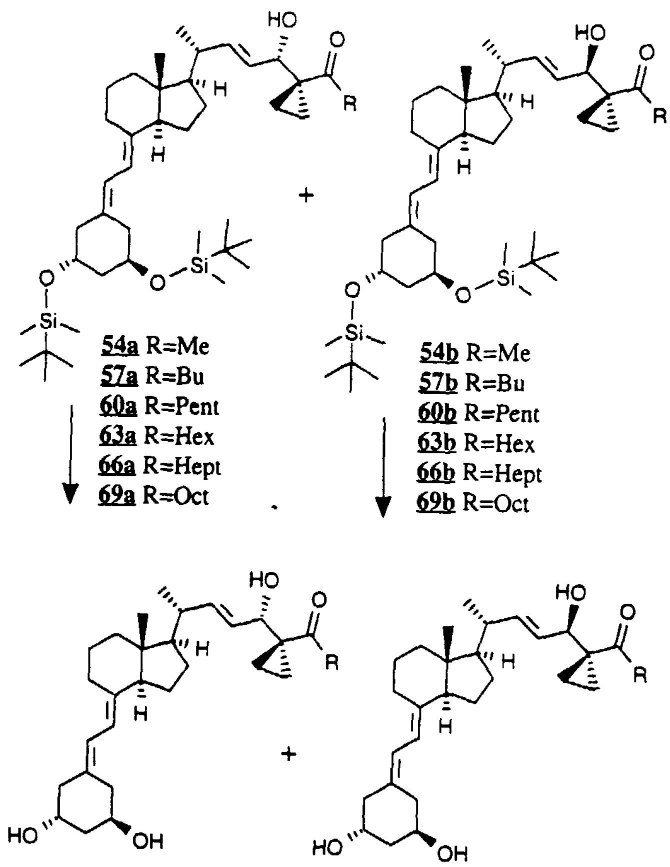

54a R=Me
57a R=Bu
60a R=Pent
63a R=Hex
66a R=Hept
69a R=Oct

54b R=Me
57b R=Bu
60b R=Pent
63b R=Hex
66b R=Hept
69b R=Oct

55a R=Me
58a R=Bu
61a R=Pent
64a R=Hex
67a R=Hept
70a R=Oct

55b R=Me
58b R=Bu
61b R=Pent
64b R=Hex
67b R=Hept
70b R=Oct

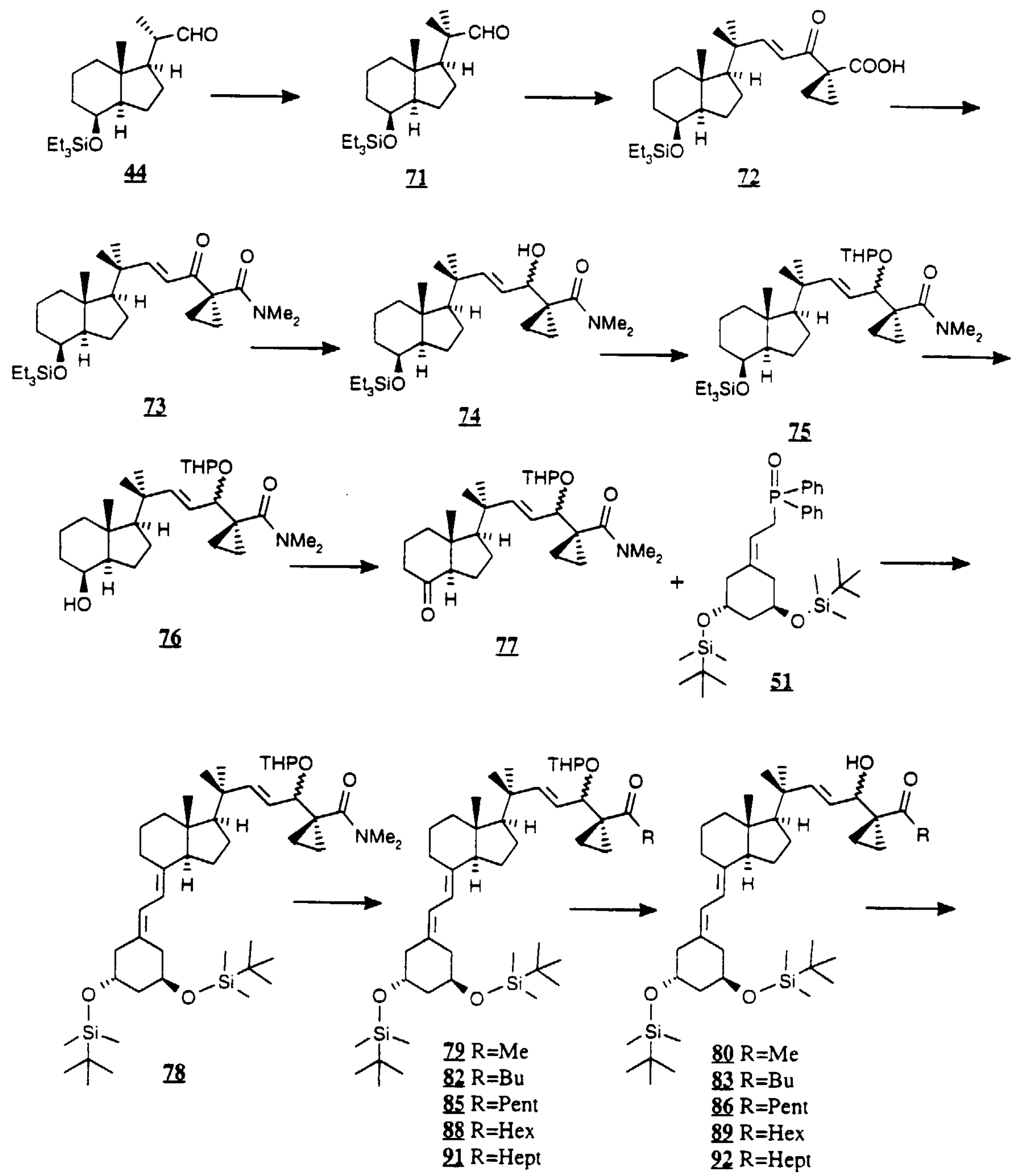

44

71

72

73

74

75

76

77

51

78

79 R=Me
82 R=Bu
85 R=Pent
88 R=Hex
91 R=Hept

80 R=Me
83 R=Bu
86 R=Pent
89 R=Hex
92 R=Hept

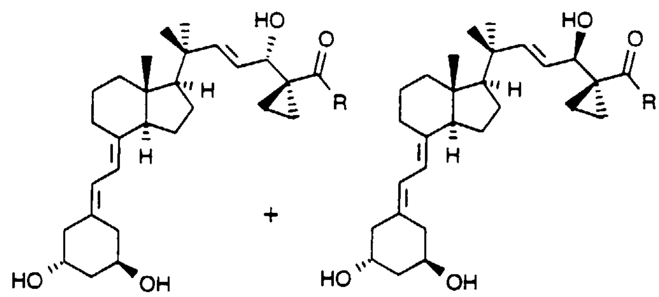

81a R=Me
84a R=Bu
87a R=Pent
90a R=Hex
93a R=Hept

81b R=Me
84b R=Bu
87b R=Pent
90b R=Hex
93b R=Hept

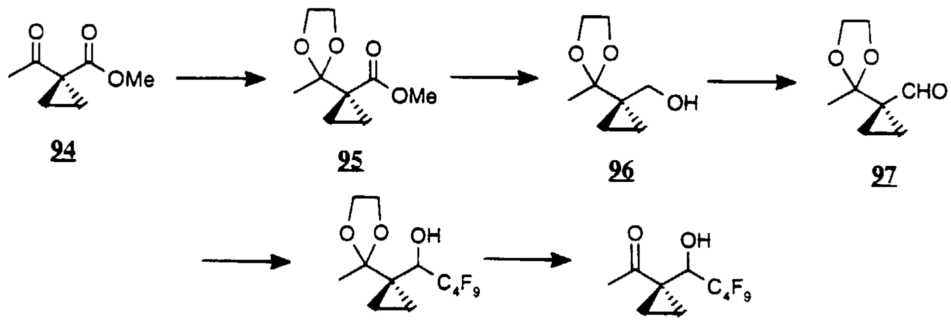

94 95 96 97

98 99

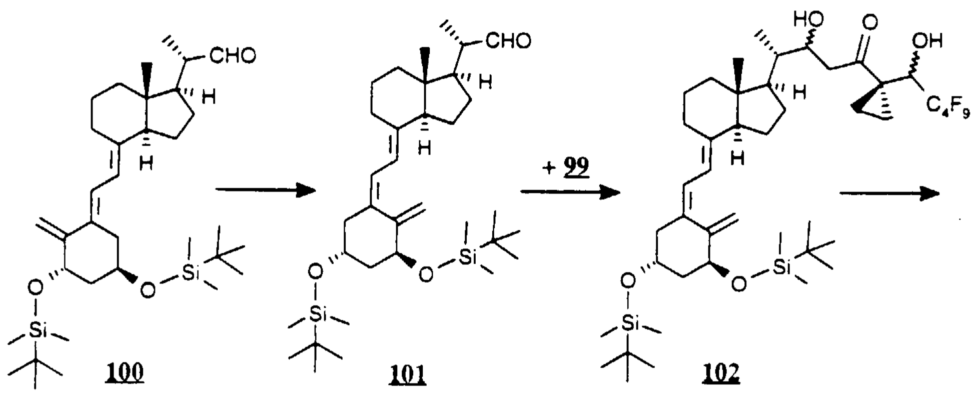


100 101 102

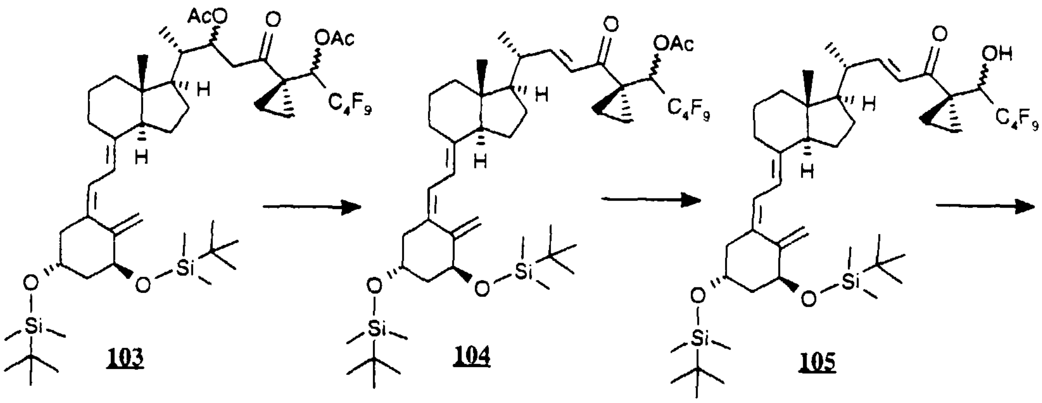

103

104

105

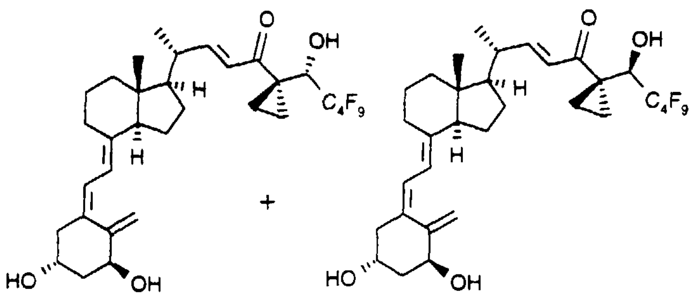

106a

106b

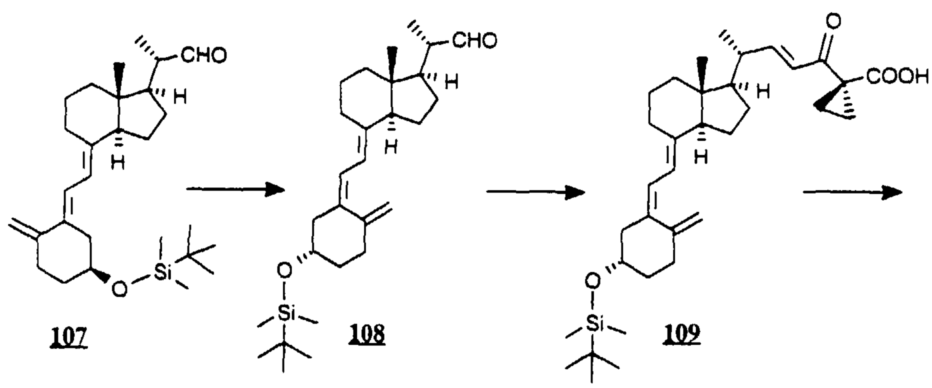

107

108

109

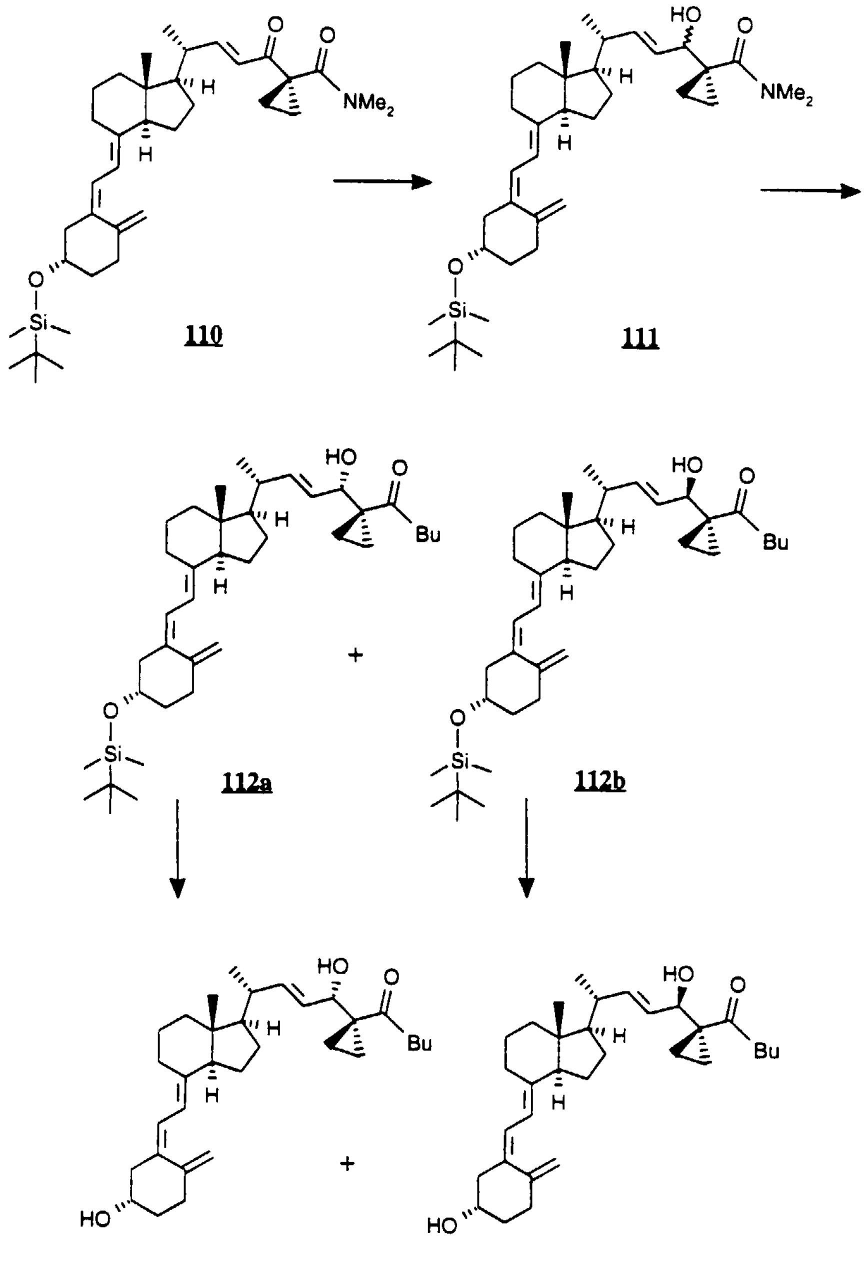

110

111

112a + 112b

113a + 113b

25b

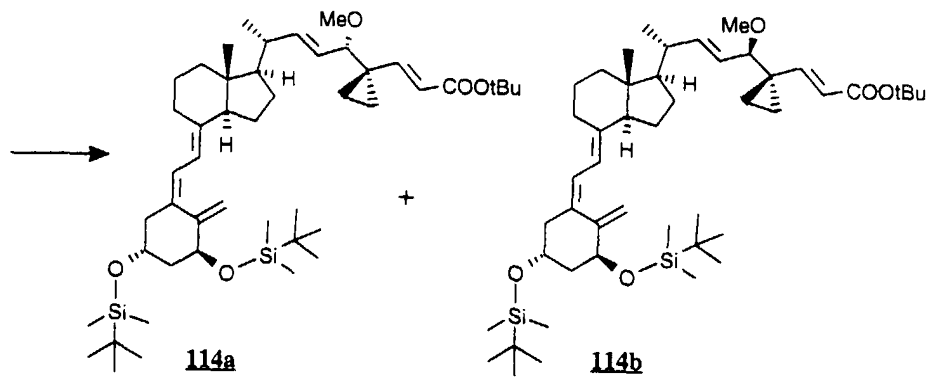

114a

114b

21b

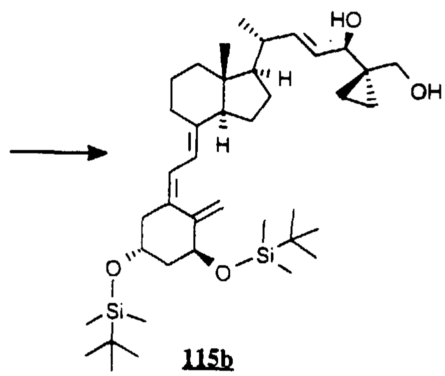

115b

22b

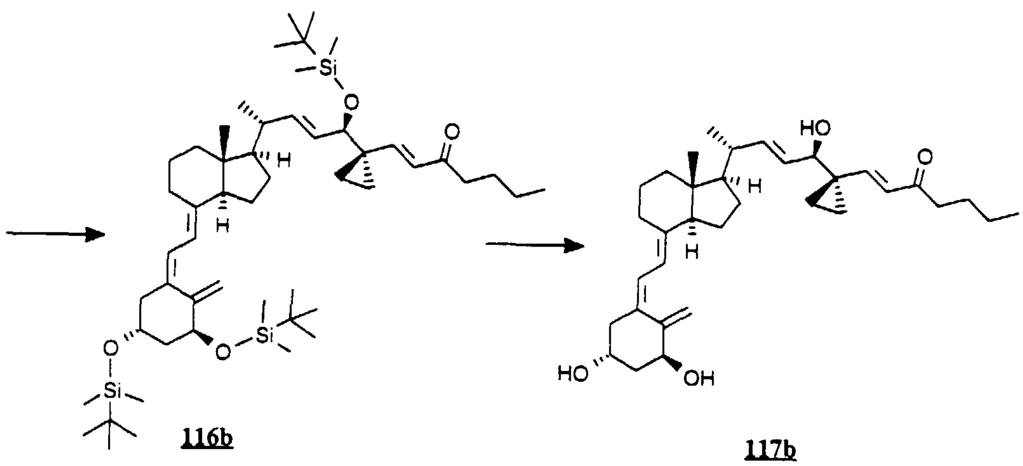

116b

117b

7b

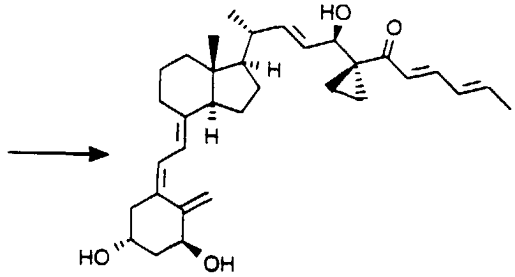

118b

**Patentansprüche**

1. Vitamin D-Derivate mit Substituenten an C-25 der allgemeinen Formel **I**,

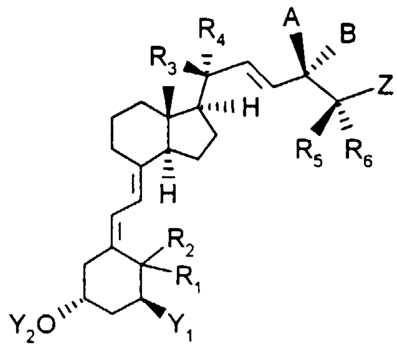

**I**

worin

$Y_1$ ein Wasserstoffatom, eine Hydroxylgruppe, eine Alkanoyloxygruppe mit 1 bis 12 C-Atomen oder eine Aroyloxygruppe,
$Y_2$ ein Wasserstoffatom oder eine Alkanoylgruppe mit 1 bis 12 C-Atomen oder eine Aroylgruppe,
$R_1$ und $R_2$ je ein Wasserstoffatom oder gemeinsam eine exocyclische Methylengruppe,
$R_3$ und $R_4$ unabhängig voneinander ein Wasserstoffatom, ein Chlor- oder Fluoratom, eine Alkylgruppe mit 1 bis 4 Kohlenstoffatomen, gemeinsam eine Methylengruppe oder gemeinsam mit dem quartären Kohlenstoffatom 20 einen 3-7-gliedrigen, gesättigten oder ungesättigten carbocyclischen Ring,
A und B gemeinsam eine Ketogruppe oder A eine Gruppe OR' und B ein Wasserstoffatom oder B eine Gruppe OR' und A ein Wasserstoffatom, wobei R' ein Wasserstoffatom oder eine gerad- oder verzweigtkettige, gesättigte Alkanoylgruppe mit bis zu 9 Kohlenstoffatomen oder eine Aroylgruppe ist,
$R_5$ und $R_6$ gleichzeitig je ein Wasserstoffatom, ein Chlor- oder Fluoratom, eine Trifluormethylgruppe, einen gerad- oder verzweigtkettigen, gesättigten oder ungesättigten Kohlenwasserstoffrest mit bis zu 4 Kohlenstoffatomen oder $R_5$ und $R_6$ gemeinsam mit dem Kohlenstoffatom 25 einen 3-7-gliedrigen, gesättigten oder ungesättigten carbocyclischen Ring und Z einen gerad- oder verzweigtkettigen, gesättigten oder ungesättigten Kohlenwasserstoffrest mit bis zu 12 Kohlenstoffatomen, der auch carbo- oder heterocyclische Struktur oder Partialstruktur haben kann und an beliebigen Positionen Ketogruppen, Hydroxygruppen, die ihrerseits verethert oder verestert sein können, Aminogruppen, Halogenatome oder Carbonsäureester oder -amideinheiten aufweisen kann und durch eine Carbonylgruppe, eine Hydroxymethylengruppe oder eine Ethendiyl-Einheit mit dem Kohlenstoffatom 25 verknüpft ist.

2. Vitamin D-Derivate nach Anspruch **I**,

(5*Z*,7*E*,22*E*)-(1*S*,3*R*,24*R*)-25-(1-Oxopentyl)-26,27-cyclo-9,10-secocholesta-5,7,10(19),22-tetraen-1,3,24-triol
(5*Z*,7*E*,22*E*)-(1*S*,3*R*,24*S*)-25-(1-Oxopentyl)-26,27-cyclo-9,10-secocholesta-5,7,10(19),22-tetraen-1,3,24-triol
(5*Z*,7*E*,22*E*)-(1*S*,3*R*,24*R*)-25-Acetyl-26,27-cyclo-9,10-secocholesta-5,7,10(19),22-tetraen-1,3,24-triol
(5*Z*,7*E*,22*E*)-(1*S*,3*R*,24*S*)-25-Acetyl-26,27-cyclo-9,10-secocholesta-5,7,10(19),22-tetraen-1,3,24-triol
(5*Z*,7*E*,22*E*)-(1*S*,3*R*,24*R*)-25-(1-Oxopropyl)-26,27-cyclo-9,10-secocholesta-5,7,10(19),22-tetraen-1,3,24-triol
(5*Z*,7*E*,22*E*)-(1*S*,3*R*,24*S*)-25-(1-Oxopropyl)-26,27-cyclo-9,10-secocholesta-5,7,10(19),22-tetraen-1,3,24-triol
(5*Z*,7*E*,22*E*)-(1*S*,3*R*,24*R*)-25-(1-Oxobutyl)-26,27-cyclo-9,10-secocholesta-5,7,10(19),22-tetraen-1,3,24-triol
(5*Z*,7*E*,22*E*)-(1*S*,3*R*,24*S*)-25-(1-Oxobutyl)-26,27-cyclo-9,10-secocholesta-5,7,10(19),22-tetraen-1,3,24-triol
(5*Z*,7*E*,22*E*)-(1*S*,3*R*,24*R*)-25-(1-Oxohexyl)-26,27-cyclo-9,10-secocholesta-5,7,10(19),22-tetraen-1,3,24-triol
(5*Z*,7*E*,22*E*)-(1*S*,3*R*,24*S*)-25-(1-Oxohexyl)-26,27-cyclo-9,10-secocholesta-5,7,10(19),22-tetraen-1,3,24-triol
(5*Z*,7*E*,22*E*)-(1*S*,3*R*,24*R*)-25-(1-Oxoheptyl)-26,27-cyclo-9,10-secocholesta-5,7,10(19),22-tetraen-1,3,24-triol

(5*Z*,7*E*,22*E*)-(1*S*,3*R*,24*S*)-25-(1-Oxoheptyl)-26,27-cyclo-9,10-secocholesta-5,7,10(19),22-tetraen-1,3,24-triol

(5*Z*,7*E*,22*E*)-(1*S*,3*R*,24*R*)-25-(1-Oxooctyl)-26,27-cyclo-9,10-secocholesta-5,7,10(19),22-tetraen-1,3,24-triol

(5*Z*,7*E*,22*E*)-(1*S*,3*R*,24*S*)-25-(1-Oxooctyl)-26,27-cyclo-9,10-secocholesta-5,7,10(19),22-tetraen-1,3,24-triol

(5*Z*,7*E*,22*E*)-(1*S*,3*R*,24*R*)-25-(1-Oxononyl)-26,27-cyclo-9,10-secocholesta-5,7,10(19),22-tetraen-1,3,24-triol

(5*Z*,7*E*,22*E*)-(1*S*,3*R*,24*S*)-25-(1-Oxononyl)-26,27-cyclo-9,10-secocholesta-5,7,10(19),22-tetraen-1,3,24-triol

(5*Z*,7*E*,22*E*)-(1*S*,3*R*,24*R*)-25-Benzoyl-26,27-cyclo-9,10-secocholesta-5,7,10(19),22-tetraen-1,3,24-triol

(5*Z*,7*E*,22*E*)-(1*S*,3*R*,24*S*)-25-Benzoyl-26,27-cyclo-9,10-secocholesta-5,7,10(19),22-tetraen-1,3,24-triol

(5*Z*,7*E*,22*E*)-(1*S*,3*R*,24*R*)-25-(2-Furanylcarbonyl)-26,27-cyclo-9,10-secocholesta-5,7,10(19),22-tetraen-1,3,24-triol

(5*Z*,7*E*,22*E*)-(1*S*,3*R*,24*S*)-25-(2-Furanylcarbonyl)-26,27-cyclo-9,10-secocholesta-5,7,10(19),22-tetraen-1,3,24-triol

(5*Z*,7*E*,22*E*)-(1*S*,3*R*,24*R*)-25-(2,2-Dimethyl-1-oxopropyl)-26,27-cyclo-9,10-secocholesta-5,7,10(19),22-tetraen-1,3,24-triol

(5*Z*,7*E*,22*E*)-(1*S*,3*R*,24*S*)-25-(2,2-Dimethyl-1-oxopropyl)-26,27-cyclo-9,10-secocholesta-5,7,10(19),22-tetraen-1,3,24-triol

(5*Z*,7*E*,22*E*)-(1*S*,3*R*,24*R*)-25-(2-Pyridinylcarbonyl)-26,27-cyclo-9,10-secocholesta-5,7,10(19),22-tetraen-1,3,24-triol

(5*Z*,7*E*,22*E*)-(1*S*,3*R*,24*S*)-25-(2-Pyridinylcarbonyl)-26,27-cyclo-9,10-secocholesta-5,7,10(19),22-tetraen-1,3,24-triol

[5*Z*,7*E*,22*E*,25(*E*)]-(1*S*,3*R*,24*R*)-25-(1-Oxo-2-hexenyl)-26,27-cyclo-9,10-secocholesta-5,7,10(19),22-tetraen-1,3,24-triol

[5*Z*,7*E*,22*E*,25(*E*)]-(1*S*,3*R*,24*S*)-25-(1-Oxo-2-hexenyl)-26,27-cyclo-9,10-secocholesta-5,7,10(19),22-tetraen-1,3,24-triol

(5*Z*,7*E*,22*E*)-(1*S*,3*R*,24*R*)-25-(1-Oxo-2-hexinyl)-26,27-cyclo-9,10-secocholesta-5,7,10(19),22-tetraen-1,3,24-triol

(5*Z*,7*E*,22*E*)-(1*S*,3*R*,24*S*)-25-(1-Oxo-2-hexinyl)-26,27-cyclo-9,10-secocholesta-5,7,10(19),22-tetraen-1,3,24-triol

(5*Z*,7*E*,22*E*)-(1*S*,3*R*,24*R*)-25-(Cyclopropylcarbonyl)-26,27-cyclo-9,10-secocholesta-5,7,10(19),22-tetraen-1,3,24-triol

(5*Z*,7*E*,22*E*)-(1*S*,3*R*,24*S*)-25-(Cyclopropylcarbonyl)-26,27-cyclo-9,10-secocholesta-5,7,10(19),22-tetraen-1,3,24-triol

[5*Z*,7*E*,22*E*,25(*E*)]-(1*S*,3*R*,24*R*)-25-(3-Ethoxy-3-oxo-1-propenyl)-26,27-cyclo-9,10-secocholesta-5,7,10(19),22-tetraen-1,3,24-triol

[5*Z*,7*E*,22*E*,25(*E*)]-(1*S*,3*R*,24*S*)-25-(3-Ethoxy-3-oxo-1-propenyl)-26,27-cyclo-9,10-secocholesta-5,7,10(19),22-tetraen-1,3,24-triol

[5*Z*,7*E*,22*E*,25(*E*)]-(1*S*,3*R*,24*R*)-25-[3-(1,1-Dimethylethoxy)-3-oxo-1-propenyl]-26,27-cyclo-9,10-secocholesta-5,7,10(19),22-tetraen-1,3,24-triol

[5*Z*,7*E*,22*E*,25(*E*)]-(1*S*,3*R*,24*S*)-25-[3-(1,1-Dimethylethoxy)-3-oxo-1-propenyl]-26,27-cyclo-9,10-secocholesta-5,7,10(19),22-tetraen-1,3,24-triol

[5*Z*,7*E*,22*E*,25(*E*)]-(1*S*,3*R*,24*R*)-25-(3-Propoxy-3-oxo-1-propenyl)-26,27-cyclo-9,10-secocholesta-5,7,10(19),22-tetraen-1,3,24-triol

[5*Z*,7*E*,22*E*,25(*E*)]-(1*S*,3*R*,24*S*)-25-(3-Propoxy-3-oxo-1-propenyl)-26,27-cyclo-9,10-secocholesta-5,7,10(19),22-tetraen-1,3,24-triol

(5*Z*,7*E*,22*E*,25(*E*)]-(1*S*,3*R*,24*R*)-25-(3-Butoxy-3-oxo-1-propenyl)-26,27-cyclo-9,10-secocholesta-5,7,10(19),22-tetraen-1,3,24-triol

[5*Z*,7*E*,22*E*,25(*E*)]-(1*S*,3*R*,24*S*)-25-(3-Butoxy-3-oxo-1-propenyl)-26,27-cyclo-9,10-secocholesta-5,7,10(19),22-tetraen-1,3,24-triol

(5*Z*,7*E*,22*E*)-[1*S*,3*R*,24*S*,25(*S*)]-25-(1-Hydroxy-2,2,3,3,4,4,5,5,5-nonafluorpentyl)-26,27-cyclo-9,10-secocholesta-5,7,10(19),22-tetraen-1,3,24-triol

(5*Z*,7*E*,22*E*)-[1*S*,3*R*,24*S*,25(*R*)]-25-(1-Hydroxy-2,2,3,3,4,4,5,5,5-nonafluorpentyl)-26,27-cyclo-9,10-secocholesta-5,7,10(19),22-tetraen-1,3,24-triol

(5*Z*,7*E*,22*E*)-[1*S*,3*R*,24*R*,25(*S*)]-25-(1-Hydroxy-2,2,3,3,4,4,5,5,5-nonafluorpentyl)-26,27-cyclo-9,10-secocholesta-5,7,10(19),22-tetraen-1,3,24-triol

(5*Z*,7*E*,22*E*)-[1*S*,3*R*,24*R*,25(*R*)]-25-(1-Hydroxy-2,2,3,3,4,4,5,5,5-nonafluorpentyl)-26,27-cyclo-9,10-secocholesta-5,7,10(19),22-tetraen-1,3,24-triol

(5*Z*,7*E*,22*E*)-[1*S*,3*R*,24*S*,25(*S*)]-25-(1-Hydroxy-2,2,3,3,4,4,5,5,6,6,7,7,7-tridecafluorheptyl)-26,27-cyclo-9,10-secocholesta-5,7,10(19),22-tetraen-1,3,24-triol
(5*Z*,7*E*,22*E*)-[1*S*,3*R*,24*S*,25(*R*)]-25-(1-Hydroxy-2,2,3,3,4,4,5,5,6,6,7,7,7-tridecafluorheptyl)-26,27-cyclo-9,10-secocholesta-5,7,10(19),22-tetraen-1,3,24-triol
(5*Z*,7*E*,22*E*)-[1*S*,3*R*,24*R*,25(*S*)]-25-(1-Hydroxy-2,2,3,3,4,4,5,5,6,6,7,7,7-tridecafluorheptyl)-26,27-cyclo-9,10-secocholesta-5,7,10(19),22-tetraen-1,3,24-triol
(5*Z*,7*E*,22*E*)-[1*S*,3*R*,24*R*,25(*R*)]-25-(1-Hydroxy-2,2,3,3,4,4,5,5,6,6,7,7,7-tridecafluorheptyl)-26,27-cyclo-9,10-secocholesta-5,7,10(19),22-tetraen-1,3,24-triol
(5*Z*,7*E*,22*E*)-(1*S*,3*R*,24*R*)-25-(Trifluoracetyl)-26,27-cyclo-9,10-secocholesta-5,7,10(19),22-tetraen-1,3,24-triol
(5*Z*,7*E*,22*E*)-(1*S*,3*R*,24*S*)-25-(Trifluoracetyl)-26,27-cyclo-9,10-secocholesta-5,7,10(19),22-tetraen-1,3,24-triol
(5*Z*,7*E*,22*E*)-(1*S*,3*R*,24*R*)-25-(Perfluorethylcarbonyl)-26,27-cyclo-9,10-secocholesta-5,7,10(19),22-tetraen-1,3,24-triol
(5*Z*,7*E*,22*E*)-(1*S*,3*R*,24*S*)-25-(Perfluorethylcarbonyl)-26,27-cyclo-9,10-secocholesta-5,7,10(19),22-tetraen-1,3,24-triol
(5*Z*,7*E*,22*E*)-(1*S*,3*R*,24*R*)-25-(Perfluorpropylcarbonyl)-26,27-cyclo-9,10-secocholesta-5,7,10(19),22-tetraen-1,3,24-triol
(5*Z*,7*E*,22*E*)-(1*S*,3*R*,24*S*)-25-(Perfluorpropylcarbonyl)-26,27-cyclo-9,10-secocholesta-5,7,10(19),22-tetraen-1,3,24-triol
(5*Z*,7*E*,22*E*)-(1*S*,3*R*,24*R*)-25-(Perfluorbutylcarbonyl)-26,27-cyclo-9,10-secocholesta-5,7,10(19),22-tetraen-1,3,24-triol
(5*Z*,7*E*,22*E*)-(1*S*,3*R*,24*S*)-25-(Perfluorbutylcarbonyl)-26,27-cyclo-9,10-secocholesta-5,7,10(19),22-tetraen-1,3,24-triol
(5*Z*,7*E*,22*E*)-(1*S*,3*R*,24*R*)-25-(Perfluorpentylcarbonyl)-26,27-cyclo-9,10-secocholesta-5,7,10(19),22-tetraen-1,3,24-triol
(5*Z*,7*E*,22*E*)-(1*S*,3*R*,24*S*)-25-(Perfluorpentylcarbonyl)-26,27-cyclo-9,10-secocholesta-5,7,10(19),22-tetraen-1,3,24-triol
(5*Z*,7*E*,22*E*)-(1*S*-3*R*,24*R*)-25-(Perfluorhexylcarbonyl)-26,27-cyclo-9,10-secocholesta-5,7,10(19),22-tetraen-1,3,24-triol
(5*Z*,7*E*,22*E*)-(1*S*,3*R*,24*S*)-25-(Perfluorhexylcarbonyl)-26,27-cyclo-9,10-secocholesta-5,7,10(19),22-tetraen-1,3,24-triol
(5*Z*,7*E*,22*E*)-(1*S*,3*R*,24*R*)-25-Acetyl-20-methyl-26,27-cyclo-9,10-secocholesta-5,7,10(19),22-tetraen-1,3,24-triol
(5*Z*,7*E*,22*E*)-(1*S*,3*R*,24*S*)-25-Acetyl-20-methyl-26,27-cyclo-9,10-secocholesta-5,7,10(19),22-tetraen-1,3,24-triol
(5*Z*,7*E*,22*E*)-(1*S*,3*R*,24*R*)-20-Methyl-25-(1-oxopropyl)-26,27-cyclo-9,10-secocholesta-5,7,10(19),22-tetraen-1,3,24-triol
(5*Z*,7*E*,22*E*)-(1*S*,3*R*,24*S*)-20-Methyl-25-(1-oxopropyl)-26,27-cyclo-9,10-secocholesta-5,7,10(19),22-tetraen-1,3,24-triol
(5*Z*,7*E*,22*E*)-(1*S*,3*R*,24*R*)-20-Methyl-25-(1-oxobutyl)-26,27-cyclo-9,10-secocholesta-5,7,10(19),22-tetraen-1,3,24-triol
(5*Z*,7*E*,22*E*)-(1*S*,3*R*,24*S*)-20-Methyl-25-(1-oxobutyl)-26,27-cyclo-9,10-secocholesta-5,7,10(19),22-tetraen-1,3,24-triol
(5*Z*,7*E*,22*E*)-(1*S*,3*R*,24*R*)-20-Methyl-25-(1-oxopentyl)-26,27-cyclo-9,10-secocholesta-5,7,10(19),22-tetraen-1,3,24-triol
(5*Z*,7*E*,22*E*)-(1*S*,3*R*,24*S*)-20-Methyl-25-(1-oxopentyl)-26,27-cyclo-9,10-secocholesta-5,7,10(19),22-tetraen-1,3,24-triol
(5*Z*,7*E*,22*E*)-(1*S*,3*R*,24*R*)-20-Methyl-25-(1-oxohexyl)-26,27-cyclo-9,10-secocholesta-5,7,10(19),22-tetraen-1,3,24-triol
(5*Z*,7*E*,22*E*)-(1*S*,3*R*,24*S*)-20-Methyl-25-(1-oxohexyl)-26,27-cyclo-9,10-secocholesta-5,7,10(19),22-tetraen-1,3,24-triol
(5*Z*,7*E*,22*E*)-(1*S*,3*R*,24*R*)-20-Methyl-25-(1-oxoheptyl)-26,27-cyclo-9,10-secocholesta-5,7,10(19),22-tetraen-1,3,24-triol
(5*Z*,7*E*,22*E*)-(1*S*,3*R*,24*S*)-20-Methyl-25-(1-oxoheptyl)-26,27-cyclo-9,10-secocholesta-5,7,10(19),22-tetraen-1,3,24-triol
(5*Z*,7*E*,22*E*)-(1*S*,3*R*,24*R*)-20-Methyl-25-(1-oxooctyl)-26,27-cyclo-9,10-secocholesta-5,7,10(19),22-tetraen-1,3,24-triol
(5*Z*,7*E*,22*E*)-(1*S*,3*R*,24*S*)-20-Methyl-25-(1-oxooctyl)-26,27-cyclo-9,10-secocholesta-5,7,10(19),22-tetraen-1,3,24-triol
(5*Z*,7*E*,22*E*)-(1*S*,3*R*,24*R*)-20-Methyl-25-(1-oxononyl)-26,27-cyclo-9,10-secocholesta-5,7,10(19),22-tetraen-1,3,24-triol
(5,*Z*,7*E*,22*E*)-(1*S*,3*R*,24*S*)-20-Methyl-25-(1-oxononyl)-26,27-cyclo-9,10-secocholesta-5,7,10(19),22-tetraen-1,3,24-triol
(7*E*,22*E*)-(1*R*,3*R*,24*R*)-25-Acetyl-26,27-cyclo-19-nor-9,10-secocholesta-5,7,22-trien-1,3,24-triol
(7*E*,22*E*)-(1*R*,3*R*,24*S*)-25-Acetyl-26,27-cyclo-19-nor-9,10-secocholesta-5,7,22-trien-1,3,24-triol
(7*E*,22*E*)-(1*R*,3*R*,24*R*)-25-(1-Oxopropyl)-26,27-cyclo-19-nor-9,10-secocholesta-5,7,22-trien-1,3,24-triol
(7*E*,22*E*)-(1*R*,3*R*,24*S*)-25-(1-Oxopropyl)-26,27-cyclo-19-nor-9,10-secocholesta-5,7,22-trien-1,3,24-triol
(7*E*,22*E*)-(1*R*,3*R*,24*R*)-25-(1-Oxobutyl)-26,27-cyclo-19-nor-9,10-secocholesta-5,7,22-trien-1,3,24-triol
(7*E*,22*E*)-(1*R*,3*R*,24*S*)-25-(1-Oxobutyl)-26,27-cyclo-19-nor-9,10-secocholesta-5,7,22-trien-1,3,24-triol
(7*E*,22*E*)-(1*R*,3*R*,24*R*)-25-(1-Oxopentyl)-26,27-cyclo-19-nor-9,10-secocholesta-5,7,22-trien-1,3,24-triol
(7*E*,22*E*)-(1*R*,3*R*,24*S*)-25-(1-Oxopentyl)-26,27-cyclo-19-nor-9,10-secocholesta-5,7,22-trien-1,3,24-triol
(7*E*,22*E*)-(1*R*,3*R*,24*R*)-25-(1-Oxohexyl)-26,27-cyclo-19-nor-9,10-secocholesta-5,7,22-trien-1,3,24-triol
(7*E*,22*E*)-(1*R*,3*R*,24*S*)-25-(1-Oxohexyl)-26,27-cyclo-19-nor-9,10-secocholesta-5,7,22-trien-1,3,24-triol
(7*E*,22*E*)-(1*R*,3*R*,24*R*)-25-(1-Oxoheptyl)-26,27-cyclo-19-nor-9,10-secocholesta-5,7,22-trien-1,3,24-triol
(7*E*,22*E*)-(1*R*,3*R*,24*S*)-25-(1-Oxoheptyl)-26,27-cyclo-19-nor-9,10-secocholesta-5,7,22-trien-1,3,24-triol
(7*E*,22*E*)-(1*R*,3*R*,24*R*)-25-(1-Oxooctyl)-26,27-cyclo-19-nor-9,10-secocholesta-5,7,22-trien-1,3,24-triol
(7*E*,22*E*)-(1*R*,3*R*,24*S*)-25-(1-Oxooctyl)-26,27-cyclo-19-nor-9,10-secocholesta-5,7,22-trien-1,3,24-triol
(7*E*,22*E*)-(1*R*,3*R*,24*R*)-25-(1-Oxononyl)-26,27-cyclo-19-nor-9,10-secocholesta-5,7,22-trien-1,3,24-triol
(7*E*,22*E*)-(1*R*,3*R*,24*S*)-25-(1-Oxononyl)-26,27-cyclo-19-nor-9,10-secocholesta-5,7,22-trien-1,3,24-triol
(7*E*,22*E*)-(1*R*,3*R*,24*R*)-25-Acetyl-20-methyl-26,27-cyclo-19-nor-9,10-secocholesta-5,7,22-trien-1,3,24-triol
(7*E*,22*E*)-(1*R*,3*R*,24*S*)-25-Acetyl-20-methyl-26,27-cyclo-19-nor-9,10-secocholesta-5,7,22-trien-1,3,24-triol
(7*E*,22*E*)-(1*R*,3*R*,24*R*)-20-Methyl-25-(1-oxopropyl)-26,27-cyclo-19-nor-9,10-secocholesta-5,7,22-trien-1,3,24-triol
(7*E*,22*E*)-(1*R*,3*R*,24*S*)-20-Methyl-25-(1-oxopropyl)-26,27-cyclo-19-nor-9,10-secocholesta-5,7,22-trien-1,3,24-triol
(7*E*,22*E*)-(1*R*,3*R*,24*R*)-20-Methyl-25-(1-oxobutyl)-26,27-cyclo-19-nor-9,10-secocholesta-5,7,22-trien-1,3,24-triol
(7*E*,22*E*)-(1*R*,3*R*,24*S*)-20-Methyl-25-(1-oxobutyl)-26,27-cyclo-19-nor-9,10-secocholesta-5,7,22-trien-1,3,24-triol
(7*E*,22*E*)-(1*R*,3*R*,24*R*)-20-Methyl-25-(1-oxopentyl)-26,27-cyclo-19-nor-9,10-secocholesta-5,7,22-trien-1,3,24-triol
(7*E*,22*E*)-(1*R*,3*R*,24*S*)-20-Methyl-25-(1-oxopentyl)-26,27-cyclo-19-nor-9,10-secocholesta-5,7,22-trien-1,3,24-triol
(7*E*,22*E*)-(1*R*,3*R*,24*R*)-20-Methyl-25-(1-oxohexyl)-26,27-cyclo-19-nor-9,10-secocholesta-5,7,22-trien-1,3,24-triol
(7*E*,22*E*)-(1*R*,3*R*,24*S*)-20-Methyl-25-(1-oxohexyl)-26,27-cyclo-19-nor-9,10-secocholesta-5,7,22-trien-1,3,24-triol
(7*E*,22*E*)-(1*R*,3*R*,24*R*)-20-Methyl-25-(1-oxoheptyl)-26,27-cyclo-19-nor-9,10-secocholesta-5,7,22-trien-1,3,24-triol
(7*E*,22*E*)-(1*R*,3*R*,24*S*)-20-Methyl-25-(1-oxoheptyl)-26,27-cyclo-19-nor-9,10-secocholesta-5,7,22-trien-1,3,24-triol
(7*E*,22*E*)-(1*R*,3*R*,24*R*)-20-Methyl-25-(1-oxooctyl)-26,27-cyclo-19-nor-9,10-secocholesta-5,7,22-trien-1,3,24-triol
(7*E*,22*E*)-(1*R*,3*R*,24*S*)-20-Methyl-25-(1-oxooctyl)-26,27-cyclo-19-nor-9,10-secocholesta-5,7,22-trien-1,3,24-triol
(7*E*,22*E*)-(1*R*,3*R*,24*R*)-20-Methyl-25-(1-oxononyl)-26,27-cyclo-19-nor-9,10-secocholesta-5,7,22-trien-1,3,24-triol
(7*E*,22*E*)-(1*R*,3*R*,24*S*)-20-Methyl-25-(1-oxononyl)-26,27-cyclo-19-nor-9,10-secocholesta-5,7,22-trien-1,3,24-triol
(5*Z*,7*E*,22*E*)-[1*S*,3*R*,25(*R*)]-1,3-Dihydroxy-25-(1-hydroxy-2,2,3,3,4,4,5,5,5-nonafluorpentyl)-26,27-cyclo-9,10-secocholesta-5,7,10(19),22-tetraen-24-on
(5*Z*,7*E*,22*E*)-[1*S*,3*R*,25(*S*)]-1,3-Dihydroxy-25-(1-hydroxy-2,2,3,3,4,4,5,5,5-nonafluorpentyl)-26,27-cyclo-9,10-secocholesta-5,7,10(19),22-tetraen-24-on
(5*Z*,7*E*,22*E*)-[1*S*,3*R*,25(*R*)]-1,3-Dihydroxy-25-(1-hydroxy-2,2,3,3,4,4,5,5,6,6,7,7,7-tridecafluorheptyl)-26,27-cyclo-9,10-secocholesta-5,7,10(19),22-tetraen-24-on
(5*Z*,7*E*,22*E*)-[1*S*,3*R*,25(*S*)]-1,3-Dihydroxy-25-(1-hydroxy-2,2,3,3,4,4,5,5,6,6,7,7,7-tridecafluorheptyl)-26,27-cyclo-9,10-secocholesta-5,7,10(19),22-tetraen-24-on
(5*Z*,7*E*,22*E*)-(3*S*,24*R*)-25-Acetyl-26,27-cyclo-9,10-secocholesta-5,7,10(19),22-tetraen-3,24-diol
(5*Z*,7*E*,22*E*)-(3*S*,24*S*)-25-Acetyl-26,27-cyclo-9,10-secocholesta-5,7,10(19),22-tetraen-3,24-diol
(5*Z*,7*E*,22*E*)-(3*S*,24*R*)-25-(1-Oxopropyl)-26,27-cyclo-9,10-secocholesta-5,7,10(19),22-tetraen-3,24-diol
(5*Z*,7*E*,22*E*)-(3*S*,24*S*)-25-(1-Oxopropyl)-26,27-cyclo-9,10-secocholesta-5,7,10(19),22-tetraen-3,24-diol
(5*Z*,7*E*,22*E*)-(3*S*,24*R*)-25-(1-Oxobutyl)-26,27-cyclo-9,10-secocholesta-5,7,10(19),22-tetraen-3,24-diol
(5*Z*,7*E*,22*E*)-(3*S*,24*S*)-25-(1-Oxobutyl)-26,27-cyclo-9,10-secocholesta-5,7,10(19),22-tetraen-3,24-diol
(5*Z*,7*E*,22*E*)-(3*S*,24*R*)-25-(1-Oxopentyl)-26,27-cyclo-9,10-secocholesta-5,7,10(19),22-tetraen-3,24-diol
(5*Z*,7*E*,22*E*)-(3*S*,24*S*)-25-(1-Oxopentyl)-26,27-cyclo-9,10-secocholesta-5,7,10(19),22-tetraen-3,24-diol
(5*Z*,7*E*,22*E*)-(3*S*,24*R*)-25-(1-Oxohexyl)-26,27-cyclo-9,10-secocholesta-5,7,10(19),22-tetraen-3,24-diol
(5*Z*,7*E*,22*E*)-(3*S*,24*S*)-25-(1-Oxohexyl)-26,27-cyclo-9,10-secocholesta-5,7,10(19),22-tetraen-3,24-diol
(5*Z*,7*E*,22*E*)-(3*S*,24*R*)-25-(1-Oxoheptyl)-26,27-cyclo-9,10-secocholesta-5,7,10(19),22-tetraen-3,24-diol
(5*Z*,7*E*,22*E*)-(3*S*,24*S*)-25-(1-Oxoheptyl)-26,27-cyclo-9,10-secocholesta-5,7,10(19),22-tetraen-3,24-diol
(5*Z*,7*E*,22*E*)-(3*S*,24*R*)-25-(1-Oxooctyl)-26,27-cyclo-9,10-secocholesta-5,7,10(19),22-tetraen-3,24-diol
(5*Z*,7*E*,22*E*)-(3*S*,24S)-25-(1-Oxooctyl)-26,27-cyclo-9,10-secocholesta-5,7,10(19),22-tetraen-3,24-diol
(5*Z*,7*E*,22*E*)-(3*S*,24*R*)-25-(1-Oxononyl)-26,27-cyclo-9,10-secocholesta-5,7,10(19),22-tetraen-3,24-diol
(5*Z*,7*E*,22*E*)-(3*S*,24*S*)-25-(1-Oxononyl)-26,27-cyclo-9,10-secocholesta-5,7,10(19),22-tetraen-3,24-diol
[5*Z*,7*E*,22*E*,25(*E*)]-(1*S*,3*R*,24*R*)-25-(3-Oxo-1-heptenyl)-26,27-cyclo-9,10-secocholesta-5,7,10(19),22-tetraen-1,3,24-triol
[5*Z*,7*E*,22*E*,25(*E*)]-(1*S*,3*R*,24*S*)-25-(3-Oxo-1-heptenyl)-26,27-cyclo-9,10-secocholesta-5,7,10(19),22-tetraen-1,3,24-triol
[5*Z*,7*E*,22*E*,25(*E,E*)]-(1*S*,3*R*,24*R*)-25-(1-Oxo-2,4-hexadienyl)-26,27-cyclo-9,10-secocholesta-5,7,10(19), 22-tetraen-1,3,24-triol
[5*Z*,7*E*,22*E*,25(*E,E*)]-(1*S*,3*R*,24*S*)-25-(1-Oxo-2,4-hexadienyl)-26,27-cyclo-9,10-secocholesta-5,7,10(19), 22-tetraen-1,3,24-triol

**3.** Vitamin D-Derivate, nämlich

(5*Z*,7*E*,22*E*)-(1*S*,3*R*,24*R*)-25-Hydroxymethyl-26,27-cyclo-9,10-secocholesta-5,7,10(19),22-tetraen-1,3,24-triol
(5*Z*,7*E*,22*E*)-(1*S*,3*R*,24*S*)-25-Hydroxymethyl-26,27-cyclo-9,10-secocholesta-5,7,10(19),22-tetraen-1,3,24-triol.

**4.** Verfahren zur Herstellung der Vitamin D-Derivate der allgemeinen Formel **I** nach Anspruch 1 und 2, worin eine Verbindung der allgemeinen Formel **II**,

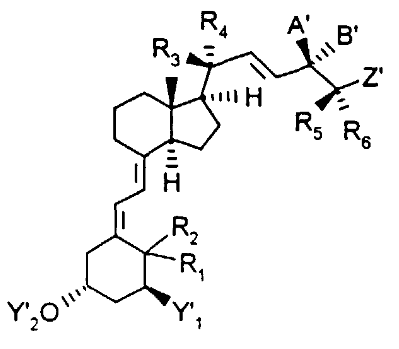

II

worin $Y'_1$ ein Wasserstoffatom oder eine geschützte Hydroxygruppe und $Y'_2$ eine Hydroxyschutzgruppe,

A' und B' gemeinsam eine Ketogruppe oder einer der beiden Substituenten eine gegebenenfalls geschützte Hydroxygruppe und der andere ein Wasserstoffatom,
Z' analoge Bedeutung wie Z hat oder gegebenenfalls schutzgruppentragende Substituenten aufweisen, bedeuten,
durch gleichzeitige oder sukzessive Abspaltung der Hydroxyschutzgruppen und gegebenenfalls durch partielle, sukzessiv oder vollständige Veresterung der freien Hydroxygruppen zu einer Verbindung der allgemeinen

Formel **I** umgesetzt wird.

**5.** Zwischenprodukte der allgemeinen Formel **XII**

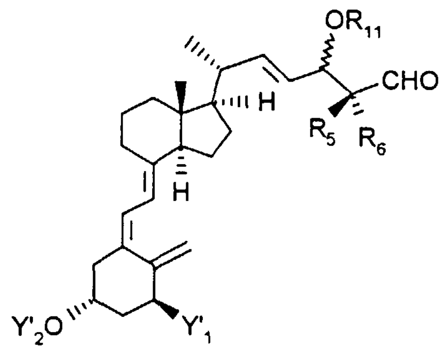

**XII**

worin

$Y'_1$, $Y'_2$, $R_5$ und $R_6$ die in Anspruch 3 genannten Bedeutungenhaben und
$R_{11}$ eine säurelabile Schutzgruppe analog $Y'_1$ oder $Y'_2$ oder die Tetrahydropyranyl-, Tetrahydrofuranyl-, Ethoxyethyl-, Methoxymethyl- oder Methoxyethoxymethylgruppe bedeutet.

**6.** Verwendung der Vitamin D-Derivate der allgemeinen Formel I zur Herstellung von Arzneimitteln.

**7.** Verwendung der Vitamin D-Derivate der allgemeinen Formel I zur Herstellung von Mitteln zur Therapie von hyperproliferativen Hauterkrankungen, Tumorerkrankungen, Präkanzerosen, Autoimmunerkrankungen, Abstoßungsreaktionen bei autologen, allogenen oder xenogenen Transplantaten, AIDS, atrophischer Haut- oder Wundheilung, sekundärem Hyperparathyroidisum, renaler Osteodystrophie, seniler und postmenopausaler Osteoporose oder von degenerativen Erkrankungen des peripheren und zentralen Nervensystems.

**8.** Verwendung von Vitamin D-Derivaten der allgemeinen Formel I, zur Herstellung von Mitteln zur Therapie von Hypercalcämien, granulomatösen Erkrankungen, paraneoplastischen Hypercalcämien, und Hypercalcämie bei Hyperparathyroidismus.

**9.** Verwendung von Vitamin D-Derivaten der allgemeinen Formel I, zur Herstellung von Mitteln zur Fertilitätskontrolle oder als Immunstimulantien, zur Therapie des Hirsutismus, zur Therapie und Prophylaxe der Arterosklerose oder zur Therapie von entzündlichen Erkrankungen.

**Claims**

**1.** Vitamin D derivative having substituents at C-25 of the general formula **I**

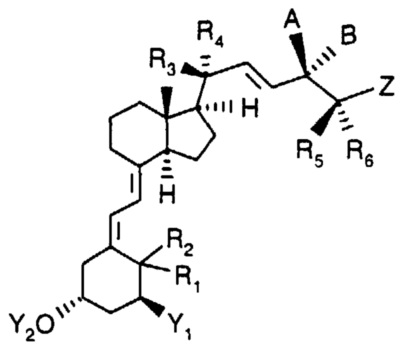

I

in which

$Y_1$ denotes a hydrogen atom, a hydroxyl group, an alkanoyloxy group having 1 to 12 carbon atoms or an aroyloxy group,
$Y_2$ denotes a hydrogen atom or an alkanoyl group having 1 to 12 carbon atoms or an aroyl group,
$R_1$ and and $R_2$ each denote a hydrogen atom or together denote an exocyclic methylene group,
$R_3$ and $R_4$ independently of one another denote a hydrogen atom, a chlorine or fluorine atom, an alkyl group having 1 to 4 carbon atoms, together denote a methylene group or together with the quaternary carbon atom 20 denote a 3-7-membered, saturated or unsaturated carbocyclic ring,
A and B together denote a keto group or A denotes a group OR' and B denotes a hydrogen atom or B denotes a group OR' and A denotes a hydrogen atom, where R' denotes a hydrogen atom or a straight-chain or branched, saturated alkanoyl group having up to 9 carbon atoms or an aroyl group,
$R_5$ and $R_6$ simultaneously each denote a hydrogen atom, a chlorine or fluorine atom, a trifluoromethyl group, a straight-chain or branched, saturated or unsaturated hydrocarbon radical having up to 4 carbon atoms, or $R_5$ and $R_6$ together with the carbon atom 25 denote a 3-7-membered, saturated or unsaturated carbocyclic ring and
Z denotes a straight-chain or branched, saturated or unsaturated hydrocarbon radical having up to 12 carbon atoms which may also have a carbo- or heterocyclic structure or partial structure and may have, at any positions, keto groups, hydroxyl groups, which for their part may be etherified or esterified, amino groups, halogen atoms or carboxylic esters or carboxamide units and which is linked by a carbonyl group, a hydroxymethylene group or an ethenediyl unit with the carbon atom 25.

**2.** Vitamin D derivative according to Claim 1,

(5Z,7E,22E)-(1S,3R,24R)-25-(1-oxopentyl)-26,27-cyclo-9,10-secocholesta-5,7,10(19),22-tetraene-1,3,24-triol
(5Z,7E,22E)-(1S,3R,24S)-25-(1-oxopentyl)-26,27-cyclo-9,10-secocholesta-5,7,10(19),22-tetraene-1,3,24-triol
(5Z,7E,22E)-(1S,3R,24R)-25-acetyl-26,27-cyclo-9,10-secocholesta-5,7,10(19),22-tetraene-1,3,24-triol
(5Z,7E,22E)-(1S,3R,24S)-25-acetyl-26,27-cyclo-9,10-secocholesta-5,7,10(19),22-tetraene-1,3,24-triol
(5Z,7E,22E)-(1S,3R,24R)-25-(1-oxopropyl)-26,27-cyclo-9,10-secocholesta-5,7,10(19),22-tetraene-1,3,24-triol
(5Z,7E,22E)-(1S,3R,24S)-25-(1-oxopropyl)-26,27-cyclo-9,10-secocholesta-5,7,10(19),22-tetraene-1,3,24-triol
(5Z,7E,22E)-(1S,3R,24R)-25-(1-oxobutyl)-26,27-cyclo-9,10-secocholesta-5,7,10(19),22-tetraene-1,3,24-triol
(5Z,7E,22E)-(1S,3R,24S)-25-(1-oxobutyl)-26,27-cyclo-9,10-secocholesta-5,7,10(19),22-tetraene-1,3,24-triol
(5Z,7E,22E)-(1S,3R,24R)-25-(1-oxohexyl)-26,27-cyclo-9,10-secocholesta-5,7,10(19),22-tetraene-1,3,24-triol
(5Z,7E,22E)-(1S,3R,24S)-25-(1-oxohexyl)-26,27-cyclo-9,10-secocholesta-5,7,10(19),22-tetraene-1,3,24-triol
(5Z,7E,22E)-(1S,3R,24R)-25-(1-oxoheptyl)-26,27-cyclo-9,10-secocholesta-5,7,10(19),22-tetraene-1,3,24-triol
(5Z,7E,22E)-(1S,3R,24S)-25-(1-oxoheptyl)-26,27-cyclo-9,10-secocholesta-5,7,10(19),22-tetraene-1,3,24-triol
(5Z,7E,22E)-(1S,3R,24R)-25-(1-oxooctyl)-26,27-cyclo-9,10-secocholesta-5,7,10(19),22-tetraene-1,3,24-triol
(5Z,7E,22E)-(1S,3R,24S)-25-(1-oxooctyl)-26,27-cyclo-9,10-secocholesta-5,7,10(19),22-tetraene-1,3,24-triol
(5Z,7E,22E)-(1S,3R,24R)-25-(1-oxononyl)-26,27-cyclo-9,10-secocholesta-5,7,10(19),22-tetraene-1,3,24-triol
(5Z,7E,22E)-(1S,3R,24S)-25-(1-oxononyl)-26,27-cyclo-9,10-secocholesta-5,7,10(19),22-tetraene-1,3,24-triol
(5Z,7E,22E)-(1S,3R,24R)-25-benzoyl-26,27-cyclo-9,10-secocholesta-5,7,10(19),22-tetraene-1,3,24-triol
(5Z,7E,22E)-(1S,3R,24S)-25-benzoyl-26,27-cyclo-9,10-secocholesta-5,7,10(19),22-tetraene-1,3,24-triol
(5Z,7E,22E)-(1S,3R,24R)-25-(2-furanylcarbonyl)-26,27-cyclo-9,10-secocholesta-5,7,10(19),22-tetraene-1,3,24-triol
(5Z,7E,22E)-(1S,3R,24S)-25-(2-furanylcarbonyl)-26,27-cyclo-9,10-secocholesta-5,7,10(19),22-tetraene-1,3,24-triol
(5Z,7E,22E)-(1S,3R,24R)-25-(2,2-dimethyl-1-oxopropyl)-26,27-cyclo-9,10-secocholesta-5,7,10(19),22-tetraene-1,3,24-triol
(5Z,7E,22E)-(1S,3R,24S)-25-(2,2-dimethyl-1-oxopropyl)-26,27-cyclo-9,10-secocholesta-5,7,10(19),22-tetraene-1,3,24-triol
(5Z,7E,22E)-(1S,3R,24R)-25-(2-pyridinylcarbonyl)-26,27-cyclo-9,10-secocholesta-5,7,10(19),22-tetraene-1,3,24-triol
(5Z,7E,22E)-(1S,3R,24S)-25-(2-pyridinylcarbonyl)-26,27-cyclo-9,10-secocholesta-5,7,10(19),22-tetraene-1,3,24-triol
[5Z,7E,22E,25(E)]-(1S,3R,24R)-25-(1-oxo-2-hexenyl)-26,27-cyclo-9,10-secocholesta-5,7,10(19),22-tetraene-1,3,24-triol
[5Z,7E,22E,25(E)]-(1S,3R,24S)-25-(1-oxo-2-hexenyl)-26,27-cyclo-9,10-secocholesta-5,7,10(19),22-tetraene-1,3,24-triol
(5Z,7E,22E)-(1S,3R,24R)-25-(l-oxo-2-hexynyl)-26,27-cyclo-9,10-secocholesta-5,7,10(19),22-tetraene-1,3,24-triol
(5Z,7E,22E)-(1S,3R,24S)-25-(1-oxo-2-hexynyl)-26,27-cyclo-9,10-secocholesta-5,7,10(19),22-tetraene-1,3,24-triol
(5Z,7E,22E)-(1S,3R,24R)-25-(cyclopropylcarbonyl)-26,27-cyclo-9,10-secocholesta-5,7,10(19),22-tetraene-1,3,24-triol
(5Z,7E,22E)-(1S,3R,24S)-25-(cyclopropylcarbonyl)-26,27-cyclo-9,10-secocholesta-5,7,10(19),22-tetraene-1,3,24-triol
[5Z,7E,22E,25(E)]-(1S,3R,24R)-25-(3-ethoxy-3-oxo-1-propenyl)-26,27-cyclo-9,10-secocholesta-5,7,10(19),22-tetraene-1,3,24-triol
[5Z,7E,22E,25(E)]-(1S,3R,24S)-25-(3-ethoxy-3-oxo-1-propenyl)-26,27-cyclo-9,10-secocholesta-5,7,10(19),22-tetraene-1,3,24-triol
[5Z,7E,22E,25(E)]-(1S,3R,24R)-25-[3-(1,1-dimethylethoxy)-3-oxo-1-propenyl]-26,27-cyclo-9,10-secocholesta-5,7,10(19),22-tetraene-1,3,24-triol
[5Z,7E,22E,25(E)]-(1S,3R,24S)-25-[3-(1,1-dimethylethoxy)-3-oxo-1-propenyl]-26,27-cyclo-9,10-secocholesta-5,7,10(19),22-tetraene-1,3,24-triol
[5Z,7E,22E,25(E)]-(1S,3R,24R)-25-(3-propoxy-3-oxo-1-propenyl)-26,27-cyclo-9,10-secocholesta-5,7,10(19),22-tetraene-1,3,24-triol
[5Z,7E,22E,25(E)]-(1S,3R,24S)-25-(3-propoxy-3-oxo-1-propenyl)-26,27-cyclo-9,10-secocholesta-5,7,10(19),22-tetraene-1,3,24-triol
[5Z,7E,22E,25(E)]-(1S,3R,24R)-25-(3-butoxy-3-oxo-1-propenyl)-26,27-cyclo-9,10-secocholesta-5,7,10(19),22-tetraene-1,3,24-triol
[5Z,7E,22E,25(E)]-(1S,3R,24S)-25-(3-butoxy-3-oxo-1-propenyl)-26,27-cyclo-9,10-secocholesta-5,7,10(19),22-tetraene-1,3,24-triol
(5Z,7E,22E)-[1S,3R,24S,25(S)]-25-(1-hydroxy-2,2,3,3,4,4,5,5,5-nonafluoropentyl)-26,27-cyclo-9,10-secocholesta-5,7,10(19),22-tetraene-1,3,24-triol
(5Z,7E,22E)-[1S,3R,24S,25(R)]-25-(1-hydroxy-2,2,3,3,4,4,5,5,5-nonafluoropentyl)-26,27-cyclo-9,10-secocholesta-5,7,10(19),22-tetraene-1,3,24-triol
(5Z,7E,22E)-[1S,3R,24R,25(S)]-25-(1-hydroxy-2,2,3,3,4,4,5,5,5-nonafluoropentyl)-26,27-cyclo-9,10-secocholesta-5,7,10(19),22-tetraene-1,3,24-triol
(5Z,7E,22E)-[1S,3R,24R,25(R)]-25-(1-hydroxy-2,2,3,3,4,4,5,5,5-nonafluoropentyl)-26,27-cyclo-9,10-secocholesta-5,7,10(19),22-tetraene-1,3,24-triol
(5Z,7E,22E)-[1S,3R,24S,25(S)]-25-(1-hydroxy-2,2,3,3,4,4,5,5,6,6,7,7,7-tridecafluoroheptyl)-26,27-cyclo-9,10-secocholesta-5,7,10(19),22-tetraene-1,3,24-triol

(5Z,7E,22E)-[1S,3R,24S,25(R)]-25-(1-hydroxy-2,2,3,3,4,4,5,5,6,6,7,7,7-tridecafluoroheptyl)-26,27-cyclo-9,10-secocholesta-5,7,10(19),22-tetraene-1,3,24-triol

(5Z,7E,22E)-[1S,3R,24R,25(S)]-25-(1-hydroxy-2,2,3,3,4,4,5,5,6,6,7,7,7-tridecafluoroheptyl)-26,27-cyclo-9,10-secocholesta-5,7,10(19),22-tetraene-1,3,24-triol

(5Z,7E,22E)-[1S,3R,24R,25(R)]-25-(1-hydroxy-2,2,3,3,4,4,5,5,6,6,7,7,7-tridecafluoroheptyl)-26,27-cyclo-9,10-secocholesta-5,7,10(19),22-tetraene-1,3,24-triol

(5Z,7E,22E)-(1S,3R,24R)-25-(trifluoroacetyl)-26,27-cyclo-9,10-secocholesta-5,7,10(19),22-tetraene-1,3,24-triol

(5Z,7E,22E)-(1S,3R,24S)-25-(trifluoroacetyl)-26,27-cyclo-9,10-secocholesta-5,7,10(19),22-tetraene-1,3,24-triol

(5Z,7E,22E)-(1S,3R,24R)-25-(perfluoroethylcarbonyl)-26,27-cyclo-9,10-secocholesta-5,7,10(19), 22-tetraene-1,3,24-triol

(5Z,7E,22E)-(1S,3R,24S)-25-(perfluoroethylcarbonyl)-26,27-cyclo-9,10-secocholesta-5,7,10(19), 22-tetraene-1,3,24-triol

(5Z,7E,22E)-(1S,3R,24R)-25-(perfluoropropylcarbonyl)-26,27-cyclo-9,10-secocholesta-5,7,10(19), 22-tetraene-1,3,24-triol

(5Z,7E,22E)-(1S,3R,24S)-25-(perfluoropropylcarbonyl)-26,27-cyclo-9,10-secocholesta-5,7,10(19), 22-tetraene-1,3,24-triol

(5Z,7E,22E)-(1S,3R,24R)-25-(perfluorobutylcarbonyl)-26,27-cyclo-9,10-secocholesta-5,7,10(19), 22-tetraene-1,3,24-triol

(5Z,7E,22E)-(1S,3R,245)-25-(perfluorobutylcarbonyl)-26,27-cyclo-9,10-secocholesta-5,7,10(19), 22-tetraene-1,3,24-triol

(5Z,7E,22E)-(1S,3R,24R)-25-(perfluoropentylcarbonyl)-26,27-cyclo-9,10-secocholesta-5,7,10(19), 22-tetraene-1,3,24-triol

(5Z,7E,22E)-(1S,3R,24S)-25-(perfluoropentylcarbonyl)-26,27-cyclo-9,10-secocholesta-5,7,10(19), 22-tetraene-1,3,24-triol

(5Z,7E,22E)-(1S,3R,24R)-25-(perfluorohexylcarbonyl)-26,27-cyclo-9,10-secocholesta-5,7,10(19), 22-tetraene-1,3,24-triol

(5Z,7E,22E)-(1S,3R,24S)-25-(perfluorohexylcarbonyl)-26,27-cyclo-9,10-secocholesta-5,7,10(19), 22-tetraene-1,3,24-triol

(5Z,7E,22E)-(1S,3R,24R)-25-acetyl-20-methyl-26,27-cyclo-9,10-secocholesta-5,7,10(19),22-tetraene-1,3,24-triol

(5Z,7E,22E)-(1S,3R,24S)-25-acetyl-20-methyl-26,27-cyclo-9,10-secocholesta-5,7,10(19),22-tetraene-1,3,24-triol

(5Z,7E,22E)-(1S,3R,24R)-20-methyl-25-(1-oxopropyl)-26,27-cyclo-9,10-secocholesta-5,7,10(19), 22-tetraene-1,3,24-triol

(5Z,7E,22E)-(1S,3R,24S)-20-methyl-25-(1-oxopropyl)-26,27-cyclo-9,10-secocholesta-5,7,10(19), 22-tetraene-1,3,24-triol

(5Z,7E,22E)-(1S,3R,24R)-20-methyl-25-(1-oxobutyl)-26,27-cyclo-9,10-secocholesta-5,7,10(19),22-tetraene-1,3,24-triol

(5Z,7E,22E)-(1S,3R,24S)-20-methyl-25-(1-oxobutyl)-26,27-cyclo-9,10-secocholesta-5,7,10(19),22-tetraene-1,3,24-triol

(5Z,7E,22E)-(1S,3R,24R)-20-methyl-25-(1-oxopentyl)-26,27-cyclo-9,10-secocholesta-5,7,10(19), 22-tetraene-1,3,24-triol

(5Z,7E,22E)-(1S,3R,24S)-20-methyl-25-(1-oxopentyl)-26,27-cyclo-9,10-secocholesta-5,7,10(19), 22-tetraene-1,3,24-triol

(5Z,7E,22E)-(1S,3R,24R)-20-methyl-25-(1-oxohexyl)-26,27-cyclo-9,10-secocholesta-5,7,10(19), 22-tetraene-1,3,24-triol

(5Z,7E,22E)-(1S,3R,24S)-20-methyl-25-(1-oxohexyl)-26,27-cyclo-9,10-secocholesta-5,7,10(19), 22-tetraene-1,3,24-triol

(5Z,7E,22E)-(1S,3R,24R)-20-methyl-25-(1-oxoheptyl)-26,27-cyclo-9,10-secocholesta-5,7,10(19), 22-tetraene-1,3,24-triol

(5Z,7E,22E)-(1S,3R,24S)-20-methyl-25-(1-oxoheptyl)-26,27-cyclo-9,10-secocholesta-5,7,10(19), 22-tetraene-1,3,24-triol

(5Z,7E,22E)-(1S,3R,24R)-20-methyl-25-(1-oxooctyl)-26,27-cyclo-9,10-secocholesta-5,7,10(19),22-tetraene-1,3,24-triol

(5Z,7E,22E)-(1S,3R,24S)-20-methyl-25-(1-oxooctyl)-26,27-cyclo-9,10-secocholesta-5,7,10(19),22-tetraene-1,3,24-triol
(5Z,7E,22E)-(1S,3R,24R)-20-methyl-25-(1-oxononyl)-26,27-cyclo-9,10-secocholesta-5,7,10(19), 22-tetraene-1,3,24-triol
(5Z,7E,22E)-(1S,3R,24S)-20-methyl-25-(1-oxononyl)-26,27-cyclo-9,10-secocholesta-5,7,10(19), 22-tetraene-1,3,24-triol
(7E,22E)-(1R,3R,24R)-25-acetyl-26,27-cyclo-19-nor-9,10-secocholesta-5,7,22-triene-1,3,24-triol
(7E,22E)-(1R,3R,24S)-25-acetyl-26,27-cyclo-19-nor-9,10-secocholesta-5,7,22-triene-1,3,24-triol
(7E,22E)-(1R,3R,24R)-25-(1-oxopropyl)-26,27-cyclo-19-nor-9,10-secocholesta-5,7,22-triene-1,3,24-triol
(7E,22E)-(1R,3R,24S)-25-(1-oxopropyl)-26,27-cyclo-19-nor-9,10-secocholesta-5,7,22-triene-1,3,24-triol
(7E,22E)-(1R,3R,24R)-25-(1-oxobutyl)-26,27-cyclo-19-nor-9,10-secocholesta-5,7,22-triene-1,3,24-triol
(7E,22E)-(1R,3R,24S)-25-(1-oxobutyl)-26,27-cyclo-19-nor-9,10-secocholesta-5,7,22-triene-1,3,24-triol
(7E,22E)-(1R,3R,24R)-25-(1-oxopentyl)-26,27-cyclo-19-nor-9,10-secocholesta-5,7,22-triene-1,3,24-triol
(7E,22E)-(1R,3R,24S)-25-(1-oxopentyl)-26,27-cyclo-19-nor-9,10-secocholesta-5,7,22-triene-1,3,24-triol
(7E,22E)-(1R,3R,24R)-25-(1-oxohexyl)-26,27-cyclo-19-nor-9,10-secocholesta-5,7,22-triene-1,3,24-triol
(7E,22E)-(1R,3R,24S)-25-(1-oxohexyl)-26,27-cyclo-19-nor-9,10-secocholesta-5,7,22-triene-1,3,24-triol
(7E,22E)-(1R,3R,24R)-25-(1-oxoheptyl)-26,27-cyclo-19-nor-9,10-secocholesta-5,7,22-triene-1,3,24-triol
(7E,22E)-(1R,3R,24S)-25-(1-oxoheptyl)-26,27-cyclo-19-nor-9,10-secocholesta-5,7,22-triene-1,3,24-triol
(7E,22E)-(1R,3R,24R)-25-(1-oxooctyl)-26,27-cyclo-19-nor-9,10-secocholesta-5,7,22-triene-1,3,24-triol
(7E,22E)-(1R,3R,24S)-25-(1-oxooctyl)-26,27-cyclo-19-nor-9,10-secocholesta-5,7,22-triene-1,3,24-triol
(7E,22E)-(1R,3R,24R)-25-(1-oxononyl)-26,27-cyclo-19-nor-9,10-secocholesta-5,7,22-triene-1,3,24-triol
(7E,22E)-(1R,3R,24S)-25-(1-oxononyl)-26,27-cyclo-19-nor-9,10-secocholesta-5,7,22-triene-1,3,24-triol
(7E,22E)-(1R,3R,24R)-25-acetyl-20-methyl-26,27-cyclo-19-nor-9,10-secocholesta-5,7,22-triene-1,3,24-triol
(7E,22E)-(1R,3R,24S)-25-acetyl-20-methyl-26,27-cyclo-19-nor-9,10-secocholesta-5,7,22-triene-1,3,24-triol
(7E,22E)-(1R,3R,24R)-20-methyl-25-(1-oxopropyl)-26,27-cyclo-19-nor-9,10-secocholesta-5,7,22-triene-1,3,24-triol
(7E,22E)-(1R,3R,24S)-20-methyl-25-(1-oxopropyl)-26,27-cyclo-19-nor-9,10-secocholesta-5,7,22-triene-1,3,24-triol
(7E,22E)-(1R,3R,24R)-20-methyl-25-(1-oxobutyl)-26,27-cyclo-19-nor-9,10-secocholesta-5,7,22-triene-1,3,24-triol
(7E,22E)-(1R,3R,24S)-20-methyl-25-(1-oxobutyl)-26,27-cyclo-19-nor-9,10-secocholesta-5,7,22-triene-1,3,24-triol
(7E,22E)-(1R,3R,24R)-20-methyl-25-(1-oxopentyl)-26,27-cyclo-19-nor-9,10-secocholesta-5,7,22-triene-1,3,24-triol
(7E,22E)-(1R,3R,24S)-20-methyl-25-(1-oxopentyl)-26,27-cyclo-19-nor-9,10-secocholesta-5,7,22-triene-1,3,24-triol
(7E,22E)-(1R,3R,24R)-20-methyl-25-(1-oxohexyl)-26,27-cyclo-19-nor-9,10-secocholesta-5,7,22-triene-1,3,24-triol
(7E,22E)-(1R,3R,24S)-20-methyl-25-(1-oxohexyl)-26,27-cyclo-19-nor-9,10-secocholesta-5,7,22-triene-1,3,24-triol
(7E,22E)-(1R,3R,24R)-20-methyl-25-(1-oxoheptyl)-26,27-cyclo-19-nor-9,10-secocholesta-5,7,22-triene-1,3,24-triol
(7E,22E)-(1R,3R,24S)-20-methyl-25-(1-oxoheptyl)-26,27-cyclo-19-nor-9,10-secocholesta-5,7,22-triene-1,3,24-triol
(7E,22E)-(1R,3R,24R)-20-methyl-25-(1-oxooctyl)-26,27-cyclo-19-nor-9,10-secocholesta-5,7,22-triene-1,3,24-triol
(7E,22E)-(1R,3R,24S)-20-methyl-25-(1-oxooctyl)-26,27-cyclo-19-nor-9,10-secocholesta-5,7,22-triene-1,3,24-triol
(7E,22E)-(1R,3R,24R)-20-methyl-25-(1-oxononyl)-26,27-cyclo-19-nor-9,10-secocholesta-5,7,22-triene-1,3,24-triol
(7E,22E)-(1R,3R,24S)-20-methyl-25-(1-oxononyl)-26,27-cyclo-19-nor-9,10-secocholesta-5,7,22-triene-1,3,24-triol
(5Z,7E,22E)-[1S,3R,25(R)]-1,3-dihydroxy-25-(1-hydroxy-2,2,3,3,4,4,5,5,5-nonafluoropentyl)-26,27-cyclo-9,10-secocholesta-5,7,10(19),22-tetraen-24-one
(5Z,7E,22E)-[1S,3R,25(S)]-1,3-dihydroxy-25-(1-hydroxy-2,2,3,3,4,4,5,5,5-nonafluoropentyl)-26,27-cyclo-9,10-secocholesta-5,7,10(19),22-tetraen-24-one
(5Z,7E,22E)-[1S,3R,25(R)]-1,3-dihydroxy-25-(1-hydroxy-2,2,3,3,4,4,5,5,6,6,7,7,7-tridecafluoroheptyl) - 26,27-cyclo-9,10-secocholesta-5,7,10(19),22-tetraen-24-one
(5Z,7E,22E)-[1S,3R,25(S)]-1,3-dihydroxy-25-(1-hydroxy-2,2,3,3,4,4,5,5,6,6,7,7,7-tridecafluoroheptyl)-

26,27-cyclo-9,10-secocholesta-5,7,10(19),22-tetraen-24-one
(5Z,7E,22E)-(3S,24R)-25-acetyl-26,27-cyclo-9,10-secocholesta-5,7,10(19),22-tetraene-3,24-diol
(5Z,7E,22E)-(3S,24S)-25-acetyl-26,27-cyclo-9,10-secocholesta-5,7,10(19),22-tetraene-3,24-diol
(5Z,7E,22E)-(3S,24R)-25-(1-oxopropyl)-26,27-cyclo-9,10-secocholesta-5,7,10(19),22-tetraene-3,24-diol
(5Z,7E,22E)-(3S,24S)-25-(1-oxopropyl)-26,27-cyclo-9,10-secocholesta-5,7,10(19),22-tetraene-3,24-diol
(5Z,7E,22E)-(3S,24R)-25-(1-oxobutyl)-26,27-cyclo-9,10-secocholesta-5,7,10(19),22-tetraene-3,24-diol
(5Z,7E,22E)-(3S,24S)-25-(1-oxobutyl)-26,27-cyclo-9,10-secocholesta-5,7,10(19),22-tetraene-3,24-diol
(5Z,7E,22E)-(3S,24R)-25-(1-oxopentyl)-26,27-cyclo-9,10-secocholesta-5,7,10(19),22-tetraene-3,24-diol
(5Z,7E,22E)-(3S,24S)-25-(1-oxopentyl)-26,27-cyclo-9,10-secocholesta-5,7,10(19),22-tetraene-3,24-diol
(5Z,7E,22E)-(3S,24R)-25-(1-oxohexyl)-26,27-cyclo-9,10-secocholesta-5,7,10(19),22-tetraene-3,24-diol
(5Z,7E,22E)-(3S,24S)-25-(1-oxohexyl)-26,27-cyclo-9,10-secocholesta-5,7,10(19),22-tetraene-3,24-diol
(5Z,7E,22E)-(3S,24R)-25-(1-oxoheptyl)-26,27-cyclo-9,10-secocholesta-5,7,10(19),22-tetraene-3,24-diol
(5Z,7E,22E)-(3S,24S)-25-(1-oxoheptyl)-26,27-cyclo-9,10-secocholesta-5,7,10(19),22-tetraene-3,24-diol
(5Z,7E,22E)-(3S,24R)-25-(1-oxooctyl)-26,27-cyclo-9,10-secocholesta-5,7,10(19),22-tetraene-3,24-diol
(5Z,7E,22E)-(3S,24S)-25-(1-oxooctyl)-26,27-cyclo-9,10-secocholesta-5,7,10(19),22-tetraene-3,24-diol
(5Z,7E,22E)-(3S,24R)-25-(1-oxononyl)-26,27-cyclo-9,10-secocholesta-5,7,10(19),22-tetraene-3,24-diol
(5Z,7E,22E)-(3S,24S)-25-(1-oxononyl)-26,27-cyclo-9,10-secocholesta-5,7,10(19),22-tetraene-3,24-diol
[5Z,7E,22E,25(E)]-(1S,3R,24R)-25-(3-oxo-1-heptenyl)-26,27-cyclo-9,10-secocholesta-5,7,10(19), 22-tetraene-1,3,24-triol
[5Z,7E,22E,25(E)]-(1S,3R,24S)-25-(3-oxo-1-heptenyl)-26,27-cyclo-9,10-secocholesta-5,7,10(19), 22-tetraene-1,3,24-triol
[5Z,7E,22E,25(E, E)]-(1S,3R,24R)-25-(1-oxo-2,4-hexadienyl)-26,27-cyclo-9,10-secocholesta-5,7,10(19), 22-tetraene-1,3,24-triol
[5Z,7E,22E,25(E, E)]-(1S,3R,24S)-25-(1-oxo-2,4-hexadienyl)-26,27-cyclo-9,10-secocholesta-5,7,10(19), 22-tetraene-1,3,24-triol

**3.** Vitamin D derivative, i.e.

(5Z,7E,22E)-(1S,3R,24R)-25-hydroxymethyl-26,27-cyclo-9,10-secocholesta-5,7,10(19),22-tetraene-1,3,24-triol
(5Z,7E,22E)-(1S,3R,24S)-25-hydroxymethyl-26,27-cyclo-9,10-secocholesta-5,7,10(19),22-tetraene-1,3,24-triol.

**4.** Process for preparing vitamin D derivatives of the general formula I according to Claim 1 and 2 in which a compound of the general formula II

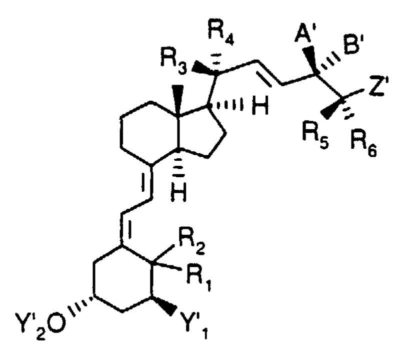

**II**

in which $Y'_1$ denotes a hydrogen atom or a protected hydroxyl group and $Y'_2$ denotes a hydroxyl protective group,

A' and B' together denote a keto group or one of the two substituents denotes an optionally protected hydroxyl group and the other denotes a hydrogen atom,
Z' has a meaning analogous to Z or has substituents which optionally carry protective groups,

is converted into a compound of the general formula I by simultaneous or successive removal of the hydroxyl protective groups and optionally by partial, successive or complete esterification of the free hydroxyl groups.

**5.** Intermediate of the general formula XII

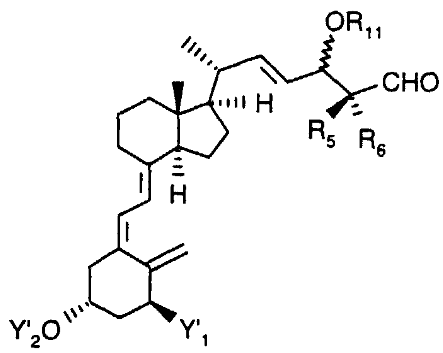

**XII**

in which

$Y'_1$, $Y'_2$, $R_5$ and $R_6$ are as defined in Claim 3 and
$R_{11}$ denotes an acid-labile protective group analogously to $Y'_1$ or $Y'_2$ or the tetrahydropyranyl, tetrahydrofuranyl, ethoxyethyl, methoxymethyl or methoxyethoxymethyl group.

**6.** Use of the vitamin D derivatives of the general formula I for preparing medicaments.

**7.** Use of the vitamin D derivatives of the general formula I for preparing compositions for the therapy of hyperproliferative disorders of the skin, tumour disorders, precanceroses, autoimmune disorders, rejection reactions in autologous, allogenic or xenogenic transplants, AIDS, atrophic skin or wound healing, secondary hyperparathyroidism, renal osteodystrophy, senile and postmenopausal osteoporosis or of degenerative disorders of the peripheral and central nervous system.

**8.** Use of vitamin D derivatives of the general formula I for preparing compositions for the therapy of hypercalcaemias, granulomatous disorders, paraneoplastic hypercalcaemias, and hypercalcaemia due to hyperparathyroidism.

**9.** Use of vitamin D derivatives of the general formula I for preparing compositions for fertility control or for use as immunostimulants, for the therapy of hirsutism, for the therapy and prophylaxis of arteriosclerosis or for the therapy of inflammatory disorders.

## Revendications

**1.** Dérivés de la vitamine D ayant des substituants en C-25 de formule générale **I**,

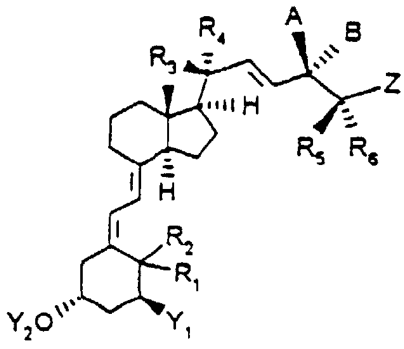

I

dans laquelle

$Y_1$ représente un atome d'hydrogène, un groupe hydroxy, un groupe alcanoyloxy ayant de 1 à 12 atomes de carbone ou un groupe aroyloxy,
$Y_2$ représente un atome d'hydrogène ou un groupe alcanoyle ayant de 1 à 12 atomes de carbone ou un groupe aroyle,
$R_1$ et $R_2$ représentent chacun un atome d'hydrogène ou conjointement un groupe méthylène exocyclique,
$R_3$ et $R_4$ représentent, indépendamment l'un de l'autre, un atome d'hydrogène, un atome de chlore ou de fluor, un groupe alkyle ayant de 1 à 4 atomes de carbone, conjointement un groupe méthylène ou, conjointement avec l'atome de carbone quaternaire 20, un noyau carbocyclique saturé ou insaturé à 3-7 chaînons,
A et B représentent conjointement un groupe céto, ou A représente un groupe OR' et B représente un atome d'hydrogène, ou B représente un groupe OR' et A représente un atome d'hydrogène, R' étant un atome d'hydrogène ou un groupe alcanoyle saturé linéaire ou ramifié ayant jusqu'à 9 atomes de carbone ou un groupe aroyle,
$R_5$ et $R_6$ représentent chacun simultanément un atome d'hydrogène, un atome de chlore ou de fluor, un groupe trifluorométhyle, un radical hydrocarboné linéaire ou ramifié, saturé ou insaturé, ayant jusqu'à 4 atomes de carbone, ou $R_5$ et $R_6$ représentent, conjointement avec l'atome de carbone 25, un noyau carbocyclique saturé ou insaturé à 3-7 chaînons,
et Z représente un radical hydrocarboné linéaire ou ramifié, saturé ou insaturé, ayant jusqu'à 12 atomes de carbone, qui peut également présenter une structure ou structure partielle carbocyclique ou hétérocyclique, et peut renfermer, en des positions quelconques, des groupes céto, des groupes hydroxy qui peuvent être éthérifiés ou estérifiés, des groupes amino, des atomes d'halogène ou des motifs ester ou amide d'acide carboxylique, et est lié à l'atome de carbone 25 par un groupe carbonyle, un groupe hydroxyméthylène ou un motif éthènediyle.

**2.** Dérivés de la vitamine D selon la revendication 1, à savoir

le (*5Z,7E,22E*)-(*1S,3R,24R*)-25-(1-oxopentyl)-26,27-cyclo-9,10-secocholesta-5,7,10(19),22-tétraène-1,3,24-triol
le (*5Z,7E,22E*)-(*1S,3R,24S*)-25-(1-oxopentyl)-26,27-cyclo-9,10-secocholesta-5,7,10(19),22-tétraène-1,3,24-triol
le (*5Z,7E,22E*)-(*1S,3R,24R*)-25-acétyl-26,27-cyclo-9,10-secocholesta-5,7,10(19),22-tétraène-1,3,24-triol
le (*5Z,7E,22E*)-(*1S,3R,24S*)-25-acétyl-26,27-cyclo-9,10-secocholesta-5,7,10(19),22-tétraène-1,3,24-triol
le (*5Z,7E,22E*)-(*1S,3R,24R*)-25-(1-oxopropyl)-26,27-cyclo-9,10-secocholesta-5,7,10(19),22-tétraène-1,3,24-triol
le (*5Z,7E,22E*)-(*1S,3R,24S*)-25-(1-oxopropyl)-26,27-cyclo-9,10-secocholesta-5,7,10(19),22-tétraène-1,3,24-triol
le (*5Z,7E,22E*)-(*1S,3R,24R*)-25-(1-oxobutyl)-26,27-cyclo-9,10-secocholesta-5,7,10(19),22-tétraène-1,3,24-triol
le (*5Z,7E,22E*)-(*1S,3R,24S*)-25-(1-oxobutyl)-26,27-cyclo-9,10-secocholesta-5,7,10(19),22-tétraène-1,3,24-triol
le (*5Z,7E,22E*)-(*1S,3R,24R*)-25-(1-oxohexyl)-26,27-cyclo-9,10-secocholesta-5,7,10(19),22-tétraène-1,3,24-triol

le (*5Z,7E,22E*)-(*1S,3R,24S*)-25-(1-oxohexyl)-26,27-cyclo-9,10-secocholesta-5,7,10(19),22-tétraène-1,3,24-triol
le (*5Z,7E,22E*)-(*1S,3R,24R*)-25-(1-oxoheptyl)-26,27-cyclo-9,10-secocholesta-5,7,10(19),22-tétraène-1,3,24-triol
le (*5Z,7E,22E*)-(*1S,3R,24S*)-25-(1-oxoheptyl)-26,27-cyclo-9,10-secocholesta-5,7,10(19),22-tétraène-1,3,24-triol
le (*5Z,7E,22E*)-(*1S,3R,24R*)-25-(1-oxooctyl)-26,27-cyclo-9,10-secocholesta-5,7,10(19),22-tétraène-1,3,24-triol
le (*5Z,7E,22E*)-(*1S,3R,24S*)-25-(1-oxooctyl)-26,27-cyclo-9,10-secocholesta-5,7,10(19),22-tétraène-1,3,24-triol
le (*5Z,7E,22E*)-(*1S,3R,24R*)-25-(1-oxononyl)-26,27-cyclo-9,10-secocholesta-5,7,10(19),22-tétraène-1,3,24-triol
le (*5Z,7E,22E*)-(*1S,3R,24S*)-25-(1-oxononyl)-26,27-cyclo-9,10-secocholesta-5,7,10(19),22-tétraène-1,3,24-triol
le (*5Z,7E,22E*)-(*1S,3R,24R*)-25-benzoyl-26,27-cycle-9,10-secocholesta-5,7,10(19),22-tétraène-1,3,24-triol
le (*5Z,7E,22E*)-(*1S,3R,24S*)-25-benzoyl-26,27-cyclo-9,10-secocholesta-5,7,10(19),22-tétraène-1,3,24-triol
le (*5Z,7E,22E*)-(*1S,3R,24R*)-25-(2-furanylcarbonyl)-26,27-cyclo-9,10-secocholesta-5,7,10(19),22-tétraène-1,3,24-triol
le (*5Z,7E,22E*)-(*1S,3R,24S*)-25-(2-furanylcarbonyl)-26,27-cyclo-9,10-secocholesta-5,7,10(19),22-tétraène-1,3,24-triol
le (*5Z,7E,22E*)-(*1S,3R,24R*)-25-(2,2-diméthyl-1-oxopropyl)-26,27-cyclo-9,10-secocholesta-5,7,10(19),22-tétraène-1,3,24-triol
le (*5Z,7E,22E*)-(*1S,3R,24S*)-25-(2,2-diméthyl-1-oxopropyl)-26,27-cyclo-9,10-secocholesta-5,7,10(19),22-tétraène-1,3,24-triol
le (*5Z,7E,22E*)-(*1S,3R,24R*)-25-(2-pyridinylcarbonyl)-26,27-cyclo-9,10-secocholesta-5,7,10(19),22-tétraène-1,3,24-triol
le (*5Z,7E,22E*)-(*1S,3R,24S*)-25-(2-pyridinylcarbonyl)-26,27-cyclo-9,10-secocholesta-5,7,10(19),22-tétraène-1,3,24-triol
le (*5Z,7E,22E*)-(*1S,3R,24R*)-25-(1-oxo-2-hexényl)-26,27-cyclo-9,10-secocholesta-5,7,10(19),22-tétraène-1,3,24-triol
le [*5Z,7E,22E,25(E)*]-(*1S,3R,24S*)-25-(1-oxo-2-hexényl)-26,27-cyclo-9,10-secocholesta-5,7,10(19),22-tétraène-1,3,24-triol
le [*5Z,7E,22E,25(E)*]-(*1S,3R,24S*)-25-(1-oxo-2-hexynyl)-26,27-cyclo-9,10-secocholesta-5,7,10(19),22-tétraène-1,3,24-triol
le (*5Z,7E,22E*)-(*1S,3R,24R*)-25-(1-oxo-2-hexynyl)-26,27-cyclo-9,10-secocholesta-5,7,10(19),22-tétraène-1,3,24-triol
le (*5Z,7E,22E*)-(*1S,3R,24R*)-25-(cyclopropylcarbonyl)-26,27-cyclo-9,10-secocholesta-5,7,10(19),22-tétraène-1,3,24-triol
le (*5Z,7E,22E*)-(*1S,3R,24S*)-25-(cyclopropylcarbonyl)-26,27-cyclo-9,10-secocholesta-5,7,10(19),22-tétraène-1,3,24-triol
le [*5Z,7E,22E,25(E)*]-(*1S,3R,24R*)-25-(3-éthoxy-3-oxo-1-propényl)-26,27-cyclo-9,10-secocholesta-5,7,10(19),22-tétraène-1,3,24-triol
le [*5Z,7E,22E,25(E)*]-(*1S,3R,24S*)-25-(3-éthoxy-3-oxo-1-propényl)-26,27-cyclo-9,10-secocholesta-5,7,10(19),22-tétraène-1,3,24-triol
le [*5Z,7E,22E,25(E)*]-(*1S,3R,24R*)-25-[3-(1,1-diméthyléthoxy)-3-oxo-1-propényl-26,27-cyclo-9,10-secocholesta-5,7,10(19),22-tétraène-1,3,24-triol
le [*5Z,7E,22E,25(E)*]-(*1S,3R,24S*)-25-[3-(1,1-diméthyléthoxy)-3-oxo-1-propényl]-26,27-cyclo-9,10-secocholesta-5,7,10(19),22-tétraène-1,3,24-triol
le [*5Z,7E,22E,25(E)*]-(*1S,3R,24R*)-25-(3-propoxy-3-oxo-1-propényl)-26,27-cyclo-9,10-secocholesta-5,7,10(19),22-tétraène-1,3,24-triol
le [*5Z,7E,22E,25(E)*]-(*1S,3R,24S*)-25-(3-propoxy-3-oxo-1-propényl)-26,27-cyclo-9,10-secocholesta-5,7,10(19),22-tétraène-1,3,24-triol
le [*5Z,7E,22E,25(E)*]-(*1S,3R,24R*)-25-(3-butoxy-3-oxo-1-propényl)-26,27-cyclo-9,10-secocholesta-5,7,10(19),22-tétraène-1,3,24-triol
le [*5Z,7E,22E,25(E)*]-(*1S,3R,24S*)-25-(3-butoxy-3-oxo-1-propényl)-26,27-cyclo-9,10-secocholesta-5,7,10(19),22-tétraène-1,3,24-triol
le (*5Z,7E,22E*)-[*1S,3R,24S,25(S)*]-25-(1-hydroxy-2,2,3,3,4,4,5,5,5-nonafluoropentyl)-26, 27-cyclo-9, 10-secocholesta-5,7,10(19),22-tétraène-1,3,24-triol

le (*5Z,7E,22E*)-(*1S,3R,24S,25(R)*]-25-(1-hydroxy-2,2,3,3,4,4,5,5,5-nonafluoropentyl)-26,27-cyclo-9,10-secocholesta-5,7,10(19),22-tétraène-1,3,24-triol
le (*5Z,7E,22E*)-[*1S,3R,24R,25(S)*]-25-(1-hydroxy-2,2,3,3,4,4,5,5,5-nonafluoropentyl)-26,27-cyclo-9,10-secocholesta-5,7,10(19),22-tétraène-1,3,24-triol
le (*5Z,7E,22E*)-[*1S,3R,24R,25(R)*]-25-(1-hydroxy-2,2,3,3,4,4,5,5,5-nonafluoropentyl)-26,27-cyclo-9,10-secocholesta-5,7,10(19),22-tétraène-1,3,24-triol
le (*5Z,7E,22E*)-[*1S,3R,24S,25(S)*]-25-(1-hydroxy-2,2,3,3,4,4,5,5,6,6,7,7,7-tridécafluoroheptyl)-26,27-cyclo-9,10-secocholesta-5,7,10(19),22-tétraène-1,3,24-triol
le (*5Z,7E,22E*)-[*1S,3R,24S,25(R)*]-25-(1-hydroxy-2,2,3,3,4,4,5,5,6,6,7,7,7-tridécafluoroheptyl)-26,27-cyclo-9,10-secocholesta-5,7,10(19),22-tétraène-1,3,24-triol
le (*5Z,7E,22E*)-[*1S,3R,24R,25(S)*]-25-(1-hydroxy-2,2,3,3,4,4,5,5,6,6,7,7,7-tridécafluoroheptyl)-26,27-cyclo-9,10-secocholesta-5,7,10(19),22-tétraène-1,3,24-triol
le (*5Z,7E,22E*)-[*1S,3R,24R,25(R)*]-25-(1-hydroxy-2,2,3,3,4,4,5,5,6,6,7,7,7-tridécafluoroheptyl)-26,27-cyclo-9,10-secocholesta-5,7,10(19),22-tétraène-1,3,24-triol
le (*5Z,7E,22E*)-(*1S,3R,24R*)-25-(trifluoroacétyl)-26,27-cyclo-9,10-secocholesta-5,7,10(19),22-tétraène-1,3,24-triol
le (*5Z,7E,22E*)-(*1S,3R,24R*)-25-(perfluoroéthylcarbonyl)-26,27-cyclo-9,10-secocholesta-5,7,10(19),22-tétraène-1,3,24-triol
le (*5Z,7E,22E*)-(*1S,3R,24S*)-25-(perfluoroéthylcarbonyl)-26,27-cyclo-9,10-secocholesta-5,7,10(19),22-tétraène-1,3,24-triol
le (*5Z,7E,22E*)-(*1S,3R,24R*)-25-(perfluoropropylcarbonyl)-26,27-cyclo-9,10-secocholesta-5,7,10(19),22-tétraène-1,3,24-triol
le (*5Z,7E,22E*)-(*1S,3R,24S*)-25-(perfluoropropylcarbonyl)-26,27-cyclo-9,10-secocholesta-5,7,10(19),22-tétraène-1,3,24-triol
le (*5Z,7E,22E*)-(*1S,3R,24R*)-25-(perfluorobutylcarbonyl)-26,27-cyclo-9,10-secocholesta-5,7,10(19),22-tétraène-1,3,24-triol
le (*5Z,7E,22E*)-(*1S,3R,24S*)-25-(perfluorobutylcarbonyl)-26,27-cyclo-9,10-secocholesta-5,7,10(19),22-tétraène-1,3,24-triol
le (*5Z,7E,22E*)-(*1S,3R,24R*)-25-(perfluoropentylcarbonyl)-26,27-cyclo-9,10-secocholesta-5,7,10(19),22-tétraène-1,3,24-triol
le (*5Z,7E,22E*)-(*1S,3R,24S*)-25-(perfluoropentylcarbonyl)-26,27-cyclo-9,10-secocholesta-5,7,10(19),22-tétraène-1,3,24-triol
le (*5Z, 7E,22E*)-(*1S,3R,24R*)-25-(perfluorohexylcarbonyl)-26,27-cyclo-9,10-secocholesta-5,7,10(19),22-tétraène-1,3,24-triol
le (*5Z,7E,22E*)-(*1S,3R,24S*)-25-(perfluorohexylcarbonyl)-26,27-cyclo-9,10-secocholesta-5,7,10(19),22-tétraène-1,3,24-triol
le (*5Z,7E,22E*)-(*1S,3R,24R*)-25-acétyl-20-méthyl-26,27-cyclo-9,10-secocholesta-5,7,10(19),22-tétraène-1,3,24-triol
le (*5Z,7E,22E*)-(*1S,3R,24S*)-25-acétyl-20-méthyl-26,27-cyclo-9,10-secocholesta-5,7,10(19),22-tétraène-1,3,24-triol
le (*5Z,7E,22E*)-(*1S,3R,24R*)-20-méthyl-25-(1-oxopropyl)-26,27-cyclo-9,10-secocholesta-5,7,10(19),22-tétraène-1,3,24-triol
le (*5Z,7E,22E*)-(*1S,3R,24S*)-20-méthyl-25-(1-oxopropyl)-26,27-cyclo-9,10-secocholesta-5,7,10(19),22-tétraène-1,3,24-triol
le (*5Z,7E,22E*)-(*1S,3R,24R*)-20-méthyl-25-(1-oxobutyl)-26,27-cyclo-9,10-secocholesta-5,7,10(19),22-tétraène-1,3,24-triol
le (*5Z,7E,22E*)-(*1S,3R,24S*)-20-méthyl-25-(1-oxobutyl)-26,27-cyclo-9,10-secocholesta-5,7,10(19),22-tétraène-1,3,24-triol
le (*5Z,7E,22E*)-(*1S,3R,24R*)-20-méthyl-25-(1-oxopentyl)-26,27-cyclo-9,10-secocholesta-5,7,10(19),22-tétraène-1,3,24-triol
le (*5Z,7E,22E*)-(*1S,3R,24S*)-20-méthyl-25-(1-oxopentyl)-26,27-cyclo-9,10-secocholesta-5,7,10(19),22-tétraène-1,3,24-triol
le (*5Z,7E,22E*)-(*1S,3R,24R*)-20-méthyl-25-(1-oxohexyl)-26,27-cyclo-9,10-secocholesta-5,7,10(19),22-tétraène-1,3,24-triol
le (*5Z,7E,22E*)-(*1S,3R,24S*)-20-méthyl-25-(1-oxohexyl)-26,27-cyclo-9,10-secocholesta-5,7,10(19),22-tétraène-1,3,24-triol
le (*5Z,7E,22E*)-(*1S,3R,24R*)-20-méthyl-25-(1-oxoheptyl)-26,27-cyclo-9,10-secocholesta-5,7,10(19),22-tétraène-1,3,24-triol

le (*5Z,7E,22E*)-(*1S,3R,24S*)-20-méthyl-25-(1-oxoheptyl)-26,27-cyclo-9,10-secocholesta-5,7,10(19),22-tétraène-1,3,24-triol
le (*5Z,7E,22E*)-(*1S,3R,24R*)-20-méthyl-25-(1-oxo-octyl)-26,27-cyclo-9,10-secocholesta-5,7,10(19),22-tétraène-1,3,24-triol
le (*5Z,7E,22E*)-(*1S,3R,24S*)-20-méthyl-25-(1-oxo-octyl)-26,27-cyclo-9,10-secocholesta-5,7,10(19),22-tétraène-1,3,24-triol
le (*5Z,7E,22E*)-(*1S,3R,24R*)-20-méthyl-25-(1-oxononyl)-26,27-cyclo-9,10-secocholesta-5,7,10(19),22-tétraène-1,3,24-triol
le (*5Z,7E,22E*)-(*1S,3R,24S*)-20-méthyl-25-(1-oxononyl)-26,27-cyclo-9,10-secocholesta-5,7,10(19),22-tétraène-1,3,24-triol
le (*7E,22E*)-(*1R,3R,24R*)-25-acétyl-26,27-cyclo-19-nor-9,10-secocholesta-5,7,22-triène-1,3,24-triol
le (*7E,22E*)-(*1R,3R,24S*)-25-acétyl-26,27-cyclo-19-nor-9,10-secocholesta-5,7,22-triène-1,3,24-triol
le (*7E,22E*)-(*1R,3R,24R*)-25-(1-oxopropyl)-26,27-cyclo-19-nor-9,10-secocholesta-5,7,22-triène-1,3,24-triol
le (*7E,22E*)-(*1R,3R,24S*)-25-(1-oxopropyl)-26,27-cycle-19-nor-9,10-secocholesta-5,7,22-triène-1,3,24-triol
le (*7E,22E*)-(*1R,3R,24R*)-25-(1-oxobutyl)-26,27-cycle-19-nor-9,10-secocholesta-5,7,22-triène-1,3,24-triol
le (*7E,22E*)-(*1R,3R,24S*)-25-(1-oxobutyl)-26,27-cyclo-19-nor-9,10-secocholesta-5,7,22-triène-1,3,24-triol
le (*7E,22E*)-(*1R,3R,24R*)-25-(1-oxopentyl)-26,27-cycle-19-nor-9,10-secocholesta-5,7,22-triène-1,3,24-triol
le (*7E,22E*)-(*1R,3R,24S*)-25-(1-oxopentyl)-26,27-cyclo-19-nor-9,10-secocholesta-5,7,22-triène-1,3,24-triol
le (*7E,22E*)-(*1R,3R,24R*)-25-(1-oxohexyl)-26,27-cycle-19-nor-9,10-secocholesta-5,7,22-triène-1,3,24-triol
le (*7E,22E*)-(*1R,3R,24S*)-25-(1-oxohexyl)-26,27-cyclo-19-nor-9,10-secocholesta-5,7,22-triène-1,3,24-triol
le (*7E,22E*)-(*1R,3R,24R*)-25-(1-oxoheptyl)-26,27-cycle-19-nor-9,10-secocholesta-5,7,22-triène-1,3,24-triol
le (*7E,22E*)-(*1R,3R,24S*)-25-(1-oxoheptyl)-26,27-cycle-19-nor-9,10-secocholesta-5,7,22-triène-1,3,24-triol
le (*7E,22E*)-(*1R,3R,24R*)-25-(1-oxo-octyl)-26,27-cycle-19-nor-9,10-secocholesta-5,7,22-triène-1,3,24-triol
le (*7E,22E*)-(*1R,3R,24S*)-25-(1-oxo-octyl)-26,27-cyclo-19-nor-9,10-secocholesta-5,7,22-triène-1,3,24-triol
le (*7E,22E*)-(*1R,3R,24R*)-25-(1-oxononyl)-26,27-cycle-19-nor-9,10-secocholesta-5,7,22-triène-1,3,24-triol
le (*7E,22E*)-(*1R,3R,24S*)-25-(1-oxononyl)-26,27-cyclo-19-nor-9,10-secocholesta-5,7,22-triène-1,3,24-triol
le (*7E,22E*)-(*1R,3R,24R*)-25-acétyl-20-méthyl-26,27-cyclo-19-nor-9,10-secocholesta-5,7,22-triène-1,3,24-triol
le (*7E,22E*)-(*1R,3R,24S*)-25-acétyl-20-méthyl-26,27-cyclo-19-nor-9,10-secocholesta-5,7,22-triène-1,3,24-triol
le (*7E,22E*)-(*1R,3R,24R*)-20-méthyl-25-(1-oxopropyl)-26,27-cyclo-19-nor-9,10-secocholesta-5,7,22-triène-1,3,24-triol
le (*7E,22E*)-(*1R,3R,24S*)-20-méthyl-25(1-oxopropyl)-26,27-cyclo-19-nor-9,10-secocholesta-5,7,22-triène-1,3,24-triol
le (*7E,22E*)-(*1R,3R,24R*)-20-méthyl-25-(1-oxobutyl)-26,27-cyclo-19-nor-9,10-secocholesta-5,7,22-triène-1,3,24-triol
le (*7E,22E*)-(*1R,3R,24S*)-20-méthyl-25-(1-oxobutyl)-26,27-cyclo-19-nor-9,10-secocholesta-5,7,22-triène-1,3,24-triol
le (*7E,22E*)-(*1R,3R,24R*)-20-méthyl-25-(1-oxopentyl) -26,27-cyclo-19-nor-9,10-secocholesta-5,7,22-triène-1,3,24-triol
le (*7E,22E*)-(*1R,3R,24S*)-20-méthyl-25-(1-oxopentyl)-26,27-cyclo-19-nor-9,10-secocholesta-5,7,22-triène-1,3,24-triol
le (*7E,22E*)-(*1R,3R,24R*)-20-méthyl-25-(1-oxohexyl)-26,27-cyclo-19-nor-9,10-secocholesta-5,7,22-triène-1,3,24-triol
le (*7E,22E*)-(*1R,3R,24S*)-20-méthyl-25-(1-oxohexyl)-26,27-cyclo-19-nor-9,10-secocholesta-5,7,22-triène-1,3,24-triol
le (*7E,22E*)-(*1R,3R,24R*)-20-méthyl-25-(1-oxoheptyl)-26,27-cyclo-19-nor-9,10-secocholesta-5,7,22-triène-1,3,24-triol
le (*7E,22E*)-(*1R,3R,24S*)-20-méthyl-25-(1-oxoheptyl)-26,27-cyclo-19-nor-9,10-secocholesta-5,7,22-triène-1,3,24-triol
le (*7E,22E*)-(*1R,3R,24R*)-20-méthyl-25-(1-oxo-octyl) -26,27-cyclo-19-nor-9,10-secocholesta-5,7,22-triène-1,3,24-triol
le (*7E,22E*)-(*1R,3R,24S*)-20-méthyl-25-(1-oxo-octyl)-26,27-cyclo-19-nor-9,10-secocholesta-5,7,22-triène-1,3,24-triol
le (*7E,22E*)-(*1R,3R,24R*)-20-méthyl-25-(1-oxononyl)-26,27-cyclo-19-nor-9,10-secocholesta-5,7,22-triène-1,3,24-triol
le (*7E,22E*)-(*1R,3R,24S*)-20-méthyl-25-(1-oxononyl)-26,27-cyclo-19-nor-9,10-secocholesta-5,7,22-triène-1,3,24-triol

la (*5Z,7E,22E*)-[*1S,3R,25(R)*]-1,3-dihydroxy-25-(1-hydroxy-2,2,3,3,4,4,5,5,5-nonafluoropentyl)-26,27-cyclo-9,10-secocholesta-5,7,10(19),22-tétraène-24-one
la (*5Z,7E,22E*)-[*1S,3R,25(S)*]-1,3-dihydroxy-25-(1-hydroxy-2,2,3,3,4,4,5,5,5-nonafluoropentyl)-26,27-cyclo-9,10-secocholesta-5,7,10(19),22-tétraène-24-one
la (*5Z,7E,22E*)-[*1S,3R,25(R)*]-1,3-dihydroxy-25-(1-hydroxy-2,2,3,3,4,4,5,5,6,6,7,7,7-tridécafluoroheptyl)-26,27-cyclo-9,10-secocholesta-5,7,10(19),22-tétraène-24-one
la (*5Z,7E,22E*)-[*1S,3R,25(S)*]-1,3-dihydroxy-25-(1-hydroxy-2,2,3,3,4,4,5,5,6,6,7,7,7-tridécafluoroheptyl)-26,27-cyclo-9,10-secocholesta-5,7,10(19),22-tétraène-24-one
le (*5Z,7E,22E*)-(*3S,24R*)-25-acétyl-26,27-cyclo-9,10-secocholesta-5,7,10(19),22-tétraène-3,24-diol
le (*5Z,7E,22E*)-(*3S,24S*)-25-acétyl-26,27-cycle-9,10-secocholesta-5,7,10(19),22-tétraène-3,24-diol
le (*5Z,7E,22E*)-(*3S,24R*)-25-(1-oxopropyl)-26,27-cyclo-9,10-secocholesta-5,7,10(19),22-tétraène-3,24-diol
le (*5Z,7E,22E*)-(*3S,24S*)-25-(1-oxopropyl)-26,27-cyclo-9,10-secocholesta-5,7,10(19),22-tétraène-3,24-diol
le (*5Z,7E,22E*)-(*3S,24R*)-25-(1-oxobutyl)-26,27-cycle-9,10-secocholesta-5,7,10(19),22-tétraène-3,24-diol
le (*5Z,7E,22E*)-(*3S,24S*)-25-(1-oxobutyl)-26,27-cyclo-9,10-secocholesta-5,7,10(19),22-tétraène-3,24-diol
le (*5Z,7E,22E*)-(*3S,24R*)-25-(1-oxopentyl)-26,27-cyclo-9,10-secocholesta-5,7,10(19),22-tétraène-3,24-diol
le (*5Z,7E,22E*)-(*3S,24S*)-25-(1-oxopentyl)-26,27-cyclo-9,10-secocholesta-5,7,10(19),22-tétraène-3,24-diol
le (*5Z,7E,22E*)-(*3S,24R*)-25-(1-oxohexyl)-26,27-cyclo-9,10-secocholesta-5,7,10(19),22-tétraène-3,24-diol
le (*5Z,7E,22E*)-(*3S,24S*)-25-(1-oxohexyl)-26,27-cycle-9,10-secocholesta-5,7,10(19),22-tétraène-3,24-diol
le (*5Z,7E,22E*)-(*3S,24R*)-25-(1-oxoheptyl)-26,27-cyclo-9,10-secocholesta-5,7,10(19),22-tétraène-3,24-diol
le (*5Z,7E,22E*)-(*3S,24S*)-25-(1-oxoheptyl)-26,27-cyclo-9,10-secocholesta-5,7,10(19),22-tétraène-3,24-diol
le (*5Z,7E,22E*)-(*3S,24R*)-25-(1-oxo-octyl)-26,27-cyclo-9,10-secocholesta-5,7,10(19),22-tétraène-3,24-diol
le (*5Z,7E,22E*)-(*3S,24S*)-25-(1-oxo-octyl)-26,27-cyclo-9,10-secocholesta-5,7,10(19),22-tétraène-3,24-diol
le (*5Z,7E,22E*)-(*3S,24R*)-25-(1-oxononyl)-26,27-cyclo-9,10-secocholesta-5,7,10(19),22-tétraène-3,24-diol
le (*5Z,7E,22E*)-(*3S,24S*)-25-(1-oxononyl)-26,27-cyclo-9,10-secocholesta-5,7,10(19),22-tétraène-3,24-diol
le [*5Z,7E,22E,25(E)*]-(*1S,3R,24R*)-25-(3-oxo-1-hepténIyl)-26,27-cyclo-9,10-secocholesta-5,7,10(19),22-tétraène-1,3,24-triol
le [*5Z,7E,22E,25(E)*]-(*1S,3R,24S*)-25-(3-oxo-1-hepténIyl)-26,27-cyclo-9,10-secocholesta-5,7,10(19),22-tétraène-1,3,24-triol
le [*5Z,7E,22E,25(E,E)*]-(*1S,3R,24R*)-25-(1-oxo-2,4-hexadiényl)-26,27-cyclo-9,10-secocholesta-5,7,10(19),22-tétraène-1,3,24-triol
le [*5Z,7E,22E,25(E,E)*]-(*1S,3R,24S*)-25-(1-oxo-2,4-hexadiényl)-26,27-cyclo-9,10-secocholesta-5,7,10(19),22-tétraène-1,3,24-triol.

**3.** Dérivés de la vitamine D, à savoir

le (*5Z,7E,22E*)-(*1S,3R,24R*)-25-hydroxyméthyl-26,27-cyclo-9,10-secocholesta-5,7,10(19),22-tétraène-1,3,24-triol
le (*5Z,7E,22E*)-(*1S,3R,24S*)-25-hydroxyméthyl-26,27-cyclo-9,10-secocholesta-5,7,10(19),22-tétraène-1,3,24-triol.

**4.** Procédé de préparation des dérivés de la vitamine D de formule générale I selon les revendications 1 et 2, dans lequel un composé de formule générale II,

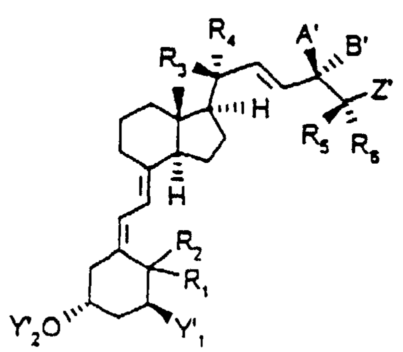

II

dans laquelle $Y'_1$ représente un atome d'hydrogène ou un groupe hydroxy protégé, et $Y'_2$ représente un groupe protecteur d'hydroxy,

A' et B' représentent conjointement un groupe céto, ou l'un des deux substituants représente un groupe hydroxy éventuellement protégé et l'autre représente un atome d'hydrogène,
Z' a une signification analogue à Z ou renferme des substituants portant éventuellement des groupes protecteurs,

est mis à réagir, par élimination simultanée ou consécutive des groupes protecteurs hydroxy et éventuellement par estérification partielle, consécutive ou totale des groupes hydroxy libres, pour donner un composé de formule générale I.

**5.** Intermédiaires de formule générale XII

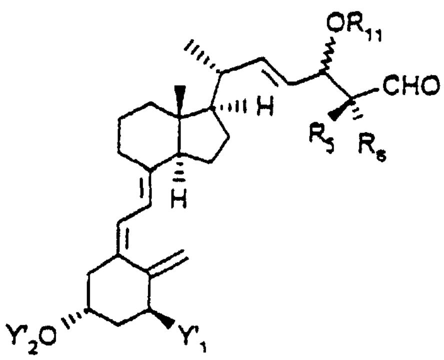

XII

dans laquelle

$Y'_1$, $Y'_2$, $R_5$ et $R_6$ ont les significations mentionnées à la revendication 3, et
$R_{11}$ représente un groupe protecteur labile vis-à-vis de l'acide analogue à $Y'_1$ ou $Y'_2$, ou le groupe tétrahydropyranyle, tétrahydrofuranyle, éthoxyéthylène, méthoxyméthyle ou méthoxyéthoxyméthyle.

**6.** Utilisation des dérivés de la vitamine D de formule générale I pour la préparation de médicaments.

**7.** Utilisation des dérivés de la vitamine D de formule générale I pour la préparation de produits destinés à la thérapie de maladies de la peau hyperprolifératives, de maladies tumorales, de prénéoplasies, de maladies auto-immunes, de réactions et répulsion dans le cas de transplantations autologues, allogènes ou xénogènes, du SIDA, d'une guérison atrophique de la peau ou des plaies, de l'hyperpara-thyroïdisme secondaire, de l'ostéodystrophie rénale, de l'ostéoporose sénile et post-ménoposique ou de maladies dégénératives du système nerveux périphérique et central.

**8.** Utilisation de dérivés de la vitamine D de formule générale I, pour la préparation de produits destinés à la thérapie d'hypercalcémies, de maladies granulomateuses, d'hypercalcémies paranéoplastiques, et de l'hypercalcémie dans le cas de l'hyperparathyroïdisme.

**9.** Utilisation de dérivés de la vitamine D de formule générale I, pour la préparation de produits destinés au contrôle des naissances ou en tant qu'immunostimulants, à la thérapie de l'hirsutisme, à la thérapie et à la prophylaxie de l'artériosclérose, ou à la thérapie de maladies inflammatoires.